(19) Europäisches Patentamt
European Patent Office
Office européen des brevets

(11) EP 1 350 792 A1

(12) # EUROPEAN PATENT APPLICATION
published in accordance with Art. 158(3) EPC

(43) Date of publication:
**08.10.2003 Bulletin 2003/41**

(21) Application number: **01270217.1**

(22) Date of filing: **10.12.2001**

(51) Int Cl.[7]: **C07D 413/12**, A61K 31/422, A61K 9/10, A61K 9/14, A61K 47/20, A61K 47/26, A61K 47/28, A61K 47/32, A61K 47/38, A61K 47/40, A61P 35/00

(86) International application number:
**PCT/JP01/10768**

(87) International publication number:
**WO 02/048141 (20.06.2002 Gazette 2002/25)**

(84) Designated Contracting States:
**AT BE CH CY DE DK ES FI FR GB GR IE IT LI LU MC NL PT SE TR**
Designated Extension States:
**AL LT LV MK RO SI**

(30) Priority: **11.12.2000 JP 2000375601**

(71) Applicant: **Takeda Chemical Industries, Ltd.**
**Osaka-shi, Osaka 541-0045 (JP)**

(72) Inventors:
- **AKIYAMA, Yohko**
  **Omihachiman-shi, Shiga 523-0898 (JP)**
- **IINUMA, Satoshi**
  **Kobe-shi, Hyogo 654-0122 (JP)**
- **BANDO, Hiroto**
  **Sakai-shi, Osaka (JP)**

(74) Representative:
**Rickard, Timothy Mark Adrian et al**
**Takeda Euro IP Department,**
**11-12 Charles II Street**
**London SW1Y 4QU (GB)**

(54) **MEDICINAL COMPOSITIONS IMPROVED IN SOLUBLITY IN WATER**

(57) Solid dispersions are provided comprising an HER2 inhibitor which is hardly or not soluble in water and a hydrophilic polymer. These solid dispersions have been improved in the solubility of the HER2 inhibitor, oral absorption and bioavailability in blood.

EP 1 350 792 A1

## Description

BACKGROUND OF THE INVENTION

Technical field

The present invention relates to a solid dispersion in which the solubility of a water-poorly soluble or insoluble HER2 inhibitory substance is improved, and a process for preparing the same.

Background

It is known that a solid dispersion prepared by forming a uniform solution or a melt of a poorly soluble compound in a hydrophilic polymer matrix, and then, making the mixture coagulated by cooling or removing a solvent improves the solubility and the absorbability and enhances the bioavailability of a drug. For example, by dispersing griseofulvin in a hydrophilic polymer, polyethylene glycol to obtain the solid dispersion, the solubility thereof is improved (J. Pharm. Sci., 60(9), 1281-1302(1971)). Further, the solid dispersion of sulfathiazole and polyvinylpyrrolidone (J. Pharm. Sci.), 58(5), 538-549(1969)), and the solid dispersion of fisoxazole or sulfamethizole and polyvinylpyrrolidone (Chem. Pharm. Bull., 27(5), 1223-1230(1979)) are known.

Japanese Patent Application No. JP-A 11-60571 describes a compound which has HER2 inhibitory activity, represented by the formula:

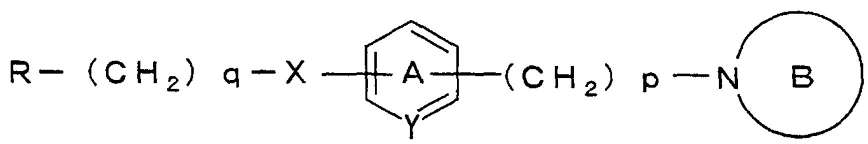

wherein R denotes an optionally substituted aromatic heterocyclic group, X denotes an oxygen atom, an optionally oxidized sulfur atom, -C(=O)- or -CH(OH)-, Y denotes CH or N, p denotes an integer of 0 to 10, q denotes an integer of 1 to 5, a group represented by the formula:

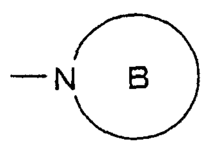

denotes an optionally substituted aromatic azole group, and a ring A may be further substituted, or a salt thereof. However it does not disclose a solid dispersion containing said compound.

SUMMARY OF THE INVENTION

An object of the present invention is to provide a novel solid dispersion in which the solubility of a water-poorly soluble or insoluble HER2 inhibitory substance is improved.

In order to attain the above object, the present inventors studied intensively, and unexpectedly found that the solubility of the HER2 inhibitory substance can be remarkably improved by making a water-poorly soluble or insoluble HER2 inhibitory substance coexist with a hydrophilic polymer. Based on this finding, the present inventors further studied, and completed the present invention.

That is, the present invention provides:

(1) a pharmaceutical composition comprising a water-poorly soluble or insoluble HER2 inhibitory substance, wherein the solubility in water of the HER2 inhibitory substance is improved;
(2) the composition described in the above (1), which is a solid dispersion;
(3) the composition described in. the above (1) or (2), which comprises a water-poorly soluble or insoluble HER2 inhibitory substance and a hydrophilic polymer;
(4) the composition described in the above (1) or (2), wherein the HER2 inhibitory substance is amorphous;

(5) the composition described in the above (3), wherein the hydrophilic polymer is cellulose derivative, polyalkenylpyrrolidone, polyalkylene glycol or methacrylic acid copolymer;
(6) the composition described in the above (3), wherein the hydrophilic polymer is an enteric polymer;
(7) the composition described in the above (3), wherein the hydrophilic polymer is hydroxypropylmethylcellulose phthalate;
(8) the composition according to any one of the above (1) to (7), which further contains lactose;
(9) the composition according to any one of the above (1) to (8), wherein the water-poorly soluble or insoluble HER2 inhibitory substance has a solubility in water of lower than 10mg/mL at 25°C;
(10) the composition according to any one of the above (1) to (8), wherein the water-poorly soluble or insoluble HER2 inhibitory substance is a compound represented by the formula:

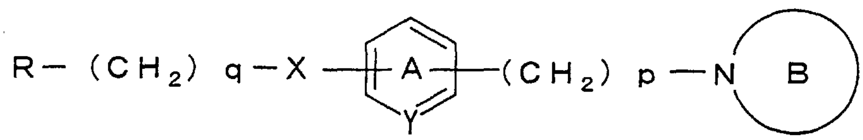

wherein R denotes an optionally substituted aromatic heterocyclic group, X denotes an oxygen atom, an optionally oxidized sulfur atom, -C(=O)- or -CH(OH)-, Y denotes CH or N, p denotes an integer of 0 to 10, q denotes an integer of 1 to 5, a group represented by the formula:

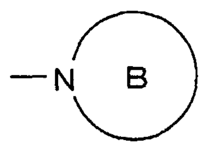

denotes an optionally substituted aromatic azole group, and ring A may be further substituted, or a salt thereof or a prodrug thereof;
(11) the composition according to any one of the above (1) to (8), wherein the water-poorly soluble or insoluble HER2 inhibitory substance is a compound represented by the formula:

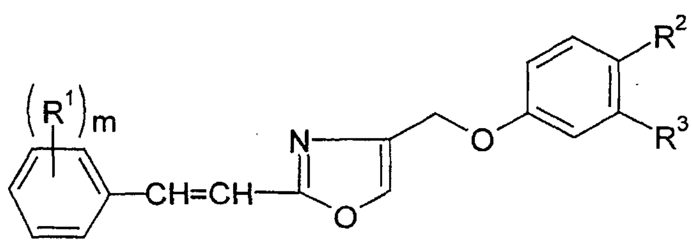

wherein m denotes 1 or 2, $R^1$ denotes halogen or optionally halogenated $C_{1-2}$alkyl, one of $R^2$ and $R^3$ denotes hydrogen atom, and the other denotes a group represented by the formula :

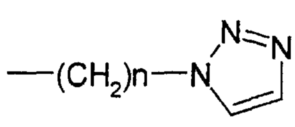

or

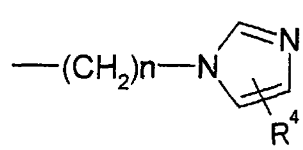

wherein n denotes 3 or 4, and $R^4$ denotes a $C_{1-4}$alkyl group substituted with 1 or 2 hydroxy group(s), or a salt thereof or a prodrug thereof;
(12) the composition described in any one of the above (1) to (8), wherein the water-poorly soluble or insoluble HER2 inhibitory substance is (i) 1-(4-{4-[(2-{(E)-2-[4-(trifluoromethyl)phenyl]ethenyl}-1,3-oxazole-4-yl)methoxy]phenyl}butyl)-1H-1,2,3-triazole, (ii) 1-(3-{3-[(2-{(E)-2-[4-(trifluoromethyl)phenyl]ethenyl}-1,3-oxazole-4-yl)methoxy]phenyl}propyl)-1H-1,2,3-triazole, or (iii) 3-(1-{4-[4-({2-[(E)-2-(2,4-difluorophenyl)ethenyl]-1,3-oxazol-4-yl}methoxy)phenyl]butyl}-1H-imidazole-2-yl) -1, 2-propanediol, or a salt thereof or a prodrug thereof;
(13) the composition described in the above (3), wherein a weight ratio of the water-poorly soluble or insoluble HER2 inhibitory substance to the hydrophilic polymer is 1:1 to 1:20;
(14) the composition described in the above (3), wherein a weight ratio of the water-poorly soluble or insoluble HER2 inhibitory substance to the hydrophilic polymer is 1:1 to 1:5;
(15) the composition described in the above (3), wherein a weight ratio of the water-poorly soluble or insoluble HER2 inhibitory substance to the hydrophilic polymer is 1:2 to 1:4;
(16) the composition described in the above (3), wherein a weight ratio of the water-poorly soluble or insoluble HER2 inhibitory substance to the hydrophilic polymer is 1:3 to 1:5;
(17) the composition described in the above (1), which is an anti-cancer agent;
(18) the composition described in the above (1), which is an agent for preventing or treating breast cancer or prostate cancer;
(19) a method for preparing a solid dispersion comprising a water-poorly soluble or insoluble HER2 inhibitory substance and a hydrophilic polymer, which comprises removing an organic solvent from a suspension or a solution of a water-poorly soluble or insoluble HER2 inhibitory substance and a hydrophilic polymer in an organic solvent; and
(20) a pharmaceutical composition containing the solid dispersion described in the above (2).

Further, the present invention provides:

(21) the solid dispersion described in the above (10), wherein a cyclic group represented by the formula:

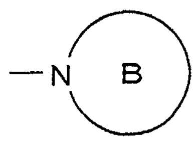

is a pyrrolyl group, an imidazolyl group, a pyrazolyl group, a triazolyl group, a tetrazolyl group or a benzimidazolyl group, each group being optionally substituted with 1 or 2 substituent(s) selected from (i) an alkyl group, (ii) an aryl group, (iii) a hydroxyalkyl group, (iv) a carboxyl group, (v) an alkoxycarbonyl group and (vi) a carbamoyl group;
(22) the solid dispersion described in the above (10), wherein p is an integer of 3 to 5;
(23) the solid dispersion described in the above (10), wherein q is 1;
(24) the solid dispersion described in the above (10), wherein X is an oxygen atom;
(25) the solid dispersion described in the above (10), wherein R is an optionally substituted oxazolyl group or an optionally substituted thiazolyl group;
(26) the solid dispersion described in the above (10), wherein R is an oxazolyl group, a benzoxazolyl group or a thiazolyl group, each group being optionally substituted with 1 or 2 substituent(s) selected from (i) an aryl group optionally substituted with 1 or 2 substituent(s) selected from a hydroxyl group, an alkoxy group, an arylalkoxy group, an alkyl group, a cyano group, a halogen atom and a tetrazolyl group, (ii) an alkyl group, (iii) a hydroxyalkyl group, (iv) an alkoxycarbonylalkyl group, (v) an alkyl group substituted with 1 or 2 aryl group(s), (vi) an alkenyl group substituted with 1 or 2 aryl group(s), (vii) a cycloalkyl group, (viii) a partially saturated naphthyl group, (ix) a thienyl group or a furyl group, being optionally substituted with 1 or 2 substituent(s) selected from a hydroxyl group, an alkoxy group, an arylalkoxy group, an alkyl group, a cyano group, an allyl group and a halogen atom, (x) a benzofuranyl group and (xi) a benzothienyl group;
(27) the solid dispersion described in the above (10), wherein R is an oxazolyl group, a benzoxazolyl group or a thiazolyl group, each group being optionally substituted with 1 or 2 substituent(s) selected from (i) an aryl group optionally substituted with 1 or 2 substituent(s) selected from a hydroxyl group, an alkoxy group, an arylalkoxy group, an alkyl group, a cyano group, a halogen atom and a tetrazolyl group, (ii) an alkyl group, (iii) a hydroxyalkyl group, (iv) an alkoxycarbonylalkyl group, (v) an alkyl group substituted with 1 or 2 aryl group(s), (vi) an alkenyl group substituted with 1 or 2 aryl group(s), (vii) a cycloalkyl group, (viii) a partially saturated naphthyl group, (ix) a thienyl group or a furyl group, being optionally substituted with 1 or 2 substituent(s) selected from a hydroxyl group, an alkoxy group, an arylalkoxy group, an alkyl group, a cyano group, an allyl group and a halogen atom, (x) a benzofuranyl group and (xi) a benzothienyl group,

X is an oxygen atom,

p is an integer of 0 to 6,
q is 1, and

a cyclic group represented by the formula:

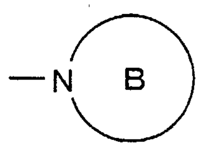

is a pyrrolyl group, an imidazolyl group, a pyrazolyl group, a triazolyl group, a tetrazolyl group or a benzimidazolyl group, each group being optionally substituted with 1 or 2 substituent(s) selected from (i) an alkyl group, (ii) an aryl group, (iii) a hydroxyalkyl group, (iv) a carboxyl group, (v) an alkoxycarbonyl group and (vi) a carbamoyl group;
(28) the solid dispersion described in the above (10), wherein R is an oxazolyl group substituted with an arylalkenyl or arylalkoxy-aryl group,

X is an oxygen atom,
p is 3 or 4,
q is 1

a cyclic group represented by the formula:

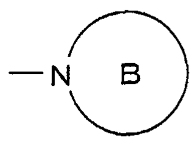

is an imidazolyl group or a triazolyl group, and
a group represented by the formula:

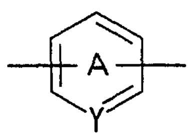

is a 1,3-phenylene group or a 1,4-phenylene group;
(29) the solid dispersion described in the above (10), wherein R is an oxazolyl group or a thiazolyl group, being substituted with a thienyl group,

X is an oxygen atom,
p is 3 or 4,
q is 1,

a cyclic group represented by the formula:

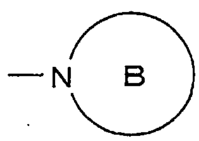

is an imidazolyl group or a triazolyl group, and

a group represented by the formula:

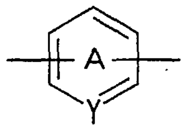

is a 1,3-phenylene group or a 1,4-phenylene group;

(30) the solid dispersion described in the above (10), wherein R is a benzoxazolyl group substituted with a thienyl group,

X is an oxygen atom,
p is 3 or 4,
q is 1,

a cyclic group represented by the formula:

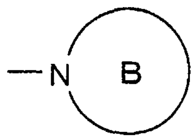

is an imidazolyl group or a triazolyl group, and

a group represented by the formula:

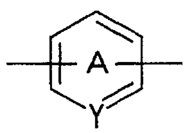

is a 1,3-phenylene group or a 1,4-phenylene group;

(31) the solid dispersion described in the above (10), wherein the water-poorly soluble or insoluble HER2 inhibitory substance is (i) 1-[4-[4-[2-[(E)-2-phenylethenyl]-4-oxazolylmethoxy]phenyl]butyl]-1,2,4-triazole, (ii) 4-[4-[4-(1-imidazolyl)butyl]phenoxymethyl]-2-[(E)-2-phenylethenyl]oxazole, (iii) 4-[4-[3-(1-imidazolyl)propyl]phenoxymethyl]-2-[(E)-2-phenylethenyl]oxazole, (iv) 4-[3-[3-(1-imidazolyl)propyl]phenoxymethyl]-2-[(E)-2-phenylethenyl]oxazole, (v) 2-(4-benzyloxyphenyl)-4-[4-[4-[3-(1-imidazolyl)propyl]phenoxymethyl]oxazole, (vi) 4-[4-[3-(1-imidazolyl)propyl]phenoxymethyl]-2-(2-thienyl)oxazole, (vii) 4-[4-[3-(1-imidazolyl)propyl]phenoxymethyl]-2-(5-methyl-2-thienyl)oxazole, (viii) 2-(5-chloro-2-thienyl)-4-[4-[3-(1-imidazolyl)-propyl]phenoxymethyl]oxazole, (ix) 4-[4-[3-(1-imidazolyl)propyl]phenoxymethyl]-2-(2-thienyl)thiazole, or (x) 5-[4-[3-(1-imidazolyl)propyl]phenoxymethyl]-2-(2-thienyl)benzoxazole, or a salt thereof or a prodrug thereof;

(32) the solid dispersion described in the above (11), wherein $R^1$ is fluoro or trifluoromethyl;

(33) the solid dispersion described in the above (11), wherein $R^2$ is a group represented by the formula:

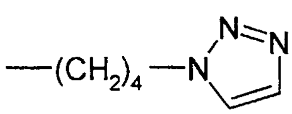

and $R^3$ is a hydrogen atom, or

$R^2$ is a hydrogen atom and $R^3$ is a group represented by the formula:

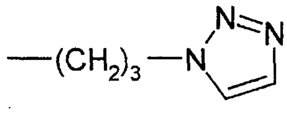

;

(34) the solid dispersion described in the above (11), wherein $R^2$ is a group represented by the formula:

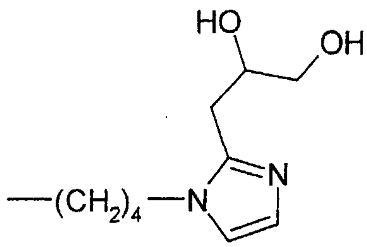

and $R^3$ is a hydrogen atom;

(35) the solid dispersion described in the above (11), wherein m is 1,

$R^1$ is 4-trifluoromethyl,

$R^2$ is a group represented by the formula:

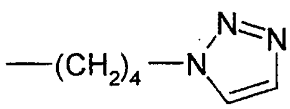

and $R^3$ is a hydrogen atom;

(36) a method for preparing a solid dispersion comprising a water-poorly soluble or insoluble HER2 inhibitory substance and a hydrophilic polymer, which comprises dissolving a water-poorly soluble or insoluble HER2 inhibitory substance in an organic solvent, adding a hydrophilic polymer to the solution to obtain a suspension or a solution and, if necessary, suspending an additive in the suspension or the solution, and then evaporating off the organic solvent;

(37) a process for preparing a solid dispersion containing a water-poorly soluble or insoluble HER2 inhibitory substance and a hydrophilic polymer, which comprises dissolving a water-poorly soluble or insoluble HER2 inhibitory substance in an organic solvent, adding a hydrophilic polymer to the solution to obtain a suspension, dissolving the suspension in an organic solvent and, if necessary, suspending an additive in the homogeneous substance obtained, and then evaporating off the organic solvent; and

(38) a pharmaceutical composition described in the above (20) which is a tablet or a capsule.

Detailed description of the invention

As used herein, "water-poorly soluble or insoluble" refers to the solubility in water at 25°C of lower than 1000ppm, preferably lower than 10ppm, or the solubility in water at 25°C of lower than 10mg/mL, preferably lower than 0.1mg/mL. The solubility can be measured by a conventional method.

As used herein, "improved water solubility" refers to, for example, improvement in the solubility in water. More specifically, it refers to, for example, about 5-fold, preferably about 10-fold, more preferably 100-fold, further preferably 1000-fold, ten thousands-fold, 100 thousands-fold, 1 million-fold or more improvement in the solubility in water at 25°C.

More specifically, it refers to the case wherein the solubility in water of a HER2 inhibitory substance to be used is, for example, 10ppm or more, preferably 1000ppm or more, more preferably 100000ppm or more at 25°C when the solubility in water of the HER2 inhibitory substance to be used is lower than 10ppm at 25°C. In addition, it refers to the case wherein the solubility in water of a HER2 inhibitory substance to be used is 0.1mg/mL or more, preferably 10mg/mL or more, further preferably 1000mg/mL or more at 25°C when the solubility in water of the HER2 inhibitory substance to be used is lower than 0.1mg/mL at 25°C.

As used herein, a "solid dispersion" refers to a dispersion in which 1 or 2 or more kinds of active component(s) is (are) dispersed in an inert carrier or a matrix in the solid state, which can be prepared by a melting method, a solvent method or a melt-solvent method (J. Pharm. Sci., Vol. 60, 1281-1302, 1971).

It is particularly desirable that the solid dispersion of the present invention is amorphous.

An average particle diameter of the solid dispersion of the present invention is not particularly limited, and usually, the size is such that a lower limit is about 0.05 μm or larger, preferably about 0.1 μm or larger, more preferably about 1 μm or larger, further preferably 3 μm or larger, and an upper limit is about 30μm or smaller, preferably about 100 μm or smaller, more preferably about 50 μm or smaller, further preferably about 10 μm or smaller.

As used herein, "HER2" refers to "growth factor receptor tyrosinekinase".

The "water-poorly soluble or insoluble HER2 inhibitory substance" is not particularly limited as far as it is a HER2 inhibitory substance exhibiting the above-mentioned solubility. More specifically, a compound (I) represented by the formula:

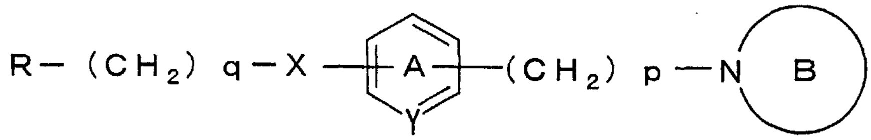

wherein R denotes an optionally substituted aromatic heterocyclic group, X denotes an oxygen atom, an optionally oxidized sulfur atom, -C(=O)- or -CH(OH)-, Y denotes CH or N, p denotes an integer of 0 to 10, q denotes an integer of 1 to 5, a group represented by the formula:

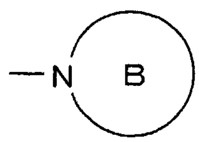

denotes an aromatic azole group, and a ring A may be further substituted, or a salt thereof or a prodrug thereof is used.

In the above formula (I), examples of a heterocyclic group in an optionally substituted aromatic heterocyclic group represented by R include (1) a 5- or 6-membered aromatic monocyclic heterocyclic group containing 1 to 4 atom (s) selected from a nitrogen atom, an oxygen atom and a sulfur atom in addition to carbon atoms as ring constituting atoms, and (2) an aromatic fused heterocyclic group formed by fusing (i) a 5- or 6- membered aromatic monocyclic heterocycle containing 1 to 4 atom(s) selected from a nitrogen atom, an oxygen atom and a sulfur atom in addition to a carbon atoms as ring constituting atoms, with (ii) a 5-or 6-membered aromatic or non-aromatic heterocycle containing 1 or 2 nitrogen atom(s) in addition to carbon atoms as ring constituting atoms, a benzene ring, or a 5-membered aromatic or non-aromatic heterocycle containing one sulfur atom in addition to carbon atoms as ring constituting atoms.

Examples of such aromatic heterocyclic groups include pyridyl (e.g. 2-pyridyl, 3-pyridyl, 4-pyridyl), pyrimidinyl (e.g. 2-pyrimidinyl, 5-pyrimidinyl, 6-pyrimidinyl), pyridazinyl (e.g. 3-pyridazinyl, 4-pyridazinyl), pyrazinyl (e.g. 2-pyrazinyl), pyrrolyl (e.g. 1-pyrrolyl, 2-pyrrolyl), imidazolyl (e.g. 1-imidazolyl, 2-imidazolyl, 4-imidazolyl, 5-imidazolyl), pyrazolyl (e.g. 1-pyrazolyl, 3-pyrazolyl, 4-pyrazolyl), isoxazolyl, isothiazolyl, thiazolyl (e.g. 2-thiazolyl, 4-thiazolyl, 5-thiazolyl), oxazolyl (e.g. 2-oxazolyl, 4-oxazolyl, 5-oxazolyl), oxadiazolyl (e.g. 1,2,4-oxadiazolyl such as 1,2,4-oxadiazol-5-yl, 1,2,3-oxadiazolyl, 1,3,4-oxadiazolyl), thiadiazolyl (e.g. 1,2,3-thiadiazolyl, 1,2,4-thiadiazolyl, 1,3,4-thiadiazolyl), triazolyl (e.g. 1,2,4-triazolyl such as 1,2,4-triazol-1-yl, 1,2,4-triazol-5-yl and the like, 1,2,3-triazolyl such as 1,2,3-triazol-1-yl, 1,2,3-triazol-2-yl, 1,2,3-triazol-4-yl and the like), tetrazolyl (e.g. tetrazol-1-yl, tetrazol-5-yl), benzimidazolyl (e.g. benzimidazol-1-yl, benzimidazol-2-yl), indolyl (e.g. indol-1-yl, indol-3-yl), indazolyl (e.g. 1H-indazol-1-yl, 1H-indazol-3-yl), pyrrolopyrazinyl (e.g. 1H-pyrrolo[2,3-b]pyrazinyl), pyrrolopyridyl (e.g. 1H-pyrrolo[2,3-b)pyridyl), imidazopyridyl (e.g. 1H-imidazo[4,5-b]pyridyl, 1H-imidazo[4,5-c]pyridyl), imidazopyrazinyl (e.g. 1H-imidazo[4,5-b]pyrazinyl), pyrrolopyridazinyl (e.g. pyrrolo[1,2-b]pyridazinyl), pyrazolopyridyl (e.g. pyrazolo[1,5-a]pyridyl), imidazopyridyl (e.g. imidazo[1,2-a] pyridyl, imidazo[1,5-a]pyridyl), imidazopyridazinyl (e.g. imidazo[1,2-b]pyridazinyl), imidazopyrimidinyl (e.g. imidazo [1,2-a]pyrimidinyl), furyl, thienyl, benzofuranyl, benzothienyl (e.g. benzo[b]thienyl), benzoxazolyl, benzthiazolyl, quinolyl, isoquinolyl, quinazolinyl and the like, and preferable examples include a 5-membered monocyclic aromatic azole group such as oxazolyl, thiazolyl, isoxazolyl, isothiazolyl, imidazolyl, triazolyl, oxaziazolyl and thiaziazolyl, an aromatic fused azole group fused with a benzene ring such as benzoxazolyl and benzothiazolyl, and a 6-membered monocyclic aromatic heterocycle such as pyridyl and pyrimidyl. More preferable examples of an aromatic heterocycle include a

5-membered monocyclic aromatic azole group such as an oxazolyl group and a thiazolyl group.

Examples of an aromatic heterocyclic group represented by R and an aromatic azole group represented by the formula

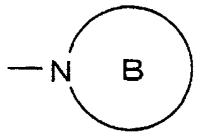

include (1) a 5-membered aromatic monocyclic heterocyclic group containing 1 to 4 nitrogen atom(s) and optionally containing one oxygen atom or one sulfur atom in addition to carbon atoms as ring constituting atoms, and (2) an aromatic fused heterocyclic group formed by fusing (i) a 5-membered aromatic monocyclic heterocycle containing 1 to 4 nitrogen atom(s) and optionally containing one oxygen atom or one sulfur atom in addition to carbon atoms as ring constituting atoms, with (ii) a 5- or 6-membered aromatic or non-aromatic heterocycle containing 1 or 2 nitrogen atom (s) in addition to carbon atoms as ring constituting atoms, a benzene ring, or a 5-membered aromatic or non-aromatic heterocycle containing one sulfur atom in addition to carbon atoms as ring constituting atoms.

Examples of such the aromatic azole group include aromatic heterocyclic groups such as pyrrolyl (e.g. 1-pyrrolyl), imidazolyl (e.g. 1-imidazolyl), pyrazolyl (e.g. 1-pyrazolyl), triazolyl (e.g. 1,2,4-triazol-1-yl, 1,2,3-triazol-1-yl), tetrazolyl (e.g. tetrazol-1-yl), benzimidazolyl (benzimidazol-1-yl), indolyl (e.g. indol-1-yl), indazolyl (e.g. 1H-indazol-1-yl), pyrrolopyrazinyl (e.g. 1H-pyrrolo[2,3-b]pyrazin-1-yl), pyrrolopyridyl (e.g. 1H-pyrrolo[2,3-b]pyridin-1-yl), imidazopyridyl (e.g. 1H-imidazo[4,5-b]pyridin-1-yl), imidazopyrazinyl (e.g. 1H-imidazo[4,5-b]pyrazin-1-yl) and the like, which are bound with $-(CH_2)_m-$ via a nitrogen atom contained as one of ring constituting atoms. Preferable examples of an aromatic azole group include an imidazolyl group and a triazolyl group.

An aromatic heterocyclic group represented by R and an aromatic azole group represented by the formula:

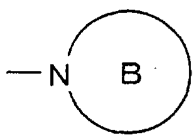

may have 1 to 3 (preferably 1 or 2) substituent(s) at a replaceable position. Examples of the substituent include an aliphatic hydrocarbon group, an alicyclic hydrocarbon group, an aromatic hydrocarbon group, an aliphatic hydrocarbon group substituted with an aromatic hydrocarbon group, an aliphatic hydrocarbon substituted with an alicyclic hydrocarbon group, an aromatic heterocyclic group, a non-aromatic heterocyclic group, an aliphatic hydrocarbon group substituted with an aromatic heterocyclic group, a halogen atom, a nitro group, a cyano group, an optionally substituted amino group, an optionally substituted acyl group, an optionally substituted hydroxyl group, an optionally substituted thiol group, and optionally esterified or amidated carboxyl group. Each of an aliphatic hydrocarbon group, an alicyclic hydrocarbon group, an aromatic hydrocarbon group, an aliphatic hydrocarbon group substituted with an aromatic hydrocarbon group, an aliphatic hydrocarbon group substituted with an alicyclic hydrocarbon group, an aromatic heterocyclic group, a non-aromatic heterocyclic group, and an aliphatic hydrocarbon group substituted with an aromatic heterocyclic group, as a substituent, may be further substituted.

Ring A may further have 1 to 4 (preferably 1 or 2) substituent(s) at a replaceable position in addition to X and $(CH_2)_P$. Examples of the substituent include substituents exemplified as a substituent which may be possessed by a substituent on an aromatic heterocyclic group represented by R, for example, an aliphatic hydrocarbon group, an alicyclic hydrocarbon group, an aromatic hydrocarbon group, an aliphatic hydrocarbon group substituted with an aromatic hydrocarbon group, an aliphatic hydrocarbon group substituted with an alicyclic hydrocarbon group, an aromatic heterocyclic group, a non-aromatic heterocyclic group, an aliphatic hydrocarbon group substituted with an aromatic heterocyclic group, a halogen atom, a nitro group, a cyano group, an optionally substituted amino group, an optionally substituted acyl group, an optionally substituted hydroxyl group, an optionally substituted thiol group, and an optionally esterified or amidated carboxyl group and the like. Each of an aliphatic hydrocarbon group, an alicyclic hydrocarbon group, an aromatic hydrocarbon group, an aliphatic hydrocarbon group substituted with an aromatic hydrocarbon group, an aliphatic hydrocarbon group substituted with an alicyclic hydrocarbon group, an aromatic heterocyclic group, a non-aromatic heterocyclic group, and an aliphatic hydrocarbon group substituted with an aromatic heterocyclic group, as a substituent, may be further substituted.

Examples of an aliphatic hydrocarbon group include a straight or branched aliphatic hydrocarbon group having

1 to 15 carbons such as an alkyl group, an alkenyl group, an alkynyl group and the like.

Examples of a preferable alkyl group include alkyl groups having 1 to 10 carbons such as methyl, ethyl, propyl, isopropyl, butyl, isobutyl, sec-butyl, tert-butyl, pentyl, isopentyl, neopentyl, tert-pentyl, hexyl, isohexyl, heptyl, octyl, nonyl, decyl, 1/1-dimethylbutyl, 2,2-dimethylbutyl, 3,3-dimethylbutyl, 2-ethylbutyl and the like, and more preferable examples include alkyl groups having 1 to 6 carbons.

Examples of a preferable alkenyl group include alkenyl groups having 2 to 10 carbons such as vinyl (ethenyl), allyl, isopropenyl, 1-propenyl, 2-methyl-1-propenyl, 1-butenyl, 2-butenyl, 3-butenyl, 2-ethyl-1-butenyl, 3-methyl-2-butenyl, 1-pentenyl, 2-pentenyl, 3-pentenyl, 4-pentenyl, 4-methyl-3-pentenyl, 1-hexenyl, 2-hexenyl, 3-hexenyl, 4-hexenyl, 5-hexenyl and the like, and more preferable examples include alkenyl groups having 2 to 6 carbons.

Examples of a preferable alkynyl group include alkynyl groups having 2, to 10 carbons such as ethynyl, 1-propynyl, 2-propynyl, 1-butynyl, 2-butynyl, 3-butynyl, 1-pentynyl, 2-pentynyl, 3-pentynyl, 4-pentynyl, 1-hexynyl, 2-hexynyl, 3-hexynyl, 4-hexynyl, 5-hexynyl and the like, and more preferable examples include alkynyl groups having 2 to 6 carbons.

Examples of an alicyclic hydrocarbon group include saturated or unsaturated alicyclic hydrocarbon groups having 3 to 12 carbons such as a cycloalkyl group, a cycloalkenyl group, a cycloalkadienyl group, a partially unsaturated fused dicyclic hydrocarbon group and the like.

Examples of a preferable cycloalkyl group include cycloalkyl groups having 3 to 10 carbons such as cyclopropyl, cyclobutyl, cyclopentyl, cyclohexyl, cycloheptyl, cyclooctyl and the like, and bicycloalkyl groups having 6 to 10 carbons such as bicycle[2.2.1]heptyl, bicyclo[2.2.2]octyl, bicyclo[3.2.1]octyl, bicyclo[3.2.2]nonyl, bicyclo[3.3.1]nonyl, bicyclo[4.2.1]nonyl, bicyclo[4.3.1]decyl and the like.

Examples of a preferable cycloalkenyl group include cycloalkenyl groups having a carbon number of 5 to 10 such as 2-cyclopenten-1-yl, 3-cyclopenten-1-yl, 2-cyclohexen-1-yl, 3-cyclohexen-1-yl and the like.

Examples of a preferable cycloalkadienyl group include cycloalkadienyl groups having 5 to 10 carbons such as 2,4-cyclopentadien-1-yl, 2,4-cyclohexadien-1-yl, 2,5-cyclohexadien-1-yl and the like.

Examples of a preferable partially unsaturated fused dicyclic hydrocarbon group include groups having 9 to 12 carbons formed by fusing a benzene ring such as an indanyl group, a partially saturated naphthyl group (e.g. a dihydronaphthyl group such as 3,4-dihydro-2-naphthyl, tetrahydronaphthyl such as 1,2,3,4-tetrahydronaphthyl) with an alicyclic hydrocarbon.

Examples of an aromatic hydrocarbon group include monocyclic or fused polycyclic aromatic hydrocarbon groups, preferable examples include an aryl group 6 to 14 carbons, such as phenyl, naphthyl, anthryl, phenanthryl, acenaphthylenyl, 9-fluorenon-2-yl and the like and, inter alia, monocyclic or fused dicyclic aromatic hydrocarbon groups such as phenyl, 1-naphthyl, 2-naphthyl and the like are preferable.

Examples of an aliphatic hydrocarbon group substituted with aromatic hydrocarbon group(s) include aliphatic hydrocarbon groups substituted with 1 to 3 (preferably 1 or 2) aromatic hydrocarbon group(s) having 7 to 20 carbons. Preferable examples of such an aliphatic hydrocarbon group substituted with an aromatic hydrocarbon group include $C_{1-6}$alkyl groups substituted with 1 to 3 $C_{6-14}$aryl group(s) (e.g. $C_{1-6}$alkyl groups substituted with 1 to 3 phenyl group (s) such as benzyl, 2-phenylethyl, 1,2-diphenylethyl, 2,2-diphenylethyl and the like, $C_{1-6}$alkyl groups substituted with 1 to 3 naphthyl group(s), 9-fluorenyl-$C_{1-6}$alkyl, etc), $C_{2-6}$alkenyl groups substituted with 1 to 3 $C_{6-14}$aryl group(s) (e.g. $C_{2-6}$alkenyl groups substituted with 1 to 3 phenyl group(s) such as (E)-2-phenylethenyl, (Z)-2-phenylethenyl, 2,2-diphenylethenyl, 2-(2-naphthyl)ethenyl, 4-phenyl-1,3-butadienyl and the like, $C_{2-6}$alkenyl substituted with 1 to 3 naphthyl group(s), 9-fluorenylidenealkyl group) and the like.

Examples of an aliphatic hydrocarbon group substituted with alicyclic hydrocarbon group(s) include the above-mentioned aliphatic hydrocarbon groups substituted with 1 to 3 (preferably 1 or 2) above-mentioned alicyclic hydrocarbon group(s). Preferable examples of such an aliphatic hydrocarbon group substituted with alicyclic hydrocarbon include $C_{1-6}$alkyl groups substituted with 1 to 3 $C_{3-10}$Cycloalkyl group(s) such as cyclopropylmethyl, cyclopropylethyl, cyclobutylmethyl, cyclopentylmethyl, 2-cyclopentenylmethyl, 3-cyclopentenylmethyl, cyclohexylmethyl, 2-cyclohexenylmethyl, 3-cyclohexenylmethyl, cyclohexylethyl, cyclohexylpropyl, cycloheptylmethyl, cycloheptylethyl and the like, $C_{2-6}$alkenyl groups substituted with 1 to 3 $C_{3-10}$Cycloalkyl group (s), $C_{1-6}$alkyl groups substituted with 1 to 3 $C_{5-10}$cycloalkenyl group(s), $C_{2-6}$alkenyl groups substituted with 1 to 3 $C_{5-10}$cycloalkenyl group(s), and the like.

Preferable examples of an aromatic heterocyclic group include a 5-or 6-membered aromatic monocyclic heterocyclic group containing 1 to 4 atom(s) selected from a nitrogen atom, an oxygen atom and a sulfur atom in addition to carbon atoms as ring constituting atoms, such as furyl, thienyl, pyrrolyl, oxazolyl, isoxazolyl, thiazolyl, isothiazolyl, imidazolyl, pyrazolyl, 1,2,3-oxadiazolyl, 1,2,4-oxadiazolyl, 1,3,4-oxadiazolyl, phlazanyl, 1,2,3-thiadiazolyl, 1,2,4-thiadiazolyl, 1,3,4-thiadiazolyl, 1,2,3-triazolyl, 1,2,4-triazolyl, tetrazolyl, pyridyl, pridazinyl, pyrimidinyl, pyrazinyl and triazinyl; and an aromatic fused heterocyclic group formed by fusing (i) a 5-or 6-membered aromatic heterocycle containing 1 to 4 atom(s) selected from a nitrogen atom, an oxygen atom and a sulfur atom in addition to carbon atoms as ring constituting atoms, with (ii) a 5- or 6-membered aromatic or non-aromatic heterocycle containing 1 or 2 nitrogen atom (s) in addition to carbon atoms as ring constituting atoms, a benzene ring, or a 5-membered aromatic or non-aromatic

heterocycle containing one sulfur atom in addition to carbon atoms as ring constituting atoms, such as benzofuranyl, isobenzofuranyl, benzo[b]thienyl, indolyl, isoindolyl, 1H-indazolyl, benzimidazolyl, benzoxazolyl, 1,2-benzisoxazolyl, benzothiazolyl, 1,2-benzisothiazolyl, 1H-benzotriazolyl, quinolyl, isoquinolyl, cinnolinyl, quinazolynyl, quinoxalinyl, phthalazinyl, naphthylidinyl, purinyl, pteridinyl, carbazolyl, α-carbolinyl, β-carbolinyl, γ-carbolinyl, acridinyl, phenoxazinyl, phenothiazinyl, phenazinyl, phenoxathinyl, thianthrenyl, phenanthridinyl, phenanthrolinyl, indolizinyl, pyrrolo[1,2-b]pyridazinyl, pyrazolo[1,5-a]pyridyl, imidazo[1,2-a]pyridyl, imidazo[1,5-a]pyridyl, imidazo[1,2-b]pyridazinyl, imidazo[1,2-a]pyrimidinyl, 1,2,4-triazolo[4,3-a]pyridyl and 1,2,4-triazolo[4,3-b]pyridazinyl.

Preferable examples of a non-aromatic heterocyclic group include 3 to 7-membered non-aromatic heterocyclic groups containing 1 or 2 atom(s) selected from a nitrogen atom, an oxygen atom and a sulfur atom in addition to carbon atoms as ring constituting atoms, such as oxiranyl, azetidinyl, oxetanyl, thietanyl, pyrrolidinyl, tetrahydrofuryl, thiolanyl, piperidyl, tetrahydropyranyl, morpholinyl, thiomorpholinyl, piperazinyl and the like.

Examples of an aliphatic hydrocarbon group substituted with aromatic heterocyclic group(s) include aliphatic hydrocarbon groups having 1 to 6 carbons (e.g. $C_{1-6}$alkyl group, $C_{2-6}$alkenyl group, etc.) substituted with 1 to 3 (preferably 1 or 2) above-mentioned aromatic heterocyclic group(s). Preferable examples of an aliphatic hydrocarbon group substituted with an aromatic heterocyclic group include $C_{1-6}$alkyl groups substituted with 1 to 3 substituent(s), for example a furyl group, a thienyl group, an imidazolyl group and a pyridyl group (e.g. (2-furyl)methyl, thienylmethyl, 2-(1-imidazolyl)ethyl, etc.), $C_{2-6}$alkenyl groups substituted with 1 to 3 of a furyl group, a thienyl group, an imidazolyl group or a pyridyl group (e.g. 2-(2-furyl)ethenyl, 2-thienylethenyl, etc.), and the like.

Examples of a halogen atom include fluorine, chlorine, bromine and iodine and, inter alia, fluorine and chlorine are preferable.

Examples of an optionally substituted amino group include amino groups (e.g. methylamino, dimethylamino, ethylamino, diethylamino, dibutylamino, diallylamino, cyclohexylamino, acetylamino, propionylamino, benzoylamino, phenylamino, N-methyl-N-phenylamino, etc.), and 4- to 6-membered cyclic amino groups (e.g. 1-azetidinyl, 1-pyrrolidinyl, piperidino, morpholino, 1-piperazinyl etc.) which are optionally mono- or di-substituted with an alkyl group having 1 to 10 carbons, a cycloalkyl group having 3 to 10 carbons, an alkenyl group having 2 to 10 carbons, a cycloalkenylgroup having 5 to 10 carbons, an acyl group having 1 to 10 carbons or an aromatic hydrocarbon group having 6 to 12 carbons.

Herein, 4- to 6-membered cyclic amino groups may be further substituted with, for example, (i) a $C_{1-6}$alkyl group, (ii) a $C_{6-14}$aryl group (e.g. phenyl, naphthyl, etc.) optionally substituted with halogen, $C_{1-6}$alkoxy group or trifluoromethyl, (iii) a 5-or 6-membered heterocyclic group containing 1 to 2 nitrogen atom(s) in addition to carbon atoms as ring constituting atoms (e.g. 2-pyridyl, pyrimidinyl) or (iv) a 6-membered cyclic amino group (e.g. piperidino, 1-piperazinyl, etc.).

Examples of an acyl group in an optionally substituted acyl group include acyl groups having 1 to 13 carbons, in particular, groups in which an alkyl group having 1 to 6 carbons, a cycloalkyl group having 3 to 10 carbons, an alkenyl group having 2 to 6 carbons, a cycloalkenyl group having 5 to 10 carbons, an aromatic hydrocarbon group having 6 to 12 carbons (e.g. phenyl, naphthyl, etc.) or an aromatic heterocycle (e.g. pyridyl) is bound to a carbonyl group, such as $C_{2-7}$alkanoyl groups (e.g. acetyl, propionyl, butyryl, isobutyryl, valeryl, isovaleryl, pivaloyl, hexanoyl, heptanoyl, octanoyl, etc.), $C_{3-10}$cycloalkylcarbonyl groups (e.g. cyclobutanecarbonyl, cyclopentanecarbonyl, cyclohexanecarbonyl, cycloheptanecarbonyl, etc.), $C_{3-7}$alkenoyl groups (e.g. crotonoyl, etc.), $C_{5-10}$cycloalkenyl-carbonyl groups (e.g. 2-cyclohexenecarbonyl, etc.), a benzoyl group, a nicotinoyl group and the like.

Examples of a substituent in an optionally substituted acyl group include an alkyl group having 1 to 3 carbons, an alkoxy group having 1 to 3 carbons, a halogen (e.g. chlorine, fluorine, bromine, etc.), a nitro group, a hydroxyl group, an amino group and the like. The number of the substituents is, for example, 1 to 3.

Examples of an optionally substituted hydroxyl group include a hydroxyl group, an alkoxy group, a cycloalkyloxy group, an alkenyloxy group, a cycloalkenyloxy group, an aralkyloxy group, an aryloxy group, an acyloxy group and the like.

Preferable examples of an alkoxy group include alkoxy groups having 1 to 10 carbons, such as methoxy, ethoxy, propoxy, isopropoxy, butoxy, isobutoxy, sec-butoxy, tert-butoxy, pentyloxy, isopentyloxy, neopentyloxy, hexyloxy, heptyloxy, nonyloxy and the like.

Preferable examples of a cycloalkyloxy group include cycloalkyloxy groups having 3 to 10 carbons, such as cyclobutoxy, cyclopentyloxy, cyclohexyloxy and the like.

Preferable example of an alkenyloxy group include alkenyloxy groups having 2 to 10 carbons, such as allyloxy, crotyloxy, 2-pentenyloxy, 3-hexenyloxy and the like.

Preferable examples of a cycloalkenyloxy group include cycloalkenyloxy groups having 5 to 10 carbons, such as 2-cyclopentenyloxy, 2-cyclohexenyloxy and the like.

Preferable examples of an aralkyloxy group include aralkyloxy groups having 7 to 20 carbons, such as $C_{6-14}$aryl-$C_{1-6}$alkoxy group, in particular, phenyl-$C_{1-6}$alkoxy group (e.g. benzyloxy, phenethyloxy, etc.), naphthyl-$C_{1-6}$alkoxy group and the like.

Preferable examples of an aryloxy group include aryloxy groups having 6 to 14 carbons optionally substituted

with an alkyl group having 1 to 3 carbons, an alkoxy group having 1 to 3 carbons, halogen, a nitro group, a hydroxyl group or an amino group, such as phenoxy, 4-chlorophenoxy and the like.

Preferable examples of an acyloxy group include acyloxy groups having 2 to 15 carbons, such as alkanoyloxy groups having 2 to 7 carbons (e.g. acetyloxy, propionyloxy, butyryloxy, isobutyryloxy, etc.), $C_{6-14}$aryl-carbonyloxy (e. g. benzoyloxy, naphthoyloxy, etc.) and the like.

Examples of an optionally substituted thiol group include a mercapto group, an alkylthio group, a cycloalkylthio group, an alkenylthio group, an aralkylthio group, an arylthio group, a heteroarylthio group, a heteroarylalkylthio group, an acylthio group and the like.

Preferable examples of an alkylthio group include alkylthio groups having 1 to 10 carbons, such as methylthio, ethylthio, propylthio, isopropiothio, butylthio, isobutylthio, sec-butylthio, tert-butylthio, pentylthio, isopentylthio, neopentylthio, hexylthio, heptylthio, nonylthio and the like.

Preferable examples of a cycloalkylthio group include cycloalkylthio groups having 3 to 10 carbons, such as cyclobutylthio, cyclopenthylthio, cyclohexylthio and the like.

Preferable examples of an alkenylthio group include alkenylthio groups having 2 to 10 carbons, such as allylthio, crotylthio, 2-pentenylthio, 3-hexenylthio and the like.

Preferable examples of an aralkylthio group include aralkylthio groups having 7 to 20 carbons, such as a $C_{6-14}$arylthio group, in particular, phenyl-$C_{1-6}$alkylthio (e.g. benzylthio, phenethylthio, etc.), naphthyl-$C_{1-6}$alkylthio group and the like.

Preferable examples of an arylthio group include arylthio groups having 6 to 14 carbons optionally substituted with an alkyl group having 1 to 3 carbons, an alkoxy group having 1 to 3 carbons, halogen, a nitro group, a hydroxyl group or an amino group, such as phenylthio, naphthylthio, 4-chlorophenylthio and the like.

Examples of a heteroarylthio group include a mercapto group substituted with the above-mentioned aromatic heterocyclic group(s), and inter alia pyridylthio (e.g. 2-pyridylthio, 3-pyridylthio, etc.), imidazolylthio (2-imidazolylthio, etc.), triazolylthio (1,2,4-triazol-5-ylthio, etc.) and the like are preferable.

Examples of a heteroarylalkylthio group include the above-mentioned alkylthio groups substituted with the above-mentioned aromatic heterocyclic group. Preferable examples of a heteroarylthio group include pyridyl-$C_{1-6}$alkylthio group (e.g. 2-pyridylmethylthio, 3-pyridylmethylthio, etc.).

Preferable examples of an acylthio group include acylthio groups having 2 to 15 carbons, such as an alkanoylthio group having 2 to 7 carbons (e.g. acetylthio, propionylthio, butyrylthio, isobutyrylthio, etc.), $C_{6-14}$aryl-carbonylthio (e.g. benzoylthio, naphthoyltio, etc.) and the like.

Examples of an optionally esterified or amidated carboxyl group include a carboxyl group, an esterified carboxyl group and an amidated carboxyl group.

Examples of an esterified carboxyl group include an alkoxycarbonyl group, an aralkyloxycarbonyl group, an aryloxycarbonyl group, a heteroarylalkyloxycarbonyl group and the like.

Preferable examples of an alkoxycarbonyl group include alkoxycarbonyl groups having 2 to 7 carbons, such as methoxycarbonyl, ethoxycarbonyl, propoxycarbonyl, butoxycarbonyl and the like.

Preferable examples of an aralkyloxycarbonyl group include aralkyloxycarbonyl groups having 8 to 21 carbons, such as phenyl-$C_{2-7}$alkoxycarbonyl (e.g. benzyloxycarbonyl, etc.), naphthyl-$C_{2-7}$alkoxycarbonyl and the like.

Preferable examples of an aryloxycarbonyl group include aryloxycarbonyl groups having 7 to 15 carbons optionally substituted with an alkyl group having 1 to 3 carbons, an alkoxy group having 1 to 3 carbons, halogen, a nitro group, a hydroxyl group or an amino group, such as phenoxycarbonyl, p-tolyloxycabonyl and the like.

Examples of heteroarylalkyloxycarbonyl include the above-mentioned alkoxycarbonyl group substituted with the above-mentioned aromatic heterocyclic group(s). Preferable examples of a heteroarylalkyloxycarbonyl group include pyridyl-$C_{2-7}$alkoxycarbonyl group (e.g. 2-pyridylmethoxycarbonyl, 3-pyridylmethoxycarbonyl, etc.) and the like.

Examples of an amidated carboxyl group include a group represented by the formula: $-CON(R^6)(R^7)$ [wherein $R^6$ and $R^7$ may be the same or different, and denote a hydrogen atom, an optionally substituted hydrocarbon group or an optionally substituted heterocyclic group]. Examples of a hydrocarbon group in an optionally substituted hydrocarbon group denoted by $R^6$ or $R^7$ include an aliphatic hydrocarbon group, an alicyclic hydrocarbon group, and an aromatic "hydrocarbon group exemplified as a substituent on an aromatic heterocyclic group denoted by R. In addition, examples of a heterocyclic group in an optionally substituted heterocyclic group denoted by $R^6$ or $R^7$ include an aromatic heterocyclic group exemplified as a substituent on an aromatic heterocycle represented by R. Examples of a substituent on a hydrocarbon group or a heterocyclic group in $R^6$ or $R^7$ include 1 to 3 substituent(s) selected from a halogen atom (e.g. chlorine, fluorine, bromine, iodine, etc.) , an alkyl group having 1 to 6 carbons, an alkoxy group having 1 to 6 carbons and the like.

When in the general formula (I) a substituent on an aromatic heterocyclic group denoted by R, an aromatic azole group denoted by the formula:

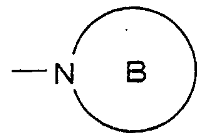

or ring A is an alicyclic hydrocarbon group, an aromatic hydrocarbon group, or an aliphatic hydrocarbon group substituted with aromatic hydrocarbon group(s), or an aromatic heterocyclic group, a non-aromatic heterocyclic group, or an aliphatic hydrocarbon group substituted with aromatic heterocyclic group(s), each of the alicyclic hydrocarbon group, the aromatic hydrocarbon group, the aromatic hydrocarbon group in an aliphatic hydrocarbon group substituted with aromatic hydrocarbon group(s), or the aromatic heterocyclic group, the non-aromatic hydrocarbon group, or the aromatic heterocyclic group in an aliphatic hydrocarbon group substituted with aromatic heterocyclic group(s) may further have 1 to 3 (preferably 1 or 2) substituent(s) at a replaceable place, and examples of such the substituent include an optionally substituted alkyl group having 1 to 6 carbons, an alkenyl group having 2 to 6 carbons, an alkynyl group having 2 to 6 carbons, a cycloalkyl group having 3 to 10 carbons, a cycloalkenyl group having 5 to 10 carbons, an aryl group having 6 to 14 carbons (e.g. phenyl, naphthyl, etc.), an aromatic heterocyclic group (e.g. thienyl, furyl, pyridyl, oxazolyl, thiazolyl, tetrazolyl, etc.), a non-aromatic heterocyclic group (e.g. tetrahydrofuryl, morpholinyl, piperidyl, pyrrolidyl, piperazinyl, etc.), an aralkyl group having 7 to 20 carbons (e.g. phenyl-$C_{1-6}$alkyl group, naphthyl-$C_{1-6}$alkyl group, etc.), an amino group, a N-mono($C_{1-6}$)alkylamino group, a N, N-di($C_{1-6}$)alkylamino group, an acylamino group having 2 to 7 carbons (e.g. $C_{2-7}$alkanoylamino group such as acetylamino, propionylamino, and benzoyl amino group, etc.), an amidino group, an acyl group having 2 to 7 carbons (e.g. alkanoyl group having 2 to 7 carbons, benzoyl group, etc.), a carbamoyl group, a N-mono($C_{1-6}$)alkylcarbamoyl group, a N, N-di ($C_{1-6}$) alkylcarbamoyl group, a sulfamoyl group, a N-mono($C_{1-6}$)alkylsulfamoyl group, a N, N-di($C_{1-6}$)alkylsulfamoyl group, a carboxyl group, an alkoxycarbonyl group having 2 to 7 carbons, an aralkyloxycarbonyl group having 8 to 21 carbons (e.g. phenyl-$C_{2-7}$alkoxycarbonyl, naphthyl-$C_{2-7}$alkoxycarbonyl, etc.), a hydroxyl group, an optionally substituted alkoxy group having 1 to 6 carbons, an alkenyloxy group having 2 to 6 carbons, a cycloalkyloxy group having 3 to 10 carbons, a cycloalkenyloxy group having 5 to 10 carbons, an aralkyloxy group having 7 to 20 carbons (e.g. phenyl-$C_{1-6}$alkoxy group, naphthyl-$C_{1-6}$alkoxy group, etc.), an aryloxy group having 6 to 14 carbons (e.g. phenoxy, naphthyloxy, etc.), a mercapto group, an alkylthio group having 1 to 6 carbons, a cycloalkylthio group having 3 to 10 carbons, an aralkylthio group having 7 to 20 carbons (e. g. phenyl-$C_{1-6}$alkyl group, naphthyl-$C_{1-6}$alkylthio group, etc.), an arylthio group having 6 to 14 carbons (e.g. phenylthio, naphthylthio group, etc.), a sulfo group, a cyano group, an azido group, a nitro group, a nitroso group, a halogen atom (e.g. fluorine, chlorine, bromine, iodine, etc. ) and the like.

Examples of the substituent in the above-mentioned optionally substituted alkoxy group having 1 to 6 carbons and the optionally substituted alkyl group having 1 to 6 carbons include 1 to 3 substituent(s) selected from a halogen atom (e.g. fluorine, chlorine, bromine, iodine, etc.), a hydroxyl group, an alkoxy group having 1 to 6 carbons and the like.

Examples of a substituted alkoxy group having 1 to 6 carbons include trifluoromethoxy, difluoromethoxy, 2,2,2-trifluoroethoxy, 1,1-difluoroethoxy and the like.

Examples of a substituted alkyl group having 1 to 6 carbons include trifluoromethyl, difluoromethyl, 2,2,2-trifluoroethyl, trichloromethyl, hydroxymethyl, methoxymethyl, ethoxyethyl, 2-methoxyethyl, 2,2-dimethoxyethyl and the like.

When in the general formula (I) a substituent on an aromatic heterocyclic group denoted by R, an aromatic azole group denoted by the formula:

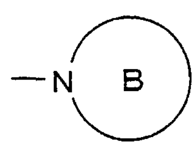

or a ring A is an aliphatic hydrocarbon.group, an aliphatic hydrocarbon group substituted with an aromatic hydrocarbon group, or an aliphatic hydrocarbon group substituted with aromatic heterocyclic group(s), each of the aliphatic hydrocarbon group, the aliphatic hydrocarbon group in an aliphatic hydrocarbon group substituted with aromatic hydrocarbon group(s), or the aliphatic hydrocarbon group in an aliphatic hydrocarbon group substituted with aromatic heterocyclic group(s) may have 1 to 3 (preferably 1 or 2) substituent(s) at a replaceable position, and examples of such the substituent include a non-aromatic heterocyclic group (e.g. tetrahydrofuryl, morpholinyl, piperidyl, pyrrolidyl, piperazinyl, etc.), an amino group, a N-mono($C_{1-6}$) alkylamino group, a N, N-di($C_{1-6}$)alkylamino group, an acylamino group having 2 to 7 carbons (e.g. $C_{2-8}$alkanoylamino group such as acetylamino and propionylamino, benzoylamino group, etc.), an amidino group, an acyl group having 2 to 7 carbons (e.g. alkanoyl group having 2 to 7 carbons, benzoyl group, etc.), a carbamoyl group, a N-mono($C_{1-6}$) alkylcarbamoyl group, a N, N-di ($C_{1-6}$) alkylcarbamoyl group, a sulfamoyl group, a N-mono($C_{1-6}$)alkylsulfamoyl group, a N, N-di($C_{1-6}$)alkylsulfamoyl group, a carboxyl group, an alkoxycarbonyl group having 2 to 7 carbons, an aralkyloxycarbonyl group having 8 to 21 carbons (e.g. phenyl-$C_{2-7}$alkoxycarbonyl group, naphthyl-$C_{2-7}$alkoxycarbonyl group, etc.), a hydroxyl group, an optionally substituted alkoxy group having 1 to 6 carbons, an alkenyloxy group having 2 to 6 carbons, a cycloalkyloxy group having 3 to 10 carbons, a cycloalkenyloxy group having 5 to 10 carbons, an aralkyloxy group having 7 to 20 carbons (e.g. phenyl-$C_{1-6}$alkoxy group, naphthyl-$C_{1-6}$alkoxy group etc.), an aryloxy group having 6 to 14 carbons (e.g. phenoxy, naphthyloxy, etc.), a mercapto group, an alkylthio group having 1 to 6 carbons, a cycloalkylthio group having 3 to 10 carbons, an aralkylthio group having 7 to 20 carbons (e.g. phenyl-$C_{1-6}$alkyl group, naphthyl $C_{1-6}$alkylthio group, etc.), an arylthio group having 6 to 14 carbons (e.g. phenylthio, naphthylthio, etc), a sulfo group, a cyano group, an azido group, a nitro group, a nitroso group, a halogen atom(e.g. fluorine, chlorine, bromine, iodine, etc.)

Examples of the substituent in the above-mentioned optionally substituted alkoxy group having 1 to 6 carbons include 1 to 3 substituent(s) selected from a halogen atom (e.g. fluorine, chlorine, bromine, iodine, etc.), a hydroxyl group, an alkoxy group having 1 to 6 carbons and the like.

Examples of the above-mentioned substituted alkoxy group having 1 to 6 carbons include trifluoromethoxy, difluoromethoxy, 2,2,2-trifluoroethoxy, 1,1-difluoroethoxy and the like.

As R group, an oxazolyl group, a benzoxazolyl group or a thiazolyl group is preferable, each of which may be substituted with 1 to 2 substituent(s) selected from (i) an aryl group (e.g. phenyl group, naphthyl group) optionally substituted with 1 or 2 substituent(s) selected from a hydroxyl group, an alkoxy group (e.g. $C_{1-6}$alkoxy group), an arylalkoxy group (e.g. phenyl-$C_{1-6}$alkoxy group), an alkyl group (e.g. $C_{1-6}$alkyl group), a cyano group, a halogen atom and a tetrazolyl group, (ii) an alkyl group (e.g. $C_{1-10}$alkyl group), (iii) a hydroxyalkyl group (e.g. hydroxyl-$C_{1-10}$alkyl group), (iv) an alkoxycarbonylalkyl group (e.g. $C_{2-7}$alkoxycarbonyl-$C_{1-10}$alkyl group), (v) an alkyl group substituted with 1 or 2 aryl group(s) (e.g. $C_{1-6}$alkyl group substituted with 1 or 2 phenyl group(s)), (vi) an alkenyl group substituted with 1 or 2 aryl group(s) (e.g. $C_{2-6}$alkenyl group substituted with 1 or 2 phenyl group(s)), (vii) a cycloalkyl group (e.g. $C_{3-10}$cycloalkyl group), (viii) a partially saturated naphthyl group (e.g. dihydronaphthyl group), (ix) a thienyl group or a furyl group which may be each substituted with 1 or 2 substituent(s) selected from a hydroxyl group, an alkoxy group, an arylalkoxy group, an alkyl group, a cyano group, an allyl group and a halogen atom, (x) a benzofuranyl group and (xi) a benzothienyl group. An oxazolyl group substituted with arylalkenyl group(s) (e.g. phenyl-$C_{2-6}$alkenyl group) and an oxazolyl group substituted with an arylalkoxyl-aryl group (e.g. phenyl-$C_{1-6}$alkoxy-phenyl group) are more preferable as R group.

As an aromatic azole group denoted by the formula:

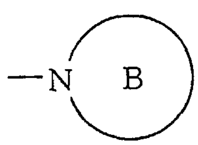

a pyrrolyl group, an imidazolyl group, a pyrazolyl group, a triazolyl group, a tetrazolyl group or a benzimidazolyl group is preferable, each of which may be optionally substituted with 1 or 2 substituent(s) selected from (i) an alkyl group (e.g. $C_{1-10}$alkyl group), (ii) an aryl group (e.g. phenyl group), (iii) a hydroxyalkyl group (e.g. hydroxyl-$C_{1-10}$alkyl group), (iv) a carboxyl group, (v) an alkoxycarbonyl group (e.g. $C_{2-7}$alkoxycarbonyl group) and (vi) a carbamoyl group, and an imidazolyl group and a triazolyl group are more preferable.

A ring A forms an optionally substituted benzene ring or an optionally substituted pyridine ring depending on a kind of Y (CH or N). A preferable example includes an optionally substituted benzene ring, and more preferable examples include a benzene ring or a pyridine ring, optionally substituted with 1 or 2 $C_{1-6}$alkoxy group(s).

Preferable examples of a group represented by the formula:

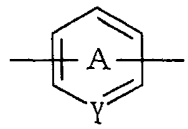

include a group represented by the formula:

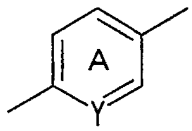

or

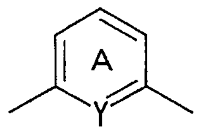

, and most preferable examples include a 1,3-phenylene group and a 1,4-phenylene group.

X denotes an oxygen atom (0), an optionally oxidized sulfur atom [S(O)$_k$ (k denotes an integer of 0 to 2)], -C(=0) -or -CH(OH)-, and preferable examples include an oxygen atom and the like.

p denotes an integer of 0 to 10, preferable examples include an integer of 0 to 6, and more preferable examples include of an integer of 3 to 5.

q denotes an integer of 1 to 5, and preferable examples include 1.

As a specific example of Compound (I), the compounds prepared in Examples in JP-A No. 11-60571 are used and, inter alia, (i) 1-[4-[4-[2-[(E)-2-phenylethenyl]-4-oxazolylmethoxy]phenyl]butyl]-1,2,4-triazole, (ii) 4-[4-[4-(1-imidazolyl)butyl]phenoxymethyl]-2-[(E)-2-phenylethenyl]oxazole, (iii) 4-[4-[3-(1-imidazolyl)propyl]phenoxymethyl]-2-[(E)-2-phenylethenyl]oxazole, (iv) 4-[3-[3-(1-imidazolyl)propyl]phenoxymethyl]-2-[(E)-2-phenylethenyl]oxazole, (v) 2-(4-benzyloxyphenyl)-4-[4-[4-[3-(1-imidazolyl}propyl]phenoxymethyl]oxazole, (vi) 4-[4-[3-(1-imidazolyl)propyl]phenoxymethyl]-2-(2-thienyl)oxazole, (vii) 4-[4-[3-(1-imidazolyl)propyl]phenoxymethyl]-2-(5-methyl-2-thienyl)oxazole, (viii) 2-(5-chloro-2-thienyl)-4-[4-[3-(1-imidazolyl)-propyl]phenoxymethyl]oxazole, (ix) 4-[4-[3-(1-imidazolyl)propyl]phenoxymethyl]-2-(2-thienyl)thiazole, and (x) 5-[4-[3-(1-imidazolyl)propyl]phenoxymethyl]-2-(2-thienyl)benzoxazole are preferable.

In addition, as the above-mentioned Compound (I), for example, Compound (I') represented by the formula:

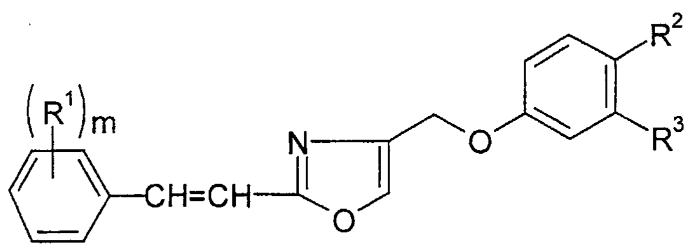

( I ' )

[wherein m denotes 1 or 2, $R^1$ denotes halogen or optionally halogenated $C_{1-2}$alkyl, one of $R^2$ and $R^3$ denotes a hydrogen atom, and the other denotes a group represented by the formula:

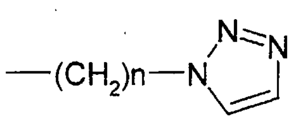

or

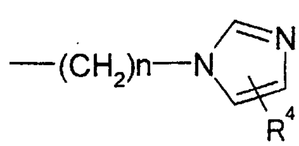

wherein n denotes 3 or 4, and $R^4$ denotes a $C_{1-4}$ alkyl group substituted with 1 or 2 hydroxy group(s)] is preferable.

Examples of the "halogen" denoted by $R^1$ in the above formula (I') include fluoro, chloro, bromo and iodo. Among them, fluoro is preferable.

Examples of the "halogen" in the "optionally halogenatged $C_{1-2}$ alkyl" denoted by $R^1$ include fluoro, chloro, bromo and iodo. Among them, fluoro is preferable.
Examples of the "$C_{1-2}$ alkyl" in the "optionally halogenated $C_{1-2}$ alkyl" denoted by $R^1$ include methyl and ethyl, and methyl is preferable.
The "$C_{1-2}$ alkyl" may have 1 to 3, preferably 2 or 3 above-mentioned halogen(s) at a replaceable position, and when the number of the halogen is 2 or more, respective halogens may be the same or different.
Examples of the "optionally halogenated $C_{1-2}$ alkyl" include methyl, ethyl and trifluoromethyl.
As $R^1$, halogen or halogenated $C_{1-2}$ alkyl is preferable, and fluoro and trifluoromethyl are more preferable.
When m is 2, each $R^1$ may be different.
A group represented by the formula:

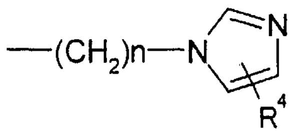

[wherein $R^4$ denotes the same meaning as that described above] denoted by $R^2$ or $R^3$ is preferably a group represented by the formula:

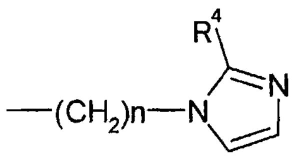

[wherein R4 denotes the same meaning as that described above].
Examples of the "$C_{1-4}$ alkyl group" in the "$C_{1-4}$ alkyl group substituted with 1 or 2 hydroxy group(s)" denoted by $R^4$ include methyl, ethyl, propyl, isopropyl, butyl, isobutyl, sec-butyl, and tert-butyl. Inter-alia, ethyl and propyl are preferable.
Specific Examples of the "$C_{1-4}$ alkyl group substituted with 1 or 2 hydroxy group(s)" include 2-hydroxyethyl, 2,3-dihydroxypropyi and 1,3-dihydroxypropyl. Inter alia, preferable is 2,3-dihydroxypropyl.
In the above formula, the case where $R^2$ is a group represented by the formula:

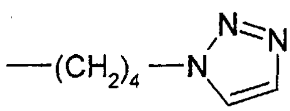

and $R^3$ is a hydrogen atom is preferable.
The case where $R^2$ is a hydrogen atom and $R^3$ is a group represented by the formula:

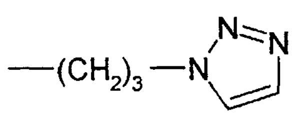

is also preferable.
The case where $R^2$ is a group represented by the formula:

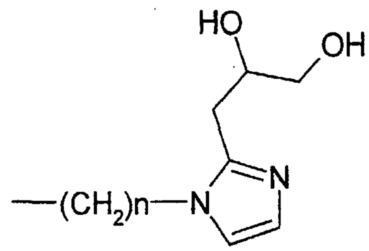

[wherein n denotes the same meaning as that described above] and $R^3$ is a hydrogen atom is also preferable, more preferably, n is 4.

Preferable examples of Compound (I') include a compound represented by the formula:

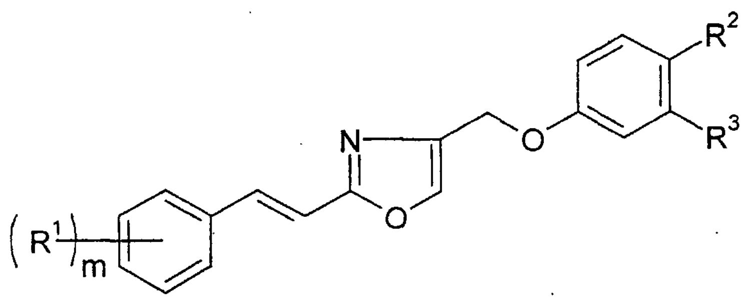

[wherein respective symbols denote the same meanings as those described above] or a salt thereof.

Among Compound (I), a compound wherein m is 1, $R^1$ is 4-trifluoromethyl, $R^2$ is a group represented by the formula:

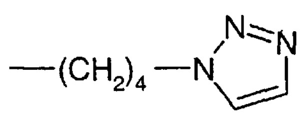

and $R^3$ is a hydrogen atom, or a salt is preferable.

Examples of Compound (I') include:

(i)1-(4-{4-[(2-{(E)-2-[4-(trifluoromethyl)phenyl]ethenyl}-1,3-oxazole-4-yl)methoxy]phenyl}butyl)-1H-1,2,3-triazole,
(ii)2-(3-{3-[(2-{(E)-2-[4-(trifluoromethyl)phenyl]ethenyl}-1,3-oxazole-4-yl)methoxy]phenyl}propyl)-1H-1,2,3-troazole,
(iii)3-(1-{4-[4-({2-[(E)-2-(2,4-difluorophenyl)ethenyl]-1,3-oxazole-4-yl}methoxy)phenyl]butyl}-1H-imidazol-2-yl)-1,2-propanediol.

As a salt of Compound (I) of the present invention, a pharmaceutically acceptable salt is preferable, and examples thereof include salts with inorganic bases, salts with organic bases, salts with inorganic acids, salts with organic acids, and salts with basic or acidic amino acids. Preferable examples of salts with inorganic bases include alkali metal salts such as sodium salt and potassium salt; alkaline earth metal salts such as calcium salt and magnesium salts; aluminium salts; ammonium salts. Preferable examples of salts with organic bases include salts with trimethylamine, triethylamine, pyridine, picoline, ethanolamine, diethanolamine, triethanolamine, dicyclohexylamine, and N, N'-dibenzylethylenediamine. Preferable examples of salts with inorganic acids include salts with hydrochloric acid, hydrobromic acid, nitric acid, sulfuric acid and phosphoric acid. Preferable examples of salts with organic acids include salts with formic acid, acetic acid, trifluoroacetic acid, fumaric acid, oxalic acid, tartaric acid, maleic acid, citric acid, succinic acid, malic acid, methanesulfonic acid, benzenesulfonic acid, and p-toluenesulfonic acid. Preferable examples of salts with basic amino acids include salts with arginine, lysine, and ornithine, and preferable examples of salts with acidic amino acids include salts with aspartic acid and glutamic acid.

Compound (I) may include two kinds of isomers, (Z)-ethenyl isomer and (E)-ethenyl isomer, and these isomers

alone and a mixture thereof are included in the present invention.

In addition, when Compound (I) has an asymmetric carbon, optical isomers occur. The isomer alone and a mixture thereof are included in the present invention.

Compound (I) or a salt thereof is obtained by a known per se method, for example, by a method described in JP-A No. 11-60571.

In particular, Compound (I') or a salt thereof is obtained, for example, by a method denoted by the following reaction formulas A to H.

Respective symbols of compounds in a schematic view of the following reaction formulas denote the same meaning as those described above. Compounds in the following reaction formulas may include salts thereof.

Reaction formula A

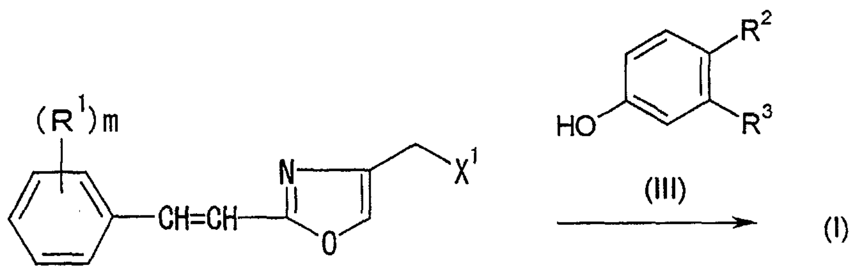

(II)

Examples of a "leaving group" denoted by $X^1$ include halogen (e.g. chloro and bromo) and a group represented by the formula: $-OSO_2R^5$ [wherein $R^5$ denotes alkyl or aryl optionally having a substituent].

Examples of the "alkyl" denoted by $R^5$ include $C_{1-6}$ alkyl such as methyl, ethyl and propyl.

Examples of the "aryl" in the "aryl optionally having a substituent" denoted by $R^5$ include $C_{6-14}$ aryl such as phenyl.

Examples of the "substituent" in the "aryl optionally having a substituent" denoted by $R^5$ include $C_{1-6}$ alkyl such as methyl, ethyl and propyl.

Examples of the "aryl optionally having a substituent" include phenyl (e.g. p-tolyl) optionally having $C_{1-6}$ alkyl.

Compound (II) is reacted with Compound (III) to obtain Compound (I').

In the present reaction, usually, Compound (II) is condensed to Compound (III) in the presence of a base.

Examples of the "base" include hydroxides of an alkali metal or an alkaline earth metal (e.g. sodium hydroxide and potassium hydroxide), carbonates of an alkali metal or an alkaline earth metal (e.g. sodium bicarbonate, sodium carbonate, potassium carbonate), amines (e.g. pyridine, triethylamine, N,N-dimethylaniline), hydrides of an alkali metal or an alkaline earth metal (e.g. sodium hydride, potassium hydride, calcium hydride), and lower alkoxides of an alkali metal or an alkaline earth metal (e.g. sodium methoxide, sodium ethoxide, potassium tert-butoxide).

A preferable amount of the "base" to be used is about 1 to 5 mole equivalent relative to Compound (II).

A preferable amount of "Compound (III)" to be used is about 0.5 to 5 mole equivalent relative to Compound (II).

Advantageously, this reaction is performed in the presence of a solvent having no influence on the reaction. The solvent is not particularly limited as far as the reaction progresses, however for example aromatic hydrocarbons, ethers, ketones, halogenated hydrocarbons, amides, sulfoxides or a mixture of two or more of them are used.

A reaction temperature is usually -50 to +150°C, preferably about -10 to +100°C. A reaction time is usually 0.5 to 48 hours.

Compound (II) can be prepared by a known per se method or according to a similar method. For example, Compound (IIa) wherein X is chloro can be prepared by a method denoted by the following reaction formula B.

Reaction formula B

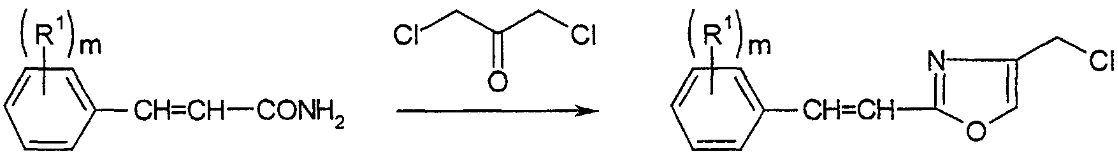

(IV) (IIa)

Compound (IV) and 1,3-dichloroacetone are subjected to a condensing and dehydrating reaction to obtain Compound (IIa) .

As for Compound (IV), when it is commercially available, a sold product may be used directly, or Compound (IV) may be prepared by a known per se method or a similar method.

An amount of the "1,3-dichloroacetone" to be used is about 1 equivalent to large excess amount (solvent amount) relative to Compound (IV).

Advantageously, this reaction is performed without a solvent or in the presence of a solvent having no influence on the reaction. The solvent is not particularly limited as far as the reaction progresses however aromatic hydrocarbons, ethers, ketones, halogenated hydrocarbons or a mixture of two or more of them are used.

A reaction temperature is usually 50 to 150°C, preferably about 60 to 120°C. A reaction time is usually 0.5 to 48 hours.

The product may be used directly as the reaction solution, or as the crude product in the next reaction, however may be isolated from the reaction mixture according to the conventional method.

Among Compound (III), Compound (IIIa) wherein $R^3$ is a hydrogen atom can be prepared by a known per se method or a similar method. For example, it can be prepared by a method denoted by the following reaction formula C.

Reaction formula C

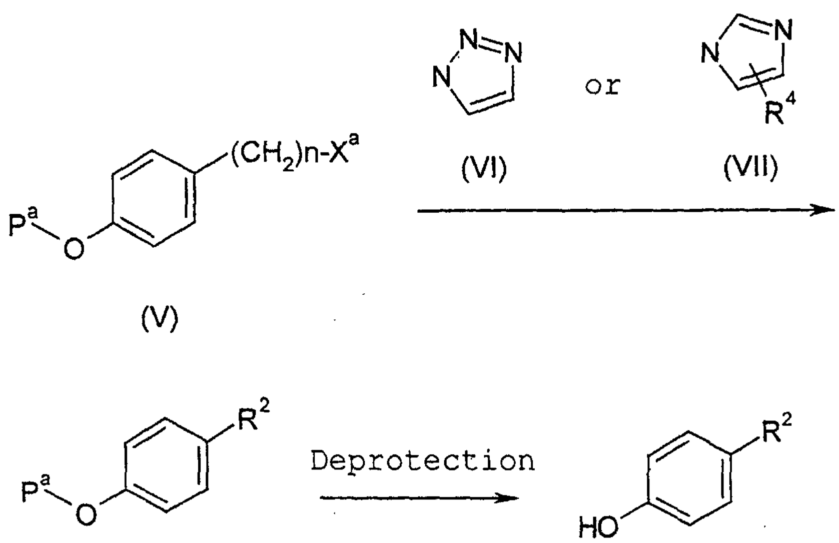


(VI) (VII)

(V)

(VIII) (IIIa)

In the above formula, $P^a$ denotes a hydrogen atom or a protecting group, and $X^a$ denotes a leaving group.

Examples of the "protecting group" denoted by $P^a$ include alkyl (e.g. $C_{1-6}$ alkyl such as methyl, ethyl) phenyl-$C_{1-6}$ alkyl (e.g. benzyl), $C_{1-6}$ alkyl-carbonyl, and alkyl-substituted silyl (e.g. trimethylsilyl, tertbutyldimethylsilyl).

Examples of the "leaving group" denoted by $X^a$ include the same "leaving groups" as those denoted by $X^1$.

Compound (V) is condensed to compound (VI) or Compound (VII) to obtain Compound (VIII), which is subjected to a deprotecting reaction, if necessary, to obtain Compound (IIIa).

As for Compound (V), Compound (VI) and Compound (VII), when they are commercially available, may be used directly, or they may be prepared by a known per se method or a similar method.

The "condensing reaction" is usually performed in the presence of a base in a solvent having no influence on the reaction.

As a "base", the base described in detail for the above reaction formula A is used.

A preferable amount of the "base" to be used is about 1 to 5 mole equivalent relative to Compound (V).

A preferable amount of the "Compound (VI) or Compound (VII)" to be used is about 0.5 to 5 mole equivalent relative to Compound (V).

The solvent is not particularly limited as far as the reaction progresses, however for example aromatic hydrocarbons, ethers, ketones, halogenated hydrocarbons, amides, sulfoxides or a mixture of two or more of them are used.

A reaction temperature is usually -50 to +150°C, preferably about -10 to +100°C. A reaction time is about 0.5 to 48 hours.

The resulting Compound (VIII) may be used dierctly as the reaction solution, or as the crude product in the next reaction, however may be isolated from the reaction mixture according to the conventional method.

The "deprotecting reaction" can be appropriately selected among the conventional methods.

For example, when $P^a$ is alkyl, Compound (VIII) is subjected to a treatment with an acid (e.g. mineral acid such as hydrobromic acid, Lewis acid such as titanium tetrachloride).

For example, when $P^a$ is phenyl-$C_{1-6}$ alkyl, Compound (VIII) is subjected to a hydrogenating reaction.

For example, when $P^a$ is alkyl-substituted silyl, Compound (VIII) is reacted with a fluoride (e.g. tetrabutylammonium fluoride).

The resulting Compound (IIIa) may be used directly as the reaction solution, or as the crude product in the next reaction, however may be isolated from the reaction mixture according to the conventional method.

Among Compound (III), Compound (IIIb) wherein $R^2$ is a hydrogen atom can be prepared by a known per se method or a similar method. For example, it can be prepared by a method denoted by the following reaction formula D.

Reaction formula D

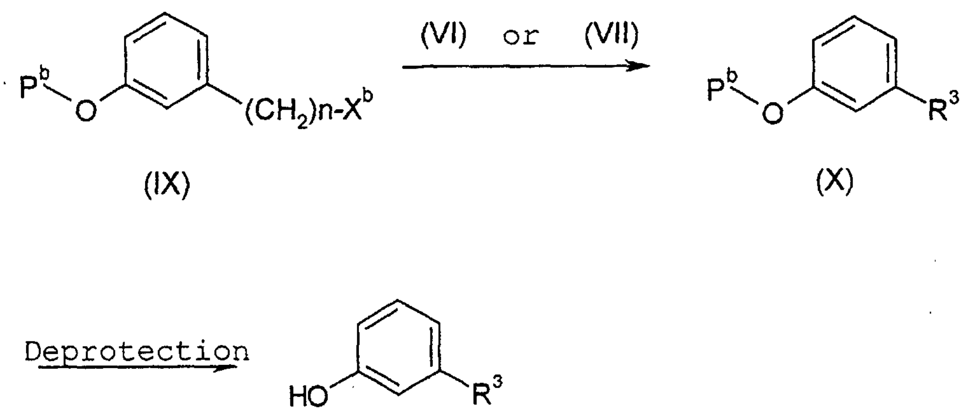


(IX) (X)

(IIIb)

In the above formula, $p^b$ denotes a hydrogen atom or a protecting group, and $X^b$ denotes a leaving group.

Examples of the "protecting group" denoted by $P^b$ include the same "protecting groups" as those denoted by $P^a$.

Examples of the "leaving group" denoted by $X^b$ include the same "leaving groups" as those denoted by $X^1$.

By a method similar to the method described in the above-mentioned reaction formula C, Compound (IX) is condensed to Compound (VI) or Compound (VII) to obtain Compound (X), which is subjected to a deprotecting reaction, if necessary, to obtain Compound (IIIb).

As for Compound (IX), when it is commercially available, it may be used directly, or Compound (IX) may be

prepared by a known per se method or a similar method.

Among Compound (I'), Compound (I'a) wherein $R^3$ is a hydrogen atom may be also prepared by a method described in the following reaction formula E.

Reaction formula E

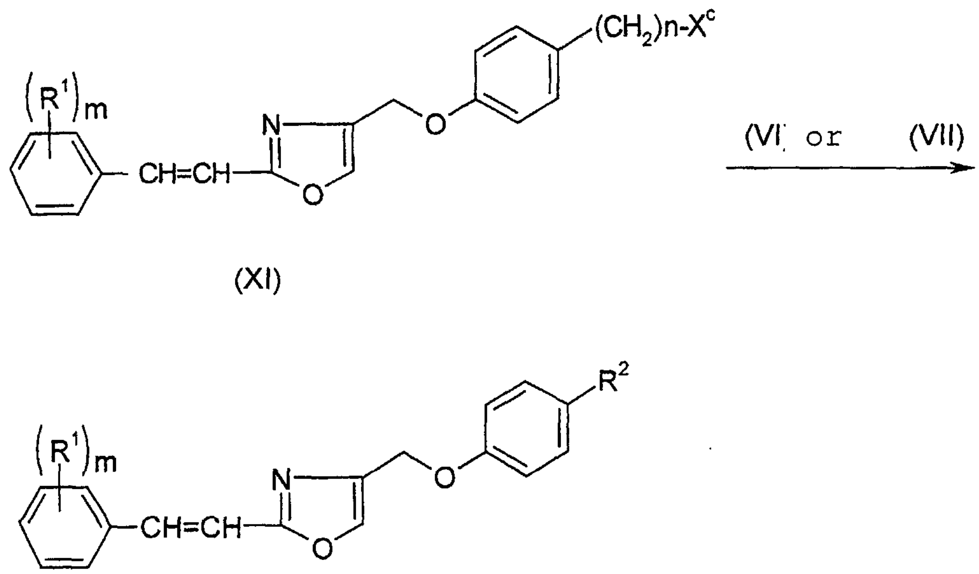


(XI)

(I'a)

In the above formula, $X^c$ denotes a leaving group.

Examples of the "leaving group" denoted by $X^c$ include the same "leaving groups" as those denoted by $X^1$.

Compound (XI) and Compound (VI) or Compound (VII) are reacted to obtain Compound (Ia).

In the present reaction, usually, Compound (XI) is condensed to Compound (VI) or Compound (VII) in the presence of a base.

As the "base", the base described in detail for the above-mentioned reaction formula A is used.

A preferable amount of the "base" to be used is about 1 to 5 mole equivalent relative to compound (XI).

Preferable amounts of the "Compound (VI)" and the "Compound (VII)" are about 0.5 to 5 mole equivalent, respectively, relative to Compound (XI).

Advantageously, this reaction is performed in the presence of a solvent having no influence on the reaction. The solvent is not particularly limited as far as the reaction progresses, however for example aromatic hydrocarbons, ethers, ketones, halogenated hydrocarbons, amides, sulfoxides or a mixture of two or more of them are used.

A reaction temperature is usually -20 to +150°C, preferably about -10 to +100°C. A reaction time is usually 0.5 to 48 hours.

Compound (XI) can be prepared by a known per se method or a similar method. For example, can be prepared by a method denoted by the following reaction formula F.

Reaction formula F

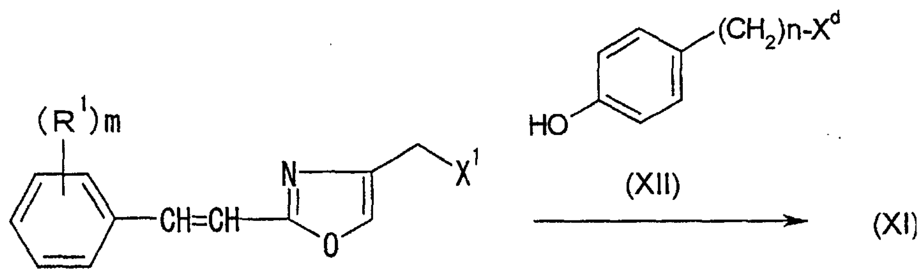


(II)

In the above formula, $X^d$ denotes a leaving group.

Examples of the "leaving group" denoted by $X^d$ include the same "leaving groups" as those denoted by $X^1$, preferably, leaving groups having the lower reactivity than that of $X^1$.

According to a method similar to the method described in the above reaction formula A, Compound (II) and Compound (XII) are reacted to obtain Compound (XI).

As for Compound (XII), when it is commercially available, it may be used directly, or Compound (XII) may be prepared by a known per se method or a similar method.

Among Compound (I'), Compound (I'b) wherein $R^2$ is a hydrogen atom may be also prepared by a method described in the following reaction formula G.

Reaction formula G

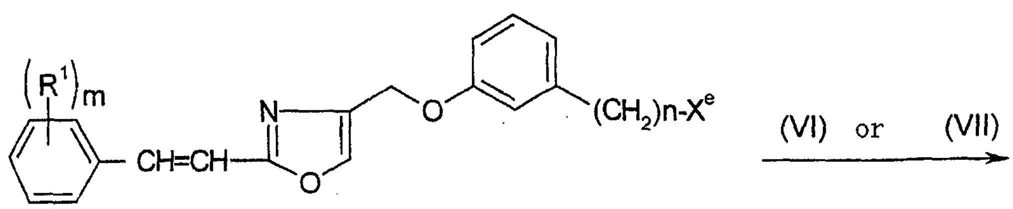


(XIII)

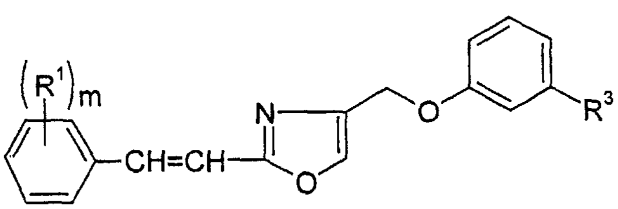

(I'b)

In the above formula, $X^e$ denotes a leaving group.

Examples of the "leaving group" denoted by $X^e$ include the same "leaving groups" as those denoted by $X^1$.

By a method similar to the method described in the above reaction formula E, Compound (XIII) is reacted with Compound (VI) or Compound (VII) to obtain Compound (I'b).

Compound (XIII) can be prepared by a known per se method or a similar method. For example, can be prepared by a method denoted by the following reaction formula H.

Reaction formula H

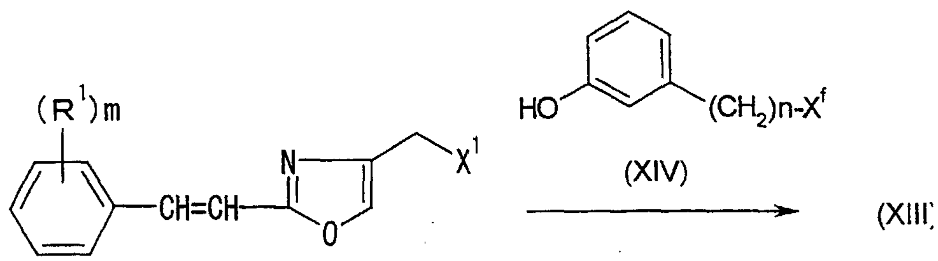


In the above formula, $X^f$ denotes a leaving group.

Examples of the "leaving group" denoted by $X^f$ include the same "leaving groups" as those denoted by $X^1$, preferably, leaving groups having the lower reactivity than that of $X^1$.

By a method similar to the method described in the above reaction formula A, Compound (II) is reacted with Compound (XIV) to obtain Compound (XIII).

As for Compound (XIV), when it is commercially available, it may be used directly, or Compound (XIV) may be prepared by a known per se method or a similar method.

As the above "aromatic hydrocarbons", for example, benzene, toluene, xylene, are used.

As the above "ethers", for example, tetrahydrofuran and dioxane are used.

As the above "ketones", for example, acetone and 2-butanone are used.

As the above "halogenated hydrocarbons", for example, chloroform and dichloromethane are used.

As the above "amides", for example, N,N-dimethylformamide is used.

As the above "sulfoxides", for example, dimethyl sulfoxide is used.

When the product is obtained as a free compound in the above-mentioned respective reactions, the free compound can be converted into a salt thereof according to the conventional method. Also, when the product is obtained as a salt, it can be converted into a free compound according to the conventional method.

When amino ($NH_2$), hydroxyl (OH), carboxyl (COOH) and the like are contained in a substituent in the above reaction, compounds in which protected these groups are protected may be used as a raw material and, after the reaction, a protecting group may be removed by a known per se method to obtain an end product. Examples of a protecting group for an amino include acyl (e.g. $C_{1-6}$ alkyl-carbonyl such as acetyl and the like; benzyloxycarbonyl; $C_{1-6}$ alkoxy-carbonyl such as tert-butoxycarbonyl and the like; phthaloyl; formyl, etc.). Examples of a protecting group for a hydroxy include $C_{1-6}$ alkyl (e.g. methyl, ethyl, etc.), phenyl-$C_{1-6}$ alkyl (e.g. benzyl, etc.), $C_{1-6}$ alkyl-carbonyl (e.g. acetyl, etc.), benzoyl, and alkyl-substituted silyl (e.g trimethylsilyl, tertbutyldimethylsilyl, etc.). Examples of a protecting group for a carboxyl include $C_{1-6}$ alkyl (e.g. methyl, ethyl, etc.), and phenyl-$C_{1-6}$ alkyl (e.g. benzyl, etc.).

Compound (I') thus obtained [including (I'a) and (I'b)] can be isolated and purified by a known per se means for separation, such as concentration, concentration under reduced pressure, solvent extraction, crystallization, recrystallization, transfer dissolution and chromatography.

When Compound (I') is obtained as a free compound, it can be converted into an end salt by a known per se method or a similar method. Conversely, when obtained as a salt, it can be converted into a free compound or other desired salt by a known per se method or a similar method.

Compound (I) may be a hydrate or a non-hydrate.

When Compound (I) is obtained as a mixture of optical active isomers, the mixture can be separated into a (R) isomer or a (S) isomer of interest by a known per se optical resolving means.

Compound (I) may be labeled with an isotope (e.g. $^3H$, $^{14}C$, etc.).

A prodrug of Compound (I) or a salt thereof (abbreviated as Compound (I)) may be a compound which is converted into Compound (I) by a reaction with enzymes or a stomach acid under the physiological conditions in a living body, that is, a compound which is changed into Compound (I) by enzymatic oxidation, reduction, hydrolysis or the like, or a compound which is changed into Compound (I) by hydrolysis with a stomach acid.

Examples of a prodrug of Compound (I) include a compound in which the amino group of Compound (I) is acylated, alkylated or phosphorylated (e.g. a compound in which the amino group of Compound (I) is eicosanoylated, alanylated, pentylaminocarbonized, (5-methyl-2-oxo-1,3-dioxolen-4-yl)methoxycarbonized, tetrahydrofuranylated, pyrrolidylmethylated, pivaloyloxymethylated, tert-butylated, etc.); a compound in which the hydroxyl group of Compound (I) is acylated, alkylated, phosphorylated or borated (e.g. a compound in which the hydroxyl group of Compound (I) is acetylated, palmitoylated, propanoylated, pivaloylated, succinated, fumarized, alanylated, dimethylaminomethylcarbonized, etc.); a compound in which the carboxyl group of Compound (I) is esterified or amidated (e.g. a compound

in which the carboxyl group of Compound (I) is ethylesterified, phenylesterified, carboxymethylesterified, dimethylaminomethylesterified, pivaloyloxymethylesterified, ethoxycarbonyloxyethylesterified, phthalizylesterified, (5-methyl-2-oxo-1,3-dioxolen-4-yl)methylesterified, cyclohexyloxycarbonylethylesterified, methylamidated, etc.). These compounds can be prepared from Compound (I) by a known per se method.

In addition, a prodrug of Compound (I) may be a compound which is changed into Compound (I) under the physiological conditions, described in pages 163-198, "Development of Medicaments" vol.7, Molecular Design published by Hirokawa Shoten in 1990.

Compound (I) or a salt thereof or a prodrug thereof has the tyrosinekinase inhibitory activity, and can be used for preventing or treating tyrosinekinase dependent diseases in a mammal. Tyrosinekinase dependent disease includes those associated with hyper-proliferation of cells due to abnormal tyrosinekinase enzyme activity. Further, since Compound (I) or a salt thereof or a prodrug thereof specifically inhibits HER2 tyrosinekinase, it is also useful as a treating agent for inhibiting proliferation of cancer expressing HER2, or as an agent for preventing change of a hormone dependent cancer to a hormone non-dependent cancer.

That is, Compound (I) or a salt thereof or a prodrug thereof can be used as a safe agent for preventing or treating diseases due to abnormal cell proliferation such as various cancers (inter alia, breast cancer, prostate cancer, pancreas cancer, stomach cancer, lung cancer, colon cancer, rectum cancer, esophagus cancer, duodenum cancer, tang cancer, pharynx cancer, brain tumor, schwannoma, non-small cell lung cancer, lung small cell cancer, liver cancer, renal cancer, bile duct cancer, corpus uteri cancer, cervix uteri cancer, ovary cancer, bladder cancer, skin cancer, vessel tumor, malignant lymphoma, malignant melanoma, thyroid gland cancer, bone tumor, hemangiofibroma, retinal sarcoma, penis cancer, infant solid cancer, Kaposi's sarcoma, Kaposi's sarcoma derived from AIDS, the upper jaw cave tumor, fibrous histiocytoma, smooth muscle sarcoma, striated muscle sarcoma, leukemia etc.), atherosclerosis, vascularization (e.g. vascularization associated with growth of solid cancer and sarcoma, vascularization associated with metastasis of tumor, and vascularization associated with diabetic retinopathy), and viral disease (HIV infection etc.), and can be used alone or together with other agents which are effective for these diseases.

Tyrosinekinase dependent diseases include cardiovascular diseases associated with the abnormal tyrosinekinase enzyme activity. Therefore, Compound (I) or a salt thereof or a prodrug thereof can be also used as an agent for preventing or treating cardiovascular disease such as restenosis.

As a hydrophilic polymer used in the solid dispersion of the present invention, for example, a water-soluble polymer, an enteric polymer and a gastric dissolving polymer are used and, inter alia, an enteric polymer is preferably used.

As a water-soluble polymer, for example, (i) cellulose derivatives such as hydroxyalkylcellulose such as hydroxypropylcellulose, hydroxymethylcellulose and the like; alkylcellulose such as methylcellulose, ethylcellulose and the like; (ii) polyalkenylpyrrolidone such as polyvinylpyrrolidone and the like; (iii) polyalkylene glycol such as polyethylene glycol and the like are used.

As an enteric polymer, for example, hydroxyalkylcellulose phthalate such as hydroxypropylmethylcellulose phthalate and the like; hydroxyalkylcellulose acetate succinate such as hydroxypropylmethylcellulose acetate succinate and the like; carboxyalkylcellulose such as carboxymethylethylcellulose and the like; cellulose acetate phthalate; a copolymer of ethyl acrylate and methacrylic acid such as methacrylic acid copolymer (Eudragit (registered trademark) L100-55) and the like; a copolymer methyl methacrylate and methacrylic acid such as methacrylic acid copolymer L, methacrylic acid copolymer S and the like are used.

As a gastric-dissolving polymer, for example, aminoalkyl methacrylate copolymer E; polyvinylacetal diethylaminoacetate are used.

Other hydrophilic polymers are also used, which can disperse a water-poorly soluble or insoluble HER2 inhibitory substance, such as a copolymer containing a small amount of quaternary ammonium group of ethyl acrylate and methyl methacrylate such as methacrylic acid copolymer RL, mehtacrylic acid copolymer RS and the like, carboxymethylcellulose, carboxyvinyl polymer, polyvinyl alcohol, gum arabic, sodium alginate, alginic acid propylene glycol ester, agar, gelatin and chitosan. Two or more hydrophilic polymers above-mentioned may be used together.

Among the forgoing, as a hydrophilic polymer, hydroxyalkylcellulose, alkylcellulose, polyalkenylpyrrolidone, polyalkylene glycol, methacrylic acid copolymer, and carboxymethylcellulose are preferable, and hydroxypropylmethylcellulose phthalate, polyvinylpyrrolidone, hydroxypropylmethylcellulose, carboxymethylethylcellulose, and methacrylic acid copolymer L are particularly preferable.

The solid dispersion of the present invention may contain additives which are generally used in the field of pharmaceutical preparations.

As an additive, pharmaceutically acceptable carriers such as various organic and inorganic carrier substances which are conventionally used as a preparation material are used. They are incorporated as an excipient, a lubricant, a binder, a disintegrating agent or a surfactant. In addition, if necessary, preparation additives such as a preservative, an antioxidant, a colorant and sweetener may be used.

As a preferable example of an excipient, for example, lactose, sucrose, D-mannitol, starch, crystalline cellulose, sucrose, porous starch, mannitol, calcium silicate (trade name: Florite-RE), magnesium aluminate metasilicate (trade name: Neusilin), light silicic anhydride (trade name: Sylysia), sucrose-starch spherical granule (trade name: Nonpareil), crystalline cellulose-carboxymethylcellulose (trade name: Avicel RC), and hydroxypropylstarch are used.

Preferable examples of a lubricant include crystalline cellulose, magnesium stearate, calcium stearate, talc, colloidal silica, corn starch, and magnesium oxide.

Preferable examples of a binder include crystalline cellulose, sucrose, D-mannitol, dextrin, hydroxypropylcellulose, hydroxypropylmethylcellulose, and polyvinylpyrrolidone.

As a preferable example of a disintegrating agent, for example, starch, carboxymethylcellulose, potassium carboxymethylcellulose, sodium crosscarmelose, sodium carboxymethylstarch, methycellulose (trade name: Metolose SM), sodium crosscarmelose, potassium carmelose, low-substituted hydroxypropylcellulose, starch sodium glycolate, and partially gelatinized starch are used.

As a surfactant, for example, polyoxyethylene polyoxypropylene glycol (trade name: Pluronic), glycerin fatty acid ester, sucrose fatty acid ester, polyoxyethylene hydrogenated castor oil, Polysorbate 80, and cetanol are used.

As a preferable example of a preservative, for example, paraoxybenzoic acid esters, chlorobutanol, benzyl alcohol, phenethyl alcohol, dehydroacetic acid, and sorbic acid are used.

As a preferable example of antioxidant, for example, sulfite and ascorbic acid are used.

The additive may be used alone, or two or more additives may be used together.

The solid dispersion of the present invention can be prepared by a known per se method, particularly, by solvent method such as spray drying method and rotary evaporation method; melt method such as twinscrew extruder method; mixing grinding method; or ultrasound method using an ultrasound generator.

More particularly, the solid dispersion of the present invention can be prepared by the following solvent method, comprising:

(1) dissolving a HER2 inhibitory substance in a suitable organic solvent,
(2) adding a hydrophilic polymer to this solution,
(3) if necessary, suspending an additive such as an excipient, a disintegrating agent, a lubricant and a surfactant in this suspension or solution, then,
(4) evaporating off the organic solvent from this homogeneous suspension under reduced pressure or under normal pressure by a conventional method such as spray drying method and rotary evaporation method.

In addition, when a more homogenous solid dispersion is desired, the homogenous suspension is prepared by the above-mentioned procedure (2), which is then subjected to the following procedures, comprising:

(5) dissolving the suspension prepared in the above preparing method (2) in a suitable organic solvent,
(6) if necessary, suspending an additive such as an excipient, a disintegrating agent, a lubricant and a surfactant, then,
(7) evaporating off the organic solvent under reduced pressure or under normal pressure by a conventional method such as spray drying method and rotary evaporation method.

An organic solvent used in the above procedure (1) is not particularly limited as far as it can dissolve a water-poorly soluble or insoluble HER2 inhibitory substance and a hydrophilic polymer and, for example, alcohols such as methanol, ethanol, propanol, isopropyl alcohol, butanol, monomethoxyethanol, ethylene glycol monomethylether and the like; ethers such as diethyl ether, dibutyl ether, diisobutyl ether, dioxane, tetrahydrofuran, ethylene glycol and the like; aliphatic hydrocarbons such as n-hexane, cyclohexane and n-heptane; aromatic hydrocarbons such as benzene, toluene and xylene; nitriles such as acetonitrile and the like; organic acids such as acetic acid, propionic acid and the like; esters such as ethyl acetate; aliphatic halogenated hydrocarbons such as dichloromethane, dichloroethane, chloroform and the like; ketones such as acetone, methyl ketone and the like; amides such as dimethylformamide, dimethyl acetamide and the like; a mixture thereof them at a suitable ratio are used. Among them, a solvent having a low boiling point such as ketones and alcohols are preferable and, inter alia, acetone or ethanol is preferable.

Procedural conditions such as a treating temperature and treating time is different depending on a raw material compound and an organic solvent to be used. Usually a treating temperature is 200°C or lower

In melt method, the solid dispersion can be prepared by warming a water-poorly soluble or insoluble HER2 inhibitory substance above its melting point, dissolving therein a hydrophilic polymer, and if necessary, an additive such as an excipient, a disintegrating agent, a lubricant and a surfactant, and then rapidly cooling the melt. For example, in twinscrew extruder method, the solid dispersion can be prepared by physically mixing a water-poorly soluble or insoluble HER2 inhibitory substance and a hydrophilic polymer, and if necessary, an additive such as an excipient, a disintegrating agent, a lubricant and a surfactant, warming the mixture at a temperature lower than a melting point of the water-poorly soluble or a insoluble HER2 inhibitory substance under a high pressure to melt the mixture, and then

rapidly cooling the melt.

In mixing grinding method, the solid dispersion can be prepared by physically mixing a water-poorly soluble or insoluble HER2 inhibitory substance and a hydrophilic polymer, and if necessary, an additive such as an excipient, a disintegrating agent, a lubricant and a surfactant, and then mixing and grinding the mixture.

In ultrasonic method, the solid dispersion can be prepared by physical mixing a water-poorly soluble or insoluble HER2 inhibitory substance and a hydrophilic polymer, and if necessary, an additive such as an excipient, a disintegrating agent, a lubricant and a surfactant, filling the mixture into a mortar to pre-mold it, and then applying an ultrasound using, for example, an ultrasonic generator.

An amount of a hydrophilic polymer is not particularly limited, however may be any amount as far as it is such an amount that a water-poorly soluble or insoluble HER2 inhibitory substance can be dispersed. For example, a suitable weight ratio of a hydrophilic polymer to a water-poorly soluble or insoluble HER2 inhibitory substance is in a range of 0.01:1 to 100:1, preferably 0.02:1 to 50:1, more preferably 0.1:2 to 20:1, still more preferably 0.3:1 to 10:1, or 1:1 to 20:1, further preferably 1:1 to 10:1, further preferably 1:1 to 5:1, especially 3 to 5 (particularly 4):1 or 2 to 4:1.

An amount of an additive is not particularly limited and, when an additive is used, a preferable weight ratio of an additive such as an excipient, a disintegrating agent, a lubricant and a surfactant, to a water-poorly soluble or insoluble HER2 inhibitory substance is usually in a range of 0.1:1 to 20:1, preferably 0.3:1 to 10:1, more preferably 1: 1 to 3:1.

An organic solvent used in the above procedure (5) is not particularly limited, however any solvent such as chloroform and dichloromethane may be used as far as it is a solvent which can dissolve the suspension in the above procedure (2).

The solid dispersion of the present invention can be used as it is as a pharmaceutical composition for oral administration, or may be formulated into pharmaceutical compositions such as fine granules, very fine granules, granules, tablets, capsules and injectables by the conventional method.

The pharmaceutical composition containing the solid dispersion of the present invention may optionally contains the above-mentioned additive, specifically, a colorant, a sweetener, a flavor, and diluent and a lubricant such as sucrose, lactose, starch, crystalline cellulose, synthetic ammonium silicate, magnesium stearate and talc in a pharmaceutical preparation for oral administration. Alternatively, the surface of a composition may be coated. to prepare a slow-releasing composition.

Since a HER2 inhibitory substance is usually water-poorly soluble or insoluble, and a ratio actually absorbed into blood to the amount administrated is small when orally administrated, it has the defect that the bioavailability is low.

However, various compositions which are prepared by arbitrarily transforming the solid dispersion of the present invention into above-mentioned various dosage forms have the remarkably improved solubility, oral absorbability or (and) absorbability into blood.

As above mentioned, the solid dispersion of the present invention attains solubilization of a water-poorly soluble or insoluble HER2 inhibitory substance, whereby, the bioavailability of a water-poorly soluble or insoluble HER2 inhibitory substance is dramatically improved.

The content of a water-poorly soluble or insoluble HER2 inhibitory substance in the solid dispersion of the present invention varies depending on a dosage form, an administration method and a carrier, and is usually 0.1 to 99% (w/w), preferably 10 to 50%(w/w), more preferably 20 to 40%(w/w), further preferably 25 to 35%(w/w) relative to a total amount of a preparation.

The content of a hydrophilic polymer in the solid dispersion of the present invention is different depending on a dosage form, an administration method and a carrier, and is usually 1 to 99.9%(w/w), preferably 50 to 90%(w/w), more preferably 60 to 80%(w/w), more preferably 65 to 75% (w/w) relative to a total amount of a preparation.

The content of an additive in the solid dispersion of the present invention varies depending on a dosage form and an administration method, and is usually 10 to 99% (w/w) relative to a total amount of a preparation.

The content of the solid dispersion in a pharmaceutical preparation of the present invention varies depending on a dosage form, an administration method and a carrier, and is usually 0.1 to 100% (w/w), preferably 5 to 80%(w/w), more preferably 30 to 60%(w/w), further preferably 33 to 50%(w/w) relative to a total amount of a preparation.

The content of an additive in a pharmaceutical preparation of the preparation varies depending on a dosage form and an administration method, and is usually 10 to 99%(w/w) relative to a total amount of a preparation.

When the solid dispersion of the present invention is mixed with other additives to prepare a composition, it is preferable that about 30 to 40% by weight of the solid dispersion and about 70 to 60% by weight of an additive are mixed into a composition. It is preferable that such the additive and ita amount used (weight ratio in preparation) are appropriately selected from lactose (about 3 to 30% by weight), crystalline cellulose (about 40 to 60% by weight), light silicic anhydride (about 0.5 to 13% by weight), potassium carmelose (about 1 to 5% by weight), a magnesium stearate, dry methacrylic acid copolymer (about 30 to 35% by weight), D-mannitol (about 35 to 45% by weight), povidone (about 6 to 10% by weight), crosspovidone (about 5 to 10% by weight) and magnesium stearate (about 0.3 to 2.0% by weight).

Other hormone therapeutic agents, chemical therapeutic agents, immune therapeutic agents or cell proliferation factor and drugs which inhibit actions of the receptor may be incorporated into the solid dispersion of the present invention or the pharmaceutical composition containing the same.

Examples of the "hormone therapeutic agent" include fosfestrol, diethylstilbestrol, chlorotrianisene, medroxyprogesterone acetate, megestrol acetate, chlormadinone acetate, cyproterone acetate, danazol, allylestrenol, gestrinone, mepartricin, raloxifene, ormeloxifene, levormeloxifene, anti-estrogen (e.g. tamoxifen citrate, toremifen citrate), pill composition, mepitiostane, testolactone, aminoglutethimide, LH-RH agonists (e.g. goserelin acetate, buserelin, leuprorelin), droloxifene, epitiostanol, ethynylestradiol sulfonate, aromatase inhibitory drugs (e.g. fadrozole hydrochloride, anastrozole, letrozole, exemestan, vorozole, formestan), anti-androgens (e.g. flutamide, bicalutamide, nilutamide), 5α-reductase inhibitory drugs (e.g. finasteride, epristeride), adrenocortical hormone series drugs (e.g. dexamethasone, prednisolone, betamethasone, triamcinolone), androgen synthesis inhibiting drugs (e.g. abiraterone and liase inhibiting drug), retinoids and drugs which delay metabolism of retinoids (e.g. liarozole), and inter alia, LH-RH agonists (e.g. goserelin acetate, buserelin, leuprorelin) are preferable.

Examples of the "chemical therapeutic agent" include an alkylating agent, a metabolism antagonist, anticancer antibiotic and a plant-derived anti-cancer agent.

Examples of the "alkylating agent" include nitrogen mustard, hydrochloric acid nitrogen mustard N-oxide, chlorambutyl, cyclophosphamide, ifosfamide, thiotepa, carboquone, improsulfan tosilate, busulfan, nimustine hydrochloride, mitobronitol, melphalan, dacarbazine, ranimustine, estramustine phosphate sodium, triethylenemelamine, carmustine, lomustine, streptozocin, pipobroman, etoglucido, altretamine, ambamustine, dibrospidium hydrochloride, fotemustine, prednimustine, pumitepa, ribomustine, temozolomide, treosulphan, trofosamide, zinostatin stimalamer, carboquone, adozelesin, cystemustine, bizelesin, platinum complexes (carboplatin, cisplatin, Miboplatin, nedaplatin, oxaliplatin).

Examples of the "metabolism antagonist" include mercaptopurine, 6-mercaptopurineriboside, thioinosine, methotrexate, enocitabine, cytarabime, cytarabine ocphosfate, ancitabine hydrochloride, 5-FU series drugs (e.g. fluorouracil, tegafur, UFT, doxifluridine, carmofur, gallocitabine, emitefur), aminopterin, calcium leucovorine, tabloid, butocine, calcium folinate, levofilinate calcium, Cladribine, emitefur, fludarabine, gemcitabine, hydroxycarbamide, pentostatin, piritrexim, idoxuridine, mitoguazone, thiazophrine, ambamustine.

Examples of the "anti-cancer antibiotic" include anthracycline anti-cancer drugs (doxorubicin hydrochloride, daunorubicin hydrochloride, aclarubicin hydrochloride, pirarubicin hydrochloride, epirubicin hydrochloride), actinomycin D, actinomycin C, mitomycin C,. chromomycin A3, bleomycin hydrochloride, breomycin sulfate, peplomycin sulfate, neocarzinostatin, mithramycin, sarcomycin, carzinophilin, mitotane, zorubicin hydrochloride, mitoxantrone hydrochloride, idarubicin hydrochloride.

Examples of "plant derived anti-cancer agent" include vinkaalkaloid anti-cancer drugs (vinblastine sulfate, vincristine sulfate, vindesine sulfate), taxane anti-cancer drugs (paclitaxel, docetaxel), ethopuside, ethopuside phosphate, teniposide, vinorelbine.

Examples of the "immune therapeutic agent (BRM)" include picibanil, krestin, sizofiran, lentinan, ubenimex, interferons, interleukins, macrophage colony stimulating factors, glanulocite colony stimulating factors, erythropoietins, lymphotoxins, BCG vaccines, corynebacterium parvum, levamisole, polysaccharide K, procodazole.

The "cell proliferation factor" in the "cell proliferation factor and a drug which inhibits the activity of a receptor thereof" may be any substance which promotes growth of cells, and examples thereof include factors which are peptides having a molecular weight of 20,000 or smaller and active at the lower concentration by binding with a receptor, specifically, (1) EGF (epidermal growth factor) or a substance having the substantially same activity [e.g. EGF, halegrin (HER2 ligand)etc.", (2) insulin or substance having the substantially same activity [e.g. insulin IGF (insulin-like growth factor)-1, IGF-2 etc.], (3) FGF (fibroblast growth factor) or a substance having the substantially same activity [e.g. acidic FGF, basic FGF, KGF (keratinocyte growth factor), FGF-10], (4) other cell proliferation factor [e.g. CSF (colony stimulating factor), EPO (erythropoietin), IL-2 (interleukin-2), NGF (nerve growth factor), PDGF (platelet-derived growth factor), TGFβ (transforming growth factorβ), HGF (hepatocyte growth factor), VEGF (vascular endothelial growth factor)].

The "receptor of cell proliferation factor", may be any receptor which has the ability to bind with the abovementioned cell proliferation factor and examples thereof include EGF receptor, halegrin receptor (HER2), insulin receptor, IGF receptor, FGF receptor-1 or FGF receptor-2, HGF receptor (c-met), VEGF receptor, SCF receptor (c-kit).

Examples of the "drug which inhibits the activity of cell proliferation factor" include herceptine (HER2 antibody), GLEEVEC (c-met, c-kit, abl inhibiting drug), and Iressa (EGF receptor inhibiting drug). In addition, a drug which inhibits the activities of a plurality of cell proliferation factors, and a drug which blocks intracellular information from cell proliferation factor are included.

In addition to the aforementioned drugs, L-asparaginase, aceglatone, procarbazine hydrochloride, protoporphyrin-cobalt complex, hematoprophin mercury-sodium, topoisomerase I inhibitory drug (e.g. irinotecan, topodecane, etc.), topoisomerase II inhibitory drugs (e.g. sobuzoxane), differentiation inducing agents (e.g. retinoid, vitamin D, etc.), vascularization inhibitory drugs, α-blockers (e.g. tamusulosin hydrochloride) can be also used.

Among the above-mentioned drugs, as a drug to be used together, LH-RH agonist (goserelin acetate, buserelin, leuprorelin), herceptine (HER2 antibody), Iressa, taxane drugs (e.g. paclitaxel, docetaxel), platinum complexes (carboplatin, cisplatin, oxaliplatin, etc.), 5-FU drugs (e.g. fluorouracil, tegafur, UFT, doxifluridine, etc.), gemcitabine, anthracyclin anti-cancer drugs (e.g. doxorubicin hydrochloride, daunorubicin hydrochloride, epirubicin hydrochloride), cyclophosphamide, ifosfamide, and vinkaalkaloid anti-cancer drugs (vinblastine sulfate, vincristine sulfate, vindesine sulfate) are preferable. In addition, these drugs may be used together with other drugs for joint therapy. Examples thereof include joint use of taxane drug and platinum complex, gemcitabine and taxane or platinum complex or 5-FU, joint use of cyclophosphamide and anthracyclic anti-cancer drug, and joint use of these and Iressa or herceptine. As an administration method for joint use, either of a method for administering an individual drug by its optimum administering method, or a method changed in which an individual administration is changed from an optimum administration method may be used.

The content of these other drugs in the solid dispersion of the present invention or the pharmaceutical preparation containing the same varies depending on a dosage form, an administration method and a carrier, and the content is usually 0 to 95%(w/w) relative to a total weight of a preparation.

The solid dispersion of the present invention or the pharmaceutical preparation containing the same has a low toxicity, and can be safely used in a mammal (e.g. human, horse, cow, dog, cat, rat, mouse, rabbit, pig, monkey etc.).

In administration of the solid dispersion of the present invention or a pharmaceutical preparation containing the same to a mammal such as human and the like, the administration method may be oral or parenteral.

A dose of the solid dispersion of the present invention or a pharmaceutical preparation containing the same varies depending on an administration route, symptom and the like and, for example, when administered as an anticancer agent to a patient with breast cancer or prostate cancer (weight 40 to 80kg), the dose is 0.5 to 100mg/ weight per day, preferably 1 to 50mg/kg weight per day, further preferably 1 to 25mg/kg weight as a water-poorly soluble or insoluble HER2 inhibitory drug. Such a dose can be administered once or two or three times per day.

The present invention will be described in detail by way of Reference Examples, Examples and Experimental Examples below, however the present invention is not limited to them.

Elution in column chromatography in Reference Examples was performed under observation by TLC (Thin Layer Chromatography). In observation by TLC, a Kieselgur $60F_{254}$ plate manufactured by Merck was employed as a TLC plate. A solvent used as an eluting solvent in column chromatography was adopted as a developing solvent. A UV-detector is adopted as a detecting method. Silica gel for column was Kieselgur $60F_{254}$ (70 to 230 mesh) manufactured by Merck. NMR spectrum denotes proton NMR, measurement was performed with VARIAN Gemini-200 (200MHz-type spectrometer) by using tetramethylsilane as an internal standard. $\delta$ value was expressed in ppm.

Abbreviations used in Reference Examples have the following meanings:

s: singlet
br; broad
d: doublet
t: triplet
q; quartet
dd: double doublet
dt: double triplet
m: multiplet
J: coupling constant
Hz: Herz
DMF: N,N-dimethylformamide
THF: tetrahydrofuran

Reference Example A1

4-Chloromethyl-2-[(E)-2-(4-methylphenyl)ethenyl]-1,3-oxazole

(i) (E)-3-(4-methylphenyl)-2-propenamide

DMF (5 drops) was added to a solution of 4-methylcinnamic acid (15,19g) in THF (100mL and oxalyl chloride (9.6mL) was added under ice-cooling. The mixture was stirred at room temperature for 2 hours. Oxalyl chloride (4.0mL) was further added. The mixture was further stirred at room temperature for 1 hour, and concentrated to dryness. The residue was dissolved in ethyl acetate (50mL), and the solution was added dropwise to a mixed solution of 25% aqueous ammonia (50mL)-ethyl acetate (20mL) under ice-cooling. The aqueous layer was salted out, and the organic layer was extracted with ethyl acetate. The extract was dried with magnesium sulfate, and concentrated under reduced pressure.

The precipitates were washed with hexane and diethyl ether to obtain the title compound (11.63g) as colorless crystals.
$^1$H-NMR ($CDCl_3$) $\delta$ : 2.37 (3H, s), 5.56 (2H, brs), 6.41 (1H, d, J = 15.8), 7.18 (2H, d, J = 8.0), 7.42 (2H, d, J = 8.0), 7.62 (1H, d, J = 15.8).
IR (KBr) : 1671, 1601, 1518, 1397, 1254, 1123, 990, 816cm$^{-1}$.

(ii) 4-Chloromethyl-2-[(E)-2-(4-methylphenyl)ethenyl]-1,3-oxazole

(E)-3-(4-methylphenyl)-2-propenamide (8.06g) and 1,3-dichloroacetone (6.98g) were refluxed for 3 hours in toluene (50mL). After cooling, the reaction solution was diluted with ethyl acetate, washed with water and a brine, dried with magnesium sulfate, and concentrated under reduced pressured. The residue was purified by subjecting to silica gel column chromatography (eluent; ethyl acetate: hexane = 1:4), to obtain the title compound (8.44g) as white powdery crystals.
$^1$H-NMR ($CDCl_3$) $\delta$ : 2.38 (3H, s), 4.54 (2H, s), 6.87 (1H, d, J = 16.2), 7.20 (2H, d, J = 8.2), 7.43 (2H, d, J = 8.2), 7.52 (1H, d, J = 16.2), 7.62 (1H, s).
IR (KBr) : 1642, 1607, 1591, 1537, 1345, 1267, 976, 943, 810cm'.

Reference Example A2

4-(Chloromethyl-2-[(E)-2-(4-fluorophenyl]ethenyl]-1,3-oxazole

4-Fluorocinnamic acid (25g) was suspended in dichloromethane (300mL), the suspension was stirred under ice-cooling. Then, DMF (0.5mL) and oxalyl chloride (15.36mL) were added dropwise, and a temperature was maintained at the same temperature for 3 hours, and then gradually returned to room temperature. The solvent was evaporated under reduced pressure, and the residue was dissolved in ethyl acetate (100mL). The solution obtained was added dropwise to an ice-cooled mixed solution of 25% aqueous ammonia (250mL) and ethyl acetate (52.5mL). The reaction solution was extracted with ethyl acetate (400mL x 2), and the extract was washed with a saturated brine, and then dried with anhydrous magnesium sulfate. The solvent was evaporated under reduced pressure, and the precipitated crystals were filtered and dried to obtain (E)-3-(4-fluoropheyl)-2-propenamide (24.4g).
The resulting (E)-3-(4-fluorophenyl)-2-propenamide (17.55g) and 1,3-dichloroacetone (12.85g.) were melted at 130°C, and stirred for 1.5 hours. The temperature was returned to room temperature, the mixture was extracted with ethyl acetate, and washed with ice-water, an aqueous saturated sodium bicarbonate solution, and a saturated brine. After dried with anhydrous sodium sulfate, the solvent was evaporated, and the residue was purified by subjecting to column chromatography (eluent; diethyl ether: hexane = 1.9→3:17) to obtain the title compound (10.5g) as colorless crystals.
$^1$H-NMR($CDCl_3$)$\delta$: 4.54(2H,s), 6.84(1H,d,J=16.0Hz), 7.09(2H,t,J=8.8Hz), 7.47-7.55(3H,m), 7.63(1H,s).
IR (KBr):3173, 3133, 3063, 3040, 1645, 1601, 1591, 1537, 1508, 1435, 1416, 1350, 1275, 1233, 1167, 1101, 999 cm$^{-1}$.

Reference Example A3 .

4-(Chloromethyl-2-[(E)-2-(4-trifluoromethylphenyl)ethenyl]-1,3-oxazole

(i) Oxalyl chloride (11.7mL) was added dropwise to a suspension of (E) -3- (4-trifluoromethylphenyl) -2-propenamide 4-trifluoromethylcinnamic acid (19.4g) and DMF (6 drops) in THF (100mL) at 0°C, and the mixture was stirred at room temperature for 2 hours. After the solvent was evaporated under reduced pressure, the residue was dissolved in ethyl acetate (60mL), and poured into 25% aqueous ammonia-ethyl acetate (5:1, 120mL). The aqueous layer was salted out, extracted with a mixed solution (650mL) of ethyl acetate-THF (12:1), followed by ethyl acetate (100mL x 2), and dried with anhydrous magnesium sulfate. The solvent was evaporated under reduced pressure, and the residue was recrystallized from ethyl acetate-hexane to obtain the title compound (18.0g) as colorless plate-like crystals.
$^1$H-NMR ($CDCl_3$) $\delta$ : 5.58 (2H, br s), 6.53 (1H, d, J = 15.8 Hz), 7.63-7.72 (5H, m).
IR (KBr) : 3326, 3167, 1686, 1636, 1617, 1404, 1190 cm$^{-1}$.

(ii) 4-Chloromethyl-2-[(E)-2-(4-trifluoromethylphenyl)ethenyl]-1,3-oxazole

A solution of (E)-3-(4-trifluoromethylphenyl)-2-propenamide (17.9g) and 1,3-dichloroacetone (14.8g) in toluene (83mL) was heated at reflux for 9 hours using a Dean-Stark apparatus. After cooling, water was added to a reaction solution, and it was extracted with ethyl acetate, washed with a saturated brine, and dried with anhydrous magnesium sulfate. After the solvent was evaporated under reduced pressure, the residue was purified by silica gel column chromatography (eluent, hexane: methyl acetate= 6:1→5:1 to obtain the title compound (15.1g) as colorless needle-like crystals.
$^1$H-NMR ($CDCl_3$) δ :4.55 (2H, d, J = 0.8 Hz), 7.00 (1H, d, J = 16.2 Hz), 7.56 (1H, d, J = 16.2 Hz), 7.64-7.68 (5H, m).
IR (KBr): 1350, 1325, 1170, 1136, 1113, 1071, 959, 826, 727, 708 $cm^{-1}$.

Reference Example A4

4-Chloromethyl-2-[(E)-2-(2,4-difluorophenyl)ethenyl]-1,3-oxazole

Using (E)-3-(2,4-difluorophenyl)-2-propenamide (9.16g) and 1,3-dichloroacetone (7.62g), a similar reaction to that of Reference Example A1-(ii) was performed to obtain the title compound (6.31g) as colorless crystals.
$^1$H-NMR ($CDCl_3$) δ : 4.55 (2H, s), 6.8-7.0 (2H, m), 6.96 (1H, d, J = 16.8), 7.45-7.7 (3H, m).

Reference Example A5

4-(Chloromethyl-2-[(E)-2-(2,6-difluorophenyl)ethenyl]-1,3-oxazole

Using (E)-(2,6-difluorophenyl)-2-propenamide (9.0g) and 1,3-dichloroacetone (7.49g), a similar reaction to that of Reference Example A1-(ii) was performed to obtain the title compound (7.18g) as a pale yellow solid.
$^1$H-NMR ($CDCl_3$) δ : 4.55 (2H, s), 6.85-7.0 (2H, m), 7.2-7.35 (2H, m), 7.55-7.7 (1H, m), 7.66 (1H, s).

Reference Example A6

3-(1H-imidazol-2-yl)-1,2-propanediol

3,4-Dihydroxybutyronitrile (30.33g) was dissolved in anhydrous methanol (12.2mL), and a 5.12N hydrogen chloride solution in ether (62mL) was added at a temperature of 5°C or lower under ice-cooling and stirring. Upon stirring at the same temperature for 35 hours, a two-layered solution was obtained. The upper layer was removed, and the lower layer was dissolved in anhydrous methanol (45mL). A solution of aminoacetaldehydedimethylacetal (31.5g) in anhydrous methanol (45mL) was added at 20°C or less under ice-cooling and stirring, followed by stirring for 27 hours. The solvent was evaporated under reduced pressure, water (57mL) and concentrated hydrochloric acid (142mL) were added to the residue, and the mixture was stirred at room temperature for 2 hours. The solvent was evaporated under reduced pressure, an aqueous potassium carbonate solution was added to the residue to adjust to pH10, and then the solvent was evaporated again. The residue was extracted with ethanol (500mL), and concentrated to dryness. After purification by silica gel column chromatography, desalting out treatment with an ion-exchanging residue (Amberlite15 afforded title compound (13.16g) as pale brown crystals.
mp 98-100 °C.
$^1$H-NMR(DMSO-$d_6$)δ:2.60(1H, dd, J=7.6Hz, 14.8Hz), 2.80(1H,dd,J=5.0Hz,14.8Hz), 3.28(1H,dd,J=5.GHz,10.2Hz), 3.35(1H,dd,J=5.4Hz,10.2Hz), 3.72-3.85(1H,m), 6.88(2H,s).
IR (KBr):3167, 3094, 2928, 2656, 1559, 1456, 1416, 1379, 1327, 1291, 1275, 1242, 1202, 1152, 1111, 1092, 1044 $cm^{-1}$.

Reference Example A7

(2R)-3-1H-imidazol-2-yl)-1,2-propanediol

(i) (2R)-1-(benzyloxy)-3-(1-trityl-1H-imidazol-2-yl)-2-propanol

n-butyllithium (1.6M hexane solution, 6.9ml) was added dropwise to a solution of 1-tritylimidazole (3.10g) in THF (80mL) under ice-cooling in the argon atmosphere. After stirred at the same temperature for 30 minutes, (R)-2-[(benzyloxy)methyl]oxirane (1.52mL) was added. After stirred for 1.5 hours under ice-cooling and at room temperature for 1 hour, water was added to the reaction solution, followed by ethyl acetate. The extract was washed with water and a brine, dried with magnesium sulfate, and concentrated under reduced pressure. The residue was purified by subjecting to the silica column gel chromatography (eluent; ethyl acetate:hexane= 1:1) to obtain the title compound (1.402g) as a pale yellow oil.
$^1$H-NMR ($CDCl_3$) δ: 2.06 (2H, dd, J = 2.8Hz, 18.0Hz), 3.08 (1H, dd, J = 5.4Hz, 9.8Hz), 3.21 (1H, dd, J = 5.4Hz, 9.8Hz), 3.55-3.7 (1H, m), 4.36 (2H, s), 6.73 (1H, d, J = 1.4Hz), 6.93 (1H, d, J = 1.4Hz), 7.0-7.4 (20H, m).

(ii) (2R)-1-(benzyloxy)-3- (1H-imidazole-2-yl)-2-propanol

1N hydrochloric acid (8mL) was added to a solution of (2R)-1-(benzyloxy)-3-(1-trityl-1H-imidazol-2-yl)-2-propanol (1.40g) in acetone (8mL), and the mixture was stirred at 50°C for 1 hour. 1N hydrochloric acid (8mL) was further added, mixture was further stirred at 50°C for 2 hours. The reaction solution was concentrated, water was added, and the material was washed with diethyl ether two times. The aqueous layer was neutralized with an aqueous sodium bicarbonate solution, extracted with ethyl acetate, washed with a brine, dried with magnesium sulfate, and concentrated under reduced pressure. The residue was purified by subjecting silica gel column chromatography (eluent; ethyl acetate: methanol = 10:1) to obtain the title compound (424mg) as a colorless oil.
$^1$H-NMR ($CDCl_3$) δ: 2.85 (1H, dd, J = 7.8Hz, 15.6Hz), 2.99 (1H, dd, J = 3.6Hz, 15.6Hz), 3.39 (1H, dd, J = 7.0Hz, 9.5Hz), 3.52 (1H, dd, J = 4.4Hz, 9.5Hz), 4.1-4.3 (1H, m), 4.55 (2H, s), 6.94 (2H, s), 7.3-7.45 (5H, m).

(iii) (2R)-3-(1H-imidazol-2-yl)-1,2-propanediol

10% palladium on carbon (50% hydrous, 85mg) was added to a solution of (2R)-1-(benzyloxy)-3-(1H-imidazole-2-yl)-2-propanol (424mg) in methanol (10mL) and the mixture was stirred at 50 to 60°C for 2 days under the hydrogen atmosphere. The catalyst was filtered, and the filtrate was concentrated to obtain the title compound (254mg) as a white solid.
$^1$H-NMR ($CDCl_3$)S: 2.58 (1H,dd,J=7.6Hz, 14.6Hz), 2.78 (1H, dd, J =5.2Hz, 14.6Hz), 3.17 (1H, d, J=5.2Hz), 3.2-3.3 (1H, m), 3.7-3.85 (1H, m), 4.6-4.7 (1H, m), 4.86 (1H, d, J=4.8Hz), 6.76 (1H, brs), 6.95 (1H, brs).
$[\alpha]_D^{22}$ = + 2.5° (C = 1.0, methanol)

Reference Example A8

(23)-3-(1H-imidazol-2-yl)-1,2-propanediol

(i) (3S)-4-(benzyloxy)-3-(trimethylsilyloxy)butyronitrile

Potassium cyanide (26mg) and 18-crown-6 (106mg) were added to a mixed solution of (2S)-2-[(benzyloxy) methyl]oxirane (6.57g) and trimethylsilancarbonitrile (5.0g), and the mixture was refluxed at 135°C for 75 minutes under the argon atmosphere. After cooling, evaporation under reduced pressure afforded the title compound (7.42g).
$^1$H-NMR ($CDCl_3$) δ : 0.15(9H,s), 2.52(1H,dd,J=6.6Hz,16.6Hz), 2.65(1H, dd, J=4.6Hz, 16.6Hz), 3.39(1H,dd,J=6.8Hz, 9.6Hz), 3.50(1H, dd, J=4.8Hz, 9.6Hz), 4.01-4.14(1H,m), 4.52(2H, s), 7.26-7.44(5H,m).
IR (neat):3065, 3032, 2957, 2903, 2865, 2251, 1607, 1588, 1497, 1454, 1416, 1366, 1254, 1209, 1117, 1001 $cm^{-1}$.

(ii) (3S)-4-(benzyloxy)-3-hydroxybutyronitrile

(3S)-4-(benzyloxy)-3-[(trimethylsilyl)oxy]butyronitrile (7.41g) was dissolved in tetrahydrofuran (28.2mL), and 1M solution of tetrabutyl ammonium fluoride in THF (28.2mL) was added under ice-cooling stirring, followed by stirring for 1.5 hours. The solvent was evaporated under reduced pressure, and the residue was dissolved in ether, and washed with water and a saturated brine. The solvent was evaporated under reduced pressure, and the residue was purified by silica gel column chromatography to obtain the title compound (4.58g) as a colorless oil.
$^1$H-NMR(DMSO-$d_6$)δ:2.56(1H, dd, J=6.4Hz, 16.8Hz),
2.70(1H,dd,J=4.6Hz,16.8Hz), 3.34(1H,dd,J=6.2Hz,9.8Hz), 3.44(1H,dd,J=5.4Hz,9.8Hz), 3.85-3.95(1H,m), 5.52(2H,d, J=5.2Hz), 7.25-7.40(5H,m).
IR (neat):3600-3200, 3065, 3032, 2867, 2253, 1605, 1586, 1497, 1454, 1416, 1364, 1308, 1254, 1208, 1101, 1078 $cm^{-1}$.

(iii) (2S)-1-(benzyloxy)-3-(1H-imidazol-2-yl)-2-propanol

Using (3S)-4-(benzyloxy)-3-hydroxybutyronitrille (6.51g), a 5.12N solution of hydrogen chloride in ether (7.0mL) and aminoacetaldehydedimethylacetal (3.58g), a similar reaction to that of Reference Example A6 was performed to obtain the title compound (2.22g) as a pale brown oil.
$^1$H-NMR($CDCl_3$)δ:2.84(1H, dd, J=7.8Hz,15.4Hz),
2.97(1H,dd,J=3.6Hz,15.4Hz), 3.41(1H,dd,J=6.8Hz,9.4Hz), 3.51(1H,dd,J=4.4Hz,9.4Hz), 4.11-4.23(1H,m), 4.54(2H,s), 6.91(2H,s), 7.27(5H,m).
IR (neat):3400-3140, 3065, 3032, 2903, 2865, 1601, 1557, 1495, 1454, 1427, 1366, 1312, 1206, 1101, 1028 $cm^{-1}$.
$[\alpha]_D^{22}$ = - 2.3° (C = 1.04, methanol)

(iv) (2S)-3-(1H-imidazol-2-yl)-1,2-propanediol

(2S)-1-(benzyloxy)-3-(1H-imidazol-2-yl)-2-propanol (1.725g) was dissolved in ethanol (30mL), and 10% palladium on carbon (1.04g) was added. The mixture was vigorously stirred at 60°C for 24 hours under hydrogen atmosphere at 5 atm. The catalyst was filtered off, the solvent was evaporated, and the residue was purified by silica gel flash column chromatography to obtain the title compound (0.945g).
The spectral data ($^1$H-NMR, IR) of the present product was identical to those of the compound of Reference Example A6.

Reference Example A9

(i) 4-(4-Benzyloxyphenyl)-3-buten-1-ol

3-Hydroxypropyltriphenylphosphonium bromide (4.02g) was suspended in dehydrated THF (30mL) under the argon atmosphere, and 60% oily sodium hydride (0.4g) was added. The mixture was refluxed for 3 hours. A solution of 4-benzyloxybenzaldehyde (2.12g) in dehydrated THF (7mL) was added dropwise to the reaction solution, followed by refluxing for 67 hours. After cooling, the insoluble materials were filtered off, the filtrate was concentrated under reduced pressure. The residue was purified by subjecting to column chromatography (eleuent; hexane : ethyl acetate = 9:1→4:1) to obtain the title compound (1.76g) as colorless crystals.
$^1$H-NMR ($CDCl_3$) δ: 2.46(0.8H,dq,J=1.4Hz,6.2Hz), 2.61(1.2H,dq,J=1.6Hz,6.4Hz), 3.71-3.78(2H,m), 5.06(1.2H,s), 5.07 (1.8H,s), 5.59(0.6H,dt,J=7.2Hz,11.6Hz), 6.07(0.4H,dt,J=7.2Hz,15.8Hz), 6.45(0.4H,d,J=15.8Hz), 6.52(0.6H,d, J=11.6Hz), 6.89-6.98(2H,m), 7.22-7.46(7H,m).
IR (KBr): 3279, 3063, 3036, 3011, 2911, 2867, 1607, 1574, 1510, 1470, 1454, 1383, 1302, 1250, 1177, 1117, 1053, 1017 $cm^{-1}$.

(ii) 4-(4-Hydroxybutyl)phenol

4-(4-Benzyloxyphenyl)-3-buten-1-ol (1.70g) was dissolved in a mixed solution of methanol and THF (1:1, 20mL), and 10% palladium on carbon (0.17g) was added. The mixture was vigorously stirred for 1.5 hours under the hydrogen atmosphere. The catalyst was filtered off, and the filtrate was concentrated under reduced pressure to obtain the title compound (1.1g) as colorless crystalline powders.
$^1$H-NMR($CDCl_3$) δ: 1.50-1.76(4H,m), 2.57(2H,t,J=7.1Hz), 3.67(2H,t,J=6.2Hz), 6.74(2H,d,J=8.4Hz), 7.03(2H,d, J=8.4Hz).
IR (KBr): 3500-3100, 3025, 2940, 2859, 1615, 1597, 1514, 1456, 1362, 1240, 1173, 1107, 1055, 1024 $cm^{-1}$.

(iii)4-[4-(Benzyloxy)phenyl]-1-butanol

Dry DMF (115mL) was added to 4-(4-hydroxybutyl)phenol (9.43g) and 65% oily sodium hydroxide (2.4g) under the argon atmosphere. The mixture was stirred for 15 minutes. Then, a solution of benzyl bromide (9.87g) in dry dimethylformamide (29.5mL) was added under ice-cooling and stirring, and mixed at the same temperature for 2 hours. Ice water and a IN aqueous potassium hydrogensulfate solution were added, followed by extraction with ethyl acetate. The organic layer was washed with a saturated brine, and dried with anhydrous sodium sulfate. The solvent was evaporated under reduced pressure, and the residue was purified by silica gel column chromatography to obtain the title compound (10.67g) as colorless crystalline powders.
$^1$H-NMR(DMSO-$d_6$)δ: 1.34-1.64(4H,m), 2.50(2H,t,J=7.0Hz), 3.39(2H,dt,J=5.2Hz,6.4Hz), 4.34(1H,t,J=5.2Hz), 5.05(2H, s), 6.90(2H,d,J=8.6Hz), 7.09(2H,d,J=8.6Hz), 7.28-7.47(5H,m).
IR (KBr): 3500-3200, 3048, 3036, 2928, 2907, 2861, 2840, 1615, 1582, 1514, 1472, 1454, 1379, 1360, 1298, 1285, 1250, 1175, 1119, 1063, 1012 $cm^{-1}$.

(iv) 4-[4-(Benzyloxy)phenyl]butyl methanesulfonate

Triethylamine (8.16mL) and methanesulfonyl chloride (4.53mL) were added dropwise to a solution of 4-(4-benzyloxy)butanol (10g) in ethyl acetate (390mL) under ice-cooling. After stirring at the same temperature for 30 minutes and at room temperature for 1 hour, the material was washed with ice water and a saturated brine. After dried with anhydrous sodium sulfate, the solvent was evaporated under reduced pressure to obtain the title compound (14g) as an oil. The present product was used in the next step without purification.
$^1$H-NMR($CDCl_3$)δ:1.64-1.86(4H,m), 2.60(2H,t,J=7.1Hz) 2.98(3H,s), 4.23(2H,t,J=6.1Hz), 5.05(2H,s), 6.91(2H,d, J=8.8Hz), 7.09(2H,d,J=8.8Hz), 7.32-7.48(5H,m).

IR (neat):3063, 3031, 2940, 2865, 1611, 1584, 1512, 1456, 1354, 1337, 1240, 1175, 1115, 1015 $cm^{-1}$.

(v) Benzyl 4-(4-iodobutyl)phenyl ether

Sodium iodide (20.25g) was dissolved in acetone (195mL), and 4-[4-(benzyloxy)phenyl]butyl methanesulfonate (13g) was added. The mixture was refluxed at 80°C for 1.5 hours. After cooling, the solvent was evaporated, ethyl acetate (750mL) was added to the residue, and the material was washed successively with water, an aqueous sodium thiosulfate solution and a saturated brine. The organic layer was dried with anhydrous magnesium sulfate, and the solvent was evaporated under reduced pressure to obtain the title compound (14.29g) as an oil. The present product was used in the next step without purification.
$^1$H-NMR($CDCl_3$) δ:1.63-1.93(4H,m), 2.57(2H,t,J=7.3Hz) , 3.19(2H,t,J=6.8Hz), 5.04(2H,s), 6.90(2H,d,J=8.8Hz), 7.09 (2H, d, J=8.8Hz), 7.30-7.47(5H,m).
IR (neat):3063, 3031, 2932, 2857, 1611, 1582, 1510, 1454, 1381, 1298, 1238, 1175, 1121, 1026 $cm^{-1}$.

(vi) 1-[4-(4-Benzyloxyphenyl)butyl]-1H-1,2,3-triazole

Benzyl 4-(4-iodobutyl)phenyl ether (1.1g), 1H-1,2,3-triazole (0.31g) and potassium carbonate (0.622g) were suspended in DMF (7.5mL), and the suspension was mixed at 70°C for 26.5 hours. After cooling, the reaction solution was extracted with ethyl acetate, and washed with water and a saturated brine. The solvent was evaporated under reduced pressure, and the residue was subjected to silica gel column chromatography (eluent; hexane: ethyl acetate =4:1→2:3) to obtain the title compound (0.391g).
$^1$H-NMR ($CDCl_3$) δ: 1.61(2H,quintet,J=7.8Hz), 1.93(2H,quintet,J=7.8Hz), 2.59(2H,t,J=7.6Hz), 4.39(2H,t,J=7.1Hz) 5.04(2H,s), 6.90(2H,d,J=8.8Hz) 7.06(2H,d,J=8.8Hz), 7.30-7.48(5H,m), 7.49(1H,s), 7.69(1H,s).
IR (KBr):3106, 3034, 2940, 2861, 1611, 1582, 1512, 1454, 1387, 1298, 1244, 1177, 1113, 1080, 1040, 1028 $cm^{-1}$.

(vii) 4-[4-(1H-1,2,3-triazol-1-yl)butyl]phenol

1-[4-(4-Benzyloxyphenyl)butyl]-1H-1,2,3-triazole (0.38g) was dissolved in methanol (7.6mL), and 10%-palladium on carbon (0.1g) was added. The mixture was vigorously stirred for 14 hours under the hydrogen atmosphere. The catalyst was filtered, the filtrate was concentrated to dryness to obtain the title compound (0.268g) as crystalline powders.
$^1$H-NMR ($CDCl_3$) δ:1.60(2H,quintet,J=7.0Hz), 1.93(2H,quintet,J=7.4Hz), 2.57(2H,t,J=7.5Hz), 4.40(2H,t,J=7.0Hz), 6.79(2H,d,J=8.6Hz), 6.99(2H, d,J=8 . 6Hz), 7.51(1H,s), 7.71(1H,s).
IR (KBr):3148, 3129, 3017, 2946, 2861, 2814, 1615, 1593, 1514, 1462, 1381, 1269, 1242, 1225, 1123, 1078 $cm^{-1}$.

Reference Example A10

4-[3-(1H-1,2,3-triazol-1-yl)propyl]phenol

Benzyl 4-(3-iodopropyl)phenyl ether (2.47g), 1H-1,2,3-triazole (629mgl) and potassium carbonate (1.26gl) were suspended in DMF (17.5mL). The mixture was stirred at 70°C for 18.5 hours. A temperature was returned to room temperature, the material was extracted with ethyl acetate, and washed with water and a saturated brine. The solvent was evaporated under reduced pressure, and the residue was purified by silica gel column chromatography (eluent, hexane : ethyl acetate=4:1→2:3) to obtain 1-[3-(4-benzyloxyphenyl)propyl]-1H-1,2,3-triazole (856mg).
$^1$H-NMR ($CDCl_3$) δ:2.23(2H,quintet,J=7.2Hz), 2.60(2H,t,J=7.5Hz), 4.38(2H, t, J=7.1Hz), 5.05(2H,s), 6.92(2H,d, J=8.8Hz), 7.10(2H, d, J=8.8Hz), 7.30-7.48(5H,m), 7.52(1H,s), 7.72(1H,s).
IR (KBr):3100, 3030, 2960, 2926, 2860, 1613, 1585, 1514, 1454, 1383, 1298, 1250, 1215, 1177, 1115, 1082, 1044, 1028, 1019 $cm^{-1}$.

1-[3-(4-Benzyloxyphenyl)propyl]-1H-1,2,3-triazole (850mg) was dissolved in methanol (29mL), and 10%-palladium on carbon (0.1g) was added. The mixture was vigorously stirred for 13 hours under the hydrogen atmosphere. The catalyst was filtered off, and the filtrate was concentrated to dryness under reduced pressure to obtain the title compound (600mg) as crystalline powders.
$^1$H-NMR($CDCl_3$)δ:2.22 (2H, quintet, J=7.0Hz), , 2.56(2H,t,J=7.0Hz), 4.38(2H,t,J=7.0Hz), 6.87(2H,d,J=8.6Hz), 7.04 (2H,d,J=8.6Hz), 7.55(1H,s), 7.74(1H,s)
IR (KBr):3127, 3100, 3015, 2932, 1615, 1595, 1516, 1456, 1373, 1244, 1223, 1175, 1121, 1080, 1038 $cm^{-1}$.

Reference Example A11

3-[3-(1H-1,2,3-triazol-1-yl)propyl]phenol

(i) 3-[3-(Benzyloxy)phenyl]-1-propanol

3-Benzyloxybenzaldehyde (21.3g) and ethyl diethylphosphonoacetate (23.6g) were suspended in dry DMF (250mL) under the argon stream. 65% oily sodium hydride (3.88g) was added in portions under ice-cooling and stirring, After completion of addition, the mixture was stirred at room temperature for 2 hours. After the solvent was evaporated, the residue was dissolved in ethyl acetate, washed with water and a saturated brine, and dried with anhydrous sodium sulfate. The solvent was evaporated under reduced pressure to obtain 33.15g of the crude product of ethyl (E)-3-[3-(benzyloxy)phenyl]-2-propenate as the oil. The present product was dissolved in ethanol (406mL), and 5% palladium on carbon [Pd-C(en)2.7g] treated with ethylenediamine was added, and the mixture was vigorously stirred under the hydrogen atmosphere. Hydrogen (1.75L) was consumed to complete hydrogenation, and the catalyst was filtered off. The solvent was evaporated under reduced pressure, and the residue was dissolved in dehydrated THF (120mL). The present solution was added dropwise under ice-cooling to a mixed solution in which lithium aluminium hydride (4.61g) had been suspended in dehydrated THF (120mL). The material was stirred under ice-cooling for 1.5 hours and at room temperature for 1 hour. The reaction solution was added to ice water. After adjusted to acidic, the material was extracted with ethyl acetate, washed with water and a saturated brine, and dried with anhydrous sodium sulfate. The solvent was evaporated under reduced pressure, and the residue was purified by silica gel column chromatography to obtain the title compound (14.39g) as a colorless oil.
$^1$H-NMR ($CDCl_3$) $\delta$ :1.80-1.96(2H,m), 2.69(2H,t,J=7.7Hz), 3.66(2H,t,J=6.4Hz), 5.05(2H,s), 6.77-6.87(3H,m), 7.20(1H, t,J=8.0Hz), 7.28-7.48(5H,m).
IR (neat):3330, 3063, 3032, 2940, 2867, 1599, 1582, 1487, 1453, 1381, 1314, 1258, 1155, 1026 $cm^{-1}$.

(ii) 3-[3-(Benzyloxy)phenyl]propyl metahnesulfonate

Using 3-(3-benzyloxyphenyl)propanol (13.5g), triethylamine (8.16mL) and methanesulfonyl chloride (4.53mL), a similar reaction to that of Reference Example A9-(iV) was performed to obtain the title compound (19.7g) as an oil.
$^1$H-NMR ($CDCl_3$) $\delta$ :2.00-2.15(2H,m), 2.73(2H,t,J=7.5Hz), 2.98(3H,s), 4.22(2H,t,J=6.3Hz), 5.06(2H,s), 6.77-6.88(3H, m), 7.22(1H,t,J=7.7Hz), 7.31-7.48(5H,m).
IR (neat):3032, 2940, 2870, 1599, 1584, 1487, 1453, 1381, 1354, 1260, 1175, 1026 $cm^{-1}$.

(iii) Benzyl 3-(3-iodopropyl)phenyl ether

Using 3-[3-(benzyloxy)phenyl]propyl methanesulfonate (19.7g) and sodium iodide (29.25g), a similar reaction to that of Reference Example A9-(v) was performed to obtain the title compound (18.4g) as an oil.
$^1$H-NMR($CDCl_3$)$\delta$:2.11(2H, quintet, J=7.3Hz), 2.70(2H,t,J=7.2Hz), 3.16(2H,t,J=6.8Hz), 5.06(2H,s), 6.78-6.87(3H,m), 7.21(1H,t,J=7.2Hz 7.32-7.48(5H,m).
IR (neat):3063, 3031, 2934, 2861, 1599, 1582, 1487, 1451, 1381, 1316, 1258, 1213, 1155, 1080, 1028 $cm^{-1}$.

(iv) 1-[3-(3-Benzyloxyphenyl)propyl]-1H-1,2,3-triazole

Under the argon atmosphere, 1H-1,2,3-triazole (0.9g) was dissolved in DMF (20mL) and 65% oily sodium hydride (0.48g) was added. After stirred for 30 minutes, a solution of benzyl 3-(3-iodopropyl)phenyl ether (3.53g) in DMF (5mL) was added, and the mixture was stirred at room temperature for 19 hours. The reaction solution was diluted with ethyl acetate, and washed with water and a saturated brine. The solvent was evaporated under reduced pressure, and the residue was subjected to column chromatography to obtain the title compound (1.1g) as the colorless crystals.
mp 74-75 °C.
$^1$H-NMR ($CDCl_3$) $\delta$ :2.25(2H,quintet,J=7.2Hz), 2.63(2H,t,J=7.3Hz), 4.37(2H, t, J=7.1Hz), 5.05(2H,s), 6.75-6.88(3H,m), 7.23(1H,t,J=8.2Hz), 7.31-7.47(5H,m), 7.49(1H, d, J=1.0Hz), 7.71(1H, d, J=1.0Hz).
IR (KBr):3125, 3063, 3032, 2944, 2867, 1599, 1584, 1487, 1453, 1381, 1316, 12.60, 1215, 1157, 1113, 1074, 1028 $cm^{-1}$.

(v) 3-[3-(1H-1,2,3-triazol-1-yl)propyl]phenol

10%-palladium on carbon (0.1g) was added to a solution of 1-[3-(3-benzyloxyphenyl)propyl]-1H-1,2,3-triazole (0.937g) in methanol (32mL), and the mixture was vigorously stirred at room temperature for 8 hours under the hydrogen

atmosphere. The catalyst was filtered off, and the filtrate was concentrated to dryness under reduced pressure to obtain the title compound (0.593g) as the colorless crystals.
mp 85-86 °C.
$^1$H-NMR ($CDCl_3$) δ :2.24(2H,quintet,J=7.1Hz), 2.60(2H,t,J=7.5Hz), 4.38(2H, t, J=7.1Hz), 6.68-6.79(3H,m), 6.96(1H,s), 7.16(1H,t,J=8.1Hz), 7.54(1H, d, J=1.0Hz), 7.73(1H, d, J=1.0Hz).
IR (KBr):3129, 3077, 3054, 2949, 2863, 2722, 2614, 1599, 1588, 1483, 1458, 1362, 1337, 1281, 1221, 1157, 1121, 1080, 1038 cm$^{-1}$.

Reference Example A12

4-{4-[2-(2-Hydroxyethyl)-1H-imidazol-1-yl]butyl}phenol

(i) 2-(1-{4-[4-(Benzyloxy)phenyl]butyl}-1H-imidazol-2-yl)-1-ethanol

Benzyl 4-(4-iodobutyl)phenyl ether (14.29g), 2-(2-hydroxyethyl)imidazole (13.1g) and potassium carbonate (5.39g) were stirred at 60°C for 16 hours in DMF (390mL). After cooling, the insoluble materials were filtered off, and the filtrate was concentrated under reduced pressure. The residue was dissolved in ethyl acetate, and washed with water and a saturated brine. The solvent was evaporated under reduced pressure, and the residue was purified by subjecting to column chromatography (eluent, ethyl acetate: methanol=19:1→9:1). The eluate was recrystallized from ethyl acetate-methanol to obtain the title compound (10.99g) as colorless crystals.
mp 75-77 °C.
$^1$H-NMR($CDCl_3$)δ:1.53-1.82(4H,m), 2.58(2H, t, J=7.1Hz), 2.78(2H,t,J=5.5Hz), 3.81(2H,t,J=6.9Hz), 4.03(2H,t, J=5.5Hz), 5.04(2H,s), 6.80(1H,d,J=1.2Hz), 6.90(2H,d,J=8.6Hz), 6.93(1H,d,J=1.2Hz), 7.05(2H,d,J=8.6Hz), 7.34-7.47 (5H,m).
IR (KBr):3144, 3032, 2934, 2859, 1611, 1582, 1514, 1495, 1456, 1431, 1381, 1298, 1273, 1244, 1175, 1150, 1121, 1109, 1051, 1026 cm$^{-1}$.

(ii) 4-{4-[2-(2-Hydroxyethyl)-1H-imidazol-1-yl]butyl}phenol

Using 2-(1-{4-[4-(Benzyloxy)pheyl]butyl}-1H-imidazol-2-yl)-1-ethanol (10.67g) and 10% palladium on carbon (1.6g), a similar reaction to that of Reference Example A11-(v) was performed to obtain the title compound (5.3g).
mp 118-119 °C.
$^1$H-NMR($CDCl_3$) δ:1.50-1.80(4H,m), 2.55(2H,t,J=7.0Hz), 2.79(2H,t,J=5.8Hz), 3.82(2H, t, J=7.0Hz), 3.97(2H,t, J=5.8Hz), 3.85-4.40(1H,br), 6.77(2H, d, J=8.4Hz), 6.80(1H,s), 6.94(1H,s), 6.96(2H,d,J=8.4Hz).
IR (KBr):3600-2400, 1615, 1593, 1516, 1489, 1456, 1373, 1252, 1171, 1150, 1125, 1103, 1055 cm$^{-1}$.

Reference Example A13

(i) 2-(1-{3-[4-(Benzyloxy)phenyl]propyl}-1H-imidazol-2-yl)-1-ethanol

Using benzyl 4-(3-iodopropyl)phenyl ether (5.28g), 2-(2-hydroxyethyl)imidazole (5.05g) and potassium carbonate (2.07g), a similar reaction to that of Reference Example A12-(i) was performed to obtain the title compound (2.78g) as colorless crystals.
mp 80-82 °C.
$^1$HNMR($CDCl_3$) δ:2.03(2H, quintet, J=7.4Hz), 2.58(2H,t,J=7.4Hz), 2.74(2H,t,J=5.6Hz), 3.82(2H,t,J=7.4Hz), 4.01(2H,t, J=5.6Hz), 5.05(2H,s), 6.83(1H,s), 6.92(2H,d,J=8.6Hz), 6.94(1H,s), 7.07(2H,d,J=8.6Hz), 7.32-7.47(5H,m).
IR (KBr):3500-3100, 3110, 3063, 3032, 2934, 2865, 1611, 1584, 1512, 1495, 1454, 1381, 1298, 1240, 1177, 1152, 1121, 1057, 1024 cm$^{-1}$.

(ii) 4-{3-[2-(2-Hydroxyethyl)-1H-imidazol-1-yl]propyl}phenol

Using 2-(1-{3-[4-(benzyloxy)phenyl]propyl}-1H-imidazol-2-yl)-1-ethanol (2.53g) and 10% palladium on carbon (0.38g), a similar reaction to that of Reference Example A11-(v) was performed to obtain the title compound (1.85g) as colorless crystals.
mp 116-117 °C.
$^1$H-NMR($CDCl_3$+$CD_3OD$) δ:2.03 (2H, quintet, J=7.3Hz),
2.55(2H,t,J=7.3Hz), 2.75(2H,t, J=6.2Hz), 3.83(2H,t,J=7.3Hz), 3.91(2H,t,J=6.2Hz), 6.77(2H,d,J=8.6Hz), 6.84(1H,d, J=1.2Hz), 6.93(1H,d,J=1.2Hz) , 6.97(2H,d,J=8.6Hz).

IR (KBr):3500-3100, 3119, 2934, 2861, 1615, 1593, 1516, 1495, 1454, 1373, 1252, 1173, 1152, 1123, 1053 $cm^{-1}$.

Reference Example A14

3-{3-[2-(2-Hydroxyethyl)-1H-imidazol-1-yl]propyl}phenol

(i) 2-(1-{3-[3-(Benzyloxy)phenyl]propyl}-1H-imidazol-2-yl)-1-ethanol

Using benzyl 3-(3-iodopropyl)phenyl ether (3.53g), 2-(2-hydroxyethyl).imidazole (1.46g) and 65% oily sodium hydride (0.48g), a similar reaction to that of Reference Example A11-(iv) was performed to obtain the title compound (2.66g) as a colorless oil.
$^1$H-NMR($CDCl_3$)δ:2.05 (2H,quintet,J=7.3Hz), 2.61(2H,t,J=7.5Hz), 2.73(2H,t,J=5.5Hz), 3.81(2H,t,J=7.3Hz), 4.02(2H,t, J=5.5$H_z$), 5.06(2H,s), 6.73-6.88(3H,m), 6.82(1H, d, J=1.2Hz), 6.95(1H,d,J=1.2Hz), 7.23(1H,t,J=8.2Hz), 7.31-7.48(5H, m).
IR (neat):3500-3100, 3067, 3034, 2938, 2867, 1599, 1584, 1524, 1491, 1453, 1381, 1316, 1260, 1155, 1119, 1053, 1026 $cm^{-1}$.

(ii) 3-{3-[2-(2-Hydroxyethyl)-1H-imidazol-1-yl]propyl}phenol

Using 2-(1-{3-[3-(Benzyloxy)phenyl]propyl}-1H-imidazol-2-yl)-1-ethanol (2.42g) and 10% palladium on carbon (0.24g), a similar reaction to that of Reference Example A11-(v) was performed to obtain the title compound (1.69g) as colorless crystals.
mp 111-113 °C.
$^1$H-NMR($CDCl_3$)δ:2.07(2H,quintet,J=6.9Hz), 2.55(2H,t,J=7.3Hz), 2.73(2H,t,J=5.9Hz), 3.80(2H, t, J=7.1Hz), 4.00(2H,t, J=5.9Hz), 6.55-6.76(3H,m), 6.86(1H, d, J=1.4Hz), 6.96(1H, d, J=1.4Hz), 7.15(1H,t,J-7.8Hz).
IR (KBr)$cm^{-1}$:3500-3100, 3046, 2940, 2865, 2712, 2604, 1599, 1588, 1528, 1483, 1456, 1372, 1279, 1250, 1155, 1123, 1057.

Reference Example A15

3-{1-[4-(4-Hydroxyphenyl)butyl]-1H-imidazol-2-yl}-1,2-propanediol

(i) 3-{1-[4-(4-Benzyloxyphenyl)butyl]-1H-imidazol-2-yl}-1,2-propanediol

Using benzyl 4-(4-iodobutyl)phenyl ether (2.05g), 2-(2,3-dihydroxypropyl)imidazole (1.0g) and 65% oily sodium hydride (0.259g), a similar reaction to that of Reference Example A11-(iv) was performed to obtain the title compound (1.23g) as colorless crystals.
$^1$H-NMR($CDCl_3$) δ:1.52-1.83(4H,m), 2.57(2H,t,J=7.1Hz), 2.78 (2H, d, J=5.2Hz), 2.79(1H, d, J=6.8Hz), 3.62 (1H, dd, J=4.8Hz, 11.2Hz ) , 3.74 (1H, dd, J=4 . 8Hz, 11.2Hz ) , 3.82(2H,t,J=7.1Hz), 4.12-4.23(1H,m) 5.04(2H,s), 6.79(1H,d, J=1.4Hz), 6.90(2H,d,J=8.6Hz), 6.91(1H,d,J=1.4Hz), 7.05(2H,d,J=8.6Hz), 7.30-7.47(5H,m).
IR (KBr):3500-3200, 3065, 3030, 2932, 2861, 1611, 1582, 1510, 1495, 1454, 1379, 1296, 1275, 1240, 1177, 1150, 1123, 1080, 1026 $cm^{-1}$.

(ii) 3-{1-[4-(4-Hydroxyphenyl)butyl]-1H-imidazol-2-yl}-1,2-propanediol

Using 3-{1-[4-(4-benzyloxyphenyl)butyl]-1H-imidazol-2-yl}-1,2-propanediol (1.22g) and 10% palladium on carbon (0.18g), a similar reaction to that of Reference Example A11-(v) was performed to obtain the title compound (0.918g) as colorless crystals.
$^1$H-NMR($CDCl_3$+$OD_3OD$) δ:1.50-1.80(4H,m), 2.55(2H,t,J=7.0Hz), 2.75(1H,d,.J=7.2Hz) , 2.76(1H, d, J=5.6Hz), 3.49 (1H, dd, J=5.4Hz,11.6Hz), 3.62(1H,dd,J=4.2Hz,11.6Hz), 3.84(2H,t,J=7.0Hz), 3.97-4.08(1H,m) 6.75(2H,d,J=8.6Hz), 6.80(1H,d,J=1.4Hz), 6.89(1H,d,J=1.4Hz), 6.97(2H,d,J=8.6Hz).
IR (KBr):3500-3100, 3011, 2936, 2859, 1613, 1595, 1516, 1489, 1456, 1372, 1360, 1252, 1171, 1150, 1125, 1101, 1030 $cm^{-1}$.

Reference Example A16

(i) 3-{1-[3-(3-Benzyloxyphenyl)propyl]-1H-imidazol-2-yl}-1,2-propanediol

Using benzyl 3-(3-iodopropyl)phenyl ether (1.98g), 2-(2,3-dihydroxypropyl)imidazole (1.0g) and 65% oily sodium hydride (0.259g), a similar reaction to that of Reference Example A11-(iv) was performed to obtain the title compound (1.31g) as a colorless oil.
$^1$H-NMR($CDCl_3$)δ:2.05(2H, quintet, J=7.3Hz), 2.60(2H,t,J=7.3Hz), 2.73 (1H, d, J=4 .8Hz), 2.74 (1H, d, J=7.2Hz), 3.61 (1H,dd,J=4.8Hz,11.2Hz), 3.74(1H,dd,J=4.8Hz,11.2Hz), 3.82(2H,t,J=7.3Hz), 4.12-4.23(1H,m), 5.06(2H,s), 6.73-6.88 (3H,m), 6.81(1H, d, J=1.2Hz), 6.93(1H,d,J=1.2Hz), 7.23(1H,t,J=8.4Hz), 7.31-7.48(5H,m).
IR (neat):3500-3200, 3063, 3032, 2934, 2865, 1599, 1584, 1526, 1489, 1454, 1381, 1316, 1260, 1155, 1123, 1082, 1028 $cm^{-1}$.

(ii) 3-{1-[3-(3-hydroxyphenyl)propyl]-1H-imidazol-2-yl}-1,2-propanediol

Using 3-{1-[3-(3-benzyloxyphenyl)propyl]-1H-imidazol-2-yl}-1,2-propanediol (1.30g) and 10% palladium on carbon (0.195g), a similar reaction to that of Reference Example 11A-(v) was performed to obtain the title compound (0.979g) as a colorless oil.
$^1$H-NMR($CDCl_3$+$CD_3OD$) δ:2.07(2H, quintet, J=7.4Hz),
2.58(2H,t,J=7.3Hz), 2.72(1H,d,J=6.8Hz), 2.72(1H,d,J=5.8Hz), 3.50(1H,dd,J=5.4Hz,11.4Hz), 3.61(1H,d,J=4.2Hz, 11.4Hz), 3.85(2H,t,J=7.3Hz), 3.98-4.10(1H,m) 6.60-6.74(3H,m), 6.86(1H,d,J=1.4Hz), 6.92(1H,d,J=1.4Hz), 7.14(1H,t, J=7.8Hz).
IR (neat):3500-3100, 3040, 2942, 2863, 1599, 1588, 1530, 1483, 1456, 1360, 1279, 1254, 1155, 1125, 1088, 1030 $cm^{-1}$.

Reference Example A17

2-[(E)-2-(2,4-difluorophenyl)ethenyl]-4-[[4-(4-iodobutyl)phenoxy]methyl]-1,3-oxazole

(i) 4-[4-[2-(E)-[2-(2,4-difluorophenyl)ethenyl]-1,3-oxazol-4-yl]methoxyphenyl]-1-butanol

60% oily sodium hydride (528mg) was added to a solution of 4-(4-hydroxyphenyl)-1-butanol (1.99g) in DMF (20mL) under ice-cooling, and the mixture was stirred at room temperature for 30 minutes. Under ice-cooling, (E)-4-chloromethyl-2-[2-(2,4-difluorophenyl)ethenyl]oxazole (3.37g) was added, and the mixture was stirred at room temperature overnight. Water and IN hydrochloric acid were added to the reaction solution, followed by extraction with ethyl acetate. The extract was dried with magnesium sulfate, concentrated under reduced pressure, and the residue was recrystallized from ethyl acetate-diethyl ether-hexane to obtain the title compound (3.71g) as colorless crystals.
mp 75-76 °C.
$^1$H-NMR ($CDCl_3$) δ: 1.5-1.7 (4H, m), 2.60 (2H, t, J = 6.8Hz), 3.66 (2H, t, J = 6.0Hz), 5.02 (2H, s), 6.8-6.9 (1H, m), 6.89 (2H, d, J = 8.4Hz), 6.98 (1H, d, J = 17.0Hz), 7.11 (2H, d, J = 8.4Hz), 7.5-7.6 (1H, m), 7.59 (1H, d, J = 17.0Hz), 7.66 (1H, s).
IR (KBr): 1613, 1514, 1493, 1431, 1279, 1246, 1140, 968, 856$cm^{-1}$.

(ii) 2-[(E)-2-(2,4-difluorophenyl)ethenyl]-4-[ [4-(4-iodobutyl)phenoxy]methyl]-1,3-oxazole

Triethylamine (1.37mL) was added to a solution of 4-[4-[2-(E)-[2-(2,4-diflouorophenyl)ethenyl]-1,3-oxazol-4-yl]methoxyphenyl]-1-butanol (3.47g) in THF (50mL), and methanesulfonyl chloride (0.77mL) was added under ice-cooling. The mixture was stirred at room temperature for 30 minutes. Water was added to the reaction solution, extracted with ethyl acetate, the extract was washed with a brine, and dried with magnesium sulfate. The solvent was evaporated, and acetone (100m and sodium iodide (6.75g) were added to the residue, and then the mixture was stirred at 40-50°C for 2 hours. The reaction solution was concentrated, and water was added, followed by extraction with ethyl acetate. The extract was washed successively with an aqueous sodium thiosulfate solution and a brine, dried with magnesium sulfate, and concentrated under reduced pressured. The precipitates were filtered, and washed with diethyl ether-hexane to obtain the title compound (3.55g) as pale yellow powders.
$^1$H-NMR ($CDCl_3$)δ: 1.6-1.9 (4H, m), 2.5-2.7 (2H, m), 3.1-3.3 (2H, m), 5.02 (2H, s), 6.8-7.2 (6H, m), 7.5-7.75 (4H, m).
IR (KBr): 1615, 1514, 1493, 1431, 1279, 1246, 1140, 966, 856$cm^{-1}$.

Reference Example A18

2-[(E)-2-(4-bromophenyl)ethenyl]-4-[[4-(4-iodobutyl)phenoxy]methyl]-1,3-oxazole

Using 4-(4-hydroxyphenyl)-1-butanol (4.99g) and (E)-4-chloromethyl-2-[2-(4-bromophenyl)ethenyl]oxazole (7.43g), a similar reaction to that of Reference Example A17-(i) was performed to obtain 4-[4-[2-(E)-[2-(4-bromophenyl) ethenyl]-1,3-oxazol-4-yl]methoxyphenyl]-1-butanol (9.70g). Using the resulting compound (4.28g), a similar reaction to that of Reference Example A17-(ii) was performed to obtain the title compound (4.47g) as white powders.
$^1$H-NMR ($CDCl_3$)δ:1.65-1.95(4H,m), 2.58(2H,t,J=7.2Hz), 3.20(2H,t,J=6.8Hz), 5.02(2H,s), 6.92(1h, d, J=16.4Hz), 6.92 (2H,d,J=8.6Hz), 7.38(2H,d,J=8.4Hz), 7.47(1H,d,J=16.4Hz), 7.52(2H,d,J=8.4Hz), 7.66(1H,s).

Reference Example B1

[1-[4-[4-[[2-[(E)-2-(4-methylphenyl)ethenyl]-1,3-oxazol-4-yl]methoxy]phenyl]butyl]-1H-1,2,3-triazole

60% oily sodium hydride (35mg) was added to a solution of 4-[4-(1H-1,2,3-triazol-1-yl)butyl]phenol (174mg) in DMF (4mL) under ice-cooling, and the mixture was stirred at room temperature for 30 minutes. Under ice-cooling, (E)-4-chloromethyl-2-[2-(4-methylphenyl)ethenyl]oxazole (206mg) was added, and the mixture was stirred at room temperature for 2 hours. Water was added to the reaction solution, and the precipitates were filtered, and then washed with water. The filtered material was dissolved in a mixed solution of THF-ethyl acetate. The solution was washed with water and a brine, dried with magnesium sulfate, and concentrated under reduced pressure. The residue was recrystallized from ethyl acetate-hexane to obtain the title compound (281mg) as colorless crystals.
mp 154-155 °C.
$^1$H-NMR($CDCl_3$)δ:1.5-1.7(2H,m),1.85-2.05(2H, m), 2.38 (3H, s), 2.60 (2H, t, J=7.5Hz), 4.39 (2H, t, J = 7.0Hz), 5.01 (2H, s), 6.87 (2H, d, J = 8.6Hz), 6.9-7.0 (1H, m), 7.19 (2H, d, J = 8.6Hz), 7.19 (2H, d, J = 8.0Hz), 7.42 (2H, d, J = 8.0Hz), 7.5-7.7 (4H, m).
IR (KBr): 1640, 1607, 1530, 1514, 1464, 1339, 1256, 1211, 1053, 974, 810cm$^{-1}$.

| Elemental analysis: for $C_{25}H_{26}N_4O_2$ | | | |
|---|---|---|---|
| Cal'd(%) | C, 72.44; | H, 6.32; | N, 13.52. |
| Found(%) | C, 72.36; | H, 6.49; | N, 13.70. |

Reference Example B2

1-{4-[4-({2-[(E)-2-(4-fluorophenyl)ethenyl]-1,3-oxazol-4-yl}methoxy)phenyl]butyl}-1H-1,2,3-triazole

DMF (5mL) was added and dissolved in 4-[4-(1H-1,2,3-triazol-1-yl)butyl]phenol (218mg) and 65% oily sodium hydride (39mg) under the argon atmosphere. 4-(Chloromethyl)-2-[(E)-2-(4-fluorophenyl)ethenyl]-1,3-oxazole (250mg) was added under ice-cooling and stirring, and the mixture was stirred at room temperature for 3 hours. Water was added to the reaction solution, followed by extraction with ethyl acetate. The extract was washed with water and a saturated brine, dried with sodium sulfate, and concentrated under reduced pressure. The residue was purified by subjecting to silica gel column chromatography (eluent; chloroform:ethanol = 24:1), and then recrystallized from ethyl acetate to obtain the title compound (368mg) as colorless crystals.
mp 124-125 °C.
$^1$H-NMR($CDCl_3$)δ:1.62(2H, quintet, J=7.0Hz),
1.94(2H,quintet,J=7.5Hz), 2.61(2H,t,J=7.5Hz), 4.40(2H,t,J=7.0Hz), 5.01(2H,s), 6.86(1H,d,J=16.0Hz), 6.92(2H,d, J=8.6Hz), 7.08(2H,d,J=8.6Hz), 7.09(2H,t,J=8.7Hz), 7.46-7.57(4H,m), 7.66(1H,s), 7.70(1H, d, J=1.0Hz).
IR (KBr):3420, 3160, 3120, 2940, 2924, 2865, 1644, 1599, 1584, 1532, 1512, 1466, 1435, 1400, 1337, 1302, 1248, 1229, 1211, 1177, 1161, 1113, 1076, 1049, 1030 cm$^{-1}$.

| Elemental analysis: for $C_{24}H_{23}N_4O_2F$ | | | |
|---|---|---|---|
| Cal'd(%) | C,68.88; | H,5.55; | N,13.39. |
| Found(%) | C,68.70; | H,5.55; | N,13.49. |

Reference Example B3

1-{3-[3-({2-[(E)-2-(4-fluorophenyl)ethenyl]-1,3-oxazol-4-yl}methoxy)phenyl]propyl}-1H-1,2,3-triazole

Using 3-[3-(1H-1,2,3-triazol-1-yl)propyl]phenol (208mg), 65% oily sodium hydride (39mg) and 4-(chloromethyl)-2-[(E)-2-(4-fluorophenyl)ethenyl]-1,3-oxazole (250mg), a similar reaction to that of Reference Example B2 was performed to obtain the title compound (366mg).
mp 105-106 °C.
$^1$H-NMR($CDCl_3$)δ:2.26(2H, quintet, J=7.2Hz), 2.64(2H,t,J=7.5Hz), 4.39(2H,t,J=7.0Hz), 5.03(2H,s), 6.78-6.89(3H,m), 6.86(1H,d,J=16.2Hz), 7.09(2H,t,J=8.6Hz), 7.25(1H,t,J=7.8Hz), 7.51(1H,d,J=16.2Hz), 7.47-7.54(3H,m), 7.68(1H,s), 7.72(1H,s).
IR (KBr):3110, 3050, 2955, 2870, 1642, 1601, 1586, 1532, 1507, 1489, 1460, 1453, 1337, 1310, 1273, 1240, 1213, 1177, 1159, 1113, 1097, 1080, 1065 $cm^{-1}$.

| Elemental analysis: for $C_{23}H_{21}N_4O_2F$ | | | |
|---|---|---|---|
| Cal'd(%) | C,68.30;. | H,5.23; | N,13.85. |
| Found(%) | C,68.22; | H,5.04; | N,14.00. |

Reference Example B4

1-(4-{4-[(2-{(E)-2-[4-(trifluoromethyl)phenyl]ethenyl}-1,3-oxazol-4-yl)methoxy]phenyl}butyl)-1H-1,2,3-triazole

Using 4-[4-(1H-1,2,3-triazol-1-yl)butyl]phenol (152mg), 65% oily sodium hydride (28mg) and 4-(chloromethyl)-2-{(E)-2-[4-(trifluoromethyl)phenyl]ethenyl}-1,3-oxazole (212mg), a similar reaction to that of Reference Example B2 was performed to obtain the title compound (290mg).
mp 160-161 °C.
$^1$H-NMR($CDCl_3$)δ:1.62(2H, quintet, J=7.0Hz),
1.94(2H,quintet,J=7.6Hz), 2.61(2H,t,J=7.4Hz), 4.40(2H,t,J=7.4Hz), 5.02(2H,s), 6.92(2H,d,J=8.6Hz), 7.02(1H,d, J=16.6Hz), 7.08(2H,d,J=8.6Hz), 7.50(1H,s), 7.56(1H,d,J=16.6Hz), 7.64(4H,s), 7.69(1H,s), 7.71(1H,s).
IR (KBr):3120, 2936, 1615, 1584, 1512, 1464, 1414, 1327, 1248, 1159, 1125, 1069 $cm^{-1}$.

| Elemental analysis: for $C_{25}H_{23}N_4O_2F_3$ | | | |
|---|---|---|---|
| Cal'd(%) | C,64.10; | H,4.95; | N,11.96. |
| Found(%) | C,64.18; | H,5.12; | N,11.98. |

Reference Example B5

1-(3-{4-[(2-{(E)-2-[4-(trifluoromethyl)phenyl]ethenyl}-1,3-oxazol-4-yl)methoxy]phenyl}propyl)-1H-1,2,3-triazole

Using 4-[3-(1H-1,2,3-triazol-1-yl)propyl]phenol (143mg), 65% oily sodium hydride (28mg) and 4-(chloromethyl)-2-{(E)-2-[4-(trifluoromethyl)phenyl]ethenyl}-1,3-oxazole (212mg), a similar reaction to that of Reference Example B2 was performed to obtain the title compound (232mg).
mp 157-158 °C.
$^1$H-NMR($CDCl_3$)δ:2.24(2H, quintet, J=7.2Hz), 2.61(2H,t,J=7.3Hz), 4.39(2H,t,J=7.2Hz), 5.03(2H,s), 6.94(2H,d, J=8.4Hz), 7.02(1H,d,J=16.4Hz), 7.11(2H,d,J=8.4Hz), 7.52(1H,s), 7.56(1H,d,J=16.4Hz), 7.64(4H,s), 7.69(1H,s), 7.72 (1H,s).
IR (KBr):3129, 3100, 2934, 1613, 1584, 1547, 1510, 1449, 1416, 1337, 1329, 1291, 1238, 1179, 1140, 1109, 1071, 1009 $cm^{-1}$.

| Elemental analysis: for $C_{24}H_{21}N_4O_2F_3$ | | | |
|---|---|---|---|
| Cal'd(%) | C,63.43; | H,4.66; | N,12.33. |
| Found(%) | C,63.21; | H,4.73; | N,12.26. |

Reference Example B6

1-(3-{3-[(2-{(E)-2-[4-(trifluoromethyl)phenyl]ethenyl}-1,3-oxazol-4-yl)methoxy]phenyl}propyl)-1H-1,2,3-triazole

Using 3-[3-(1H-1,2,3-triazol-1-yl)propyl]phenol (123mg), 65% oily sodium hydride (24mg) and 4-(chloromethyl)-2-{(E)-2-[4-(trifluoromethyl)phenyl]ethenyl}-1,3-oxazole (183mg), a similar reaction to that of Reference Example B2 was performed to obtain the title compound (248mg).
mp 115-116 °C.
$^1$H-NMR($CDCl_3$) δ:2.26(2H, quintet, J=7.2Hz), 2.64(2H,t,J=7.2Hz), 4.39(2H,t,J=7.2Hz), 5.04(2H,s), 6.77-6.91(3H,m), 7.01(1H,d,J=16.6Hz), 7.25(1H,t,J=8.4Hz), 7.52(1H,s), 7.56(1H, d, J=16.6Hz), 7.64(4H,s), 7.71(2H,s).
IR (KBr):3140, 3050, 2940, 2860, 1610, 1599, 1586, 1487, 1451, 1415, 1327, 1262, 1169, 1125, 1113, 1069, 1017 $cm^{-1}$.

| Elemental analysis: for $C_{24}H_{21}N_4O_2F_3$ | | | |
|---|---|---|---|
| Cal'd(%) | C,63.43; | H,4.66; | N,12.33. |
| Found(%) | C,63.36; | H,4.73; | N,12.26. |

Reference Example B7

1-{4-[4-({2-[(E)-2-(2,4-difluorophenyl)ethenyl]-1,3-oxazol-4-yl}methoxy)phenyl]butyl}-1H-1,2,3-triazole

Using 4-[4-(1H-1,2,3-triazol-1-yl)butyl]phenol (152mg), 65% oily sodium hydride (28mg) and 4-(chloromethyl)-2-[(E)-2-(2,4-difluorophenyl)ethenyl]-1,3-oxazole (188mg), a similar reaction to that of Reference Example B2 was performed to obtain the title compound (254mg).
mp 115-117 °C.
$^1$H-NMR($CDCl_3$)δ:1.62(2H, quintet, J=7.2Hz), 1.94(2H,quintet,J-7.5Hz), 2.60(2H,t,J=7.5Hz), 4.39(2H,t,J=7.1Hz), 5.01 (2H,s), 6.81-6.98(2H,m),
6.91(2H,d,J=8.6Hz), 6.98(1H,d,J=16.2Hz), 7.07(2H,d,J=8.6Hz), 7.47-7.53(1H,m), 7.50(1H,s) 7.59(1H,d,J=16.2Hz), 7.67(1H,s), 7.70(1H,s).
IR (KBr):3133, 2932, 2863, 1644, 1615, 1590, 1532, 1514, 1493, 1468, 1431, 1345, 1298, 1279, 1246, 1215, 1179, 1140, 1086, 1049, 1032 $cm^{-1}$.

| Elemental analysis: for $C_{24}H_{22}N_4O_2F_2$ | | | |
|---|---|---|---|
| Cal'd(%) | C,66.05; | H,5.08; | N,12.84. |
| Found(%) | C,66.03; | H,5.00; | N,13.03. |

Reference Example B8

1-{3-[3-({2-[(E)-2-(2,4-difluorophenyl)ethenyl]-1,3-oxazol-4-yl}methoxy)phenyl]propyl}-1H-1,2,3-triazole

Using 3-[3-(1H-1,2,3-triazol-1-yl)propyl]phenol (143mg), 65% oily sodium hydride (28mg) and 4-(chloromethyl)-2-[(E)-2-(2,4-difluorophenyl)ethenyl]-1,3-oxazole (188mg), a similar reaction to that of Reference Example B2 was performed to obtain the title compound (257mg).
mp 89-90 °C.
$^1$H-NMR($CDCl_3$) δ:2.26(2H, quintet, J=7.3Hz), 2.64(2H,t,J=7.4Hz), 4.39(2H,t,J=7.1Hz), 5.03(2H,s), 6.77-6.98(5H,m), 6.98(1H,d,J=16.8Hz), 7.24(1H,t,J=7.6Hz), 7.47-7.60(1H,m), 7.52(1H,s), 7.59(1H,d,J=16.8Hz), 7.68(1H,s), 7.71(1H,s).
IR (KBr):3127, 3071, 2934, 2868, 1644, 1615, 1599, 1534, 1495, 1453, 1433, 1354, 1273, 1215, 1159, 1142, 1090, 1028 $cm^{-1}$.

| Elemental analysis: for $C_{23}H_{20}N_4O_2F_2$ | | | |
|---|---|---|---|
| Cal'd(%) | C,65.39; | H,4.77; | N,13.26. |
| Found(%) | C,65.32; | H,4.56; | N,13.34. |

Reference Example B9

[1-[4-[4-[[2-[(E)-2-(2,6-difluorophenyl)ethenyl]-1,3-oxazol-4-yl]methoxy]phenyl]butyl]-1H-1,2,3-triazole

65% oily sodium hydride (41mg) was added to a solution of 4-[4-(1H-1,2,3-triazol-1-yl)butyl]phenol (217mg) in DMF (4mL) under ice-cooling. Afterstirred at room temperature for 30 minutes, 4-(chloromethyl)-2-[(E)-2-(2,6-difluorophenyl)ethenyl]-1,3-oxazole (281mg) was added under ice-cooling, and the mixture was stirred at room temperature overnight. Under ice-cooling, water was added, the precipitates were filtered, washed with water, and dissolved in THF-ethyl acetate. The material was washed with water and a dried with magnesium sulfate, and concentrated under reduced pressure. The residue was recrystallized from ethyl acetate-hexane to obtain the title compound (348mg) as colorless crystals.
$^{1}$H-NMR($CDCl_3$)δ: 1.5-1.7 (2H, m) , 1.85-2.05 (2H, m), 2.60 (2H, t, J = 7.4Hz), 4.39 (2H, t, J = 7.2Hz), 5.02 (2H, s), 6.92 (2H, d, J = 8.8Hz), 6.94 (1H, d, J = 17.4Hz), 6.85-7.35 (3H, m), 7.07 (2H, d, J = 8.8Hz), 7.61 (1H, d, J = 17.4Hz), 7.45-7.7 (3H, m).
IR (KBr) : 1620, 1586, 1514, 1464, 1244, 1024, 999, 968, 783cm$^{-1}$.

| Elemental analysis: for $C_{24}H_{22}F_2N_4O_2$ | | | |
|---|---|---|---|
| Cal'd(%) | C, 66.05; | H, 5.08; | N, 12.84. |
| Found(%) | C, 65.83; | H, 5.06; | N, 12.93. |

Reference Example B10

2-[1-[4-[4-[[2-[(E)-2-(4-methylphenyl)ethenyl] -1, 3-oxazol-4-yl]methoxy]phenyl]butyl]-1H-imidazol-2-yl]-1-ethanol

Using 4-[4-[2-(2-hydroxyethyl)-1H-imidazol-1-yl]butyl]phenol (260mg) and (E)-4-chloromethyl-2-[2-(4-methylphenyl)ethenyl]oxazole (257mg), a similar reaction to that of Reference Example B1 was performed to obtain the title compound (331mg) as colorless crystals.
mp 108-109 °C.
$^{1}$H-NMR($CDCl_3$)δ: 1.5-1.8 (4H, m), 2.38 (3H, s), 2.58 (2H, t, J = 7.0Hz), 2.79 (2H, t, J = 5.6Hz), 3.82 (2H, t, J = 6.8Hz), 4.03 (2H, t, J = 5.6Hz), 5.01 (2H, s), 6.8-6.85 (2H, m), 6.89 (1H, d, J = 16.6Hz), 6.92 (2H, d, J = 8.6Hz), 7.07 (2H, d, J = 8.6Hz), 7.19 (2H, d, J = 7.8Hz), 7.43 (2H, d, J = 7.8Hz), 7.51 (1H, d, J = 16.6Hz), 7.64 (1H, s).
IR (KBr): 1510, 1240, 1055, 806cm$^{-1}$.

| Elemental analysis: for $C_{28}H_{31}N_3O_3$ | | | |
|---|---|---|---|
| Cal'd(%) | C, 73.50; | H, 6.83; | N, 9.18. |
| Found(%) | C, 73.36; | H, 6.66; | N, 9.12. |

Reference Example B11

2-[1-[4-[4-[[2-[(E)-2-(3-methylphenyl)ethenyl]-1,3-oxazol-4-yl]methoxy]phenyl]butyl]-1H-imidazol-2-yl]-1-ethanol

Using 4-[4-[2-(2-hydroxyethyl)-1H-imidazol-1-yl]butyl]phenol (260mg) and (E)-4-chloromethyl-2-[2-(3-methylphenyl)ethenyl]oxazole (257mg), a similar reaction to that of Reference Example B1 was performed to obtain the title compound (290mg) as colorless crystals.
mp 109-111 °C.
$^{1}$H-NMR($CDCl_3$)δ: 1.55-1.8 (4H, m) , 2.38 (3H, s), 2.58 (2H, t, J = 7.0Hz), 2.78 (2H, t, J = 5.6Hz), 3.82 (2H, t, J = 7.0Hz), 4.03 (2H, t, J = 5.6Hz), 5.01 (2H, s), 6.80 (1H, d, J = 1.4HZ), 6.92 (1H, d, J = 16.6HZ), 6.92 (2H, d, J = 8.8Hz), 6.93 (1H, d, J = 1.4Hz), 7.07 (2H, d, J = 8.8Hz), 7.1-7.2 (1H, m), 7.2-7.4 (3H, m), 7.51 (1H, d, J = 16.6Hz), 7.65 (1H, s).
IR (KBr) : 1514, 1460, 1250, 1051, 976, 828, 789cm$^{-1}$.

| Elemental analysis: for $C_{28}H_{31}N_3O_3$ $0.2H_2O$ | | | |
|---|---|---|---|
| Cal'd(%) | C, 72.92; | H, 6.86; | N, 9.11. |
| Found(%) | C, 72.71; | H, 6.74; | N, 8.97. |

Reference Example B12

2-[1-[4-[4-[[2-[(E)-2-(2-methylphenyl)ethenyl] -1, 3-oxazol-4-yl]methoxy]phenyl]butyl]-1H-imidazol-2-yl]-1-ethanol

Using 4-[4-[2-(2-hydroxyethyl)-1H-imidazol-1-yl]butyl]phenol (153mg) and (E)-4-chloromethyl-2-[2-(2-methylphenyl)ethenyl]oxazole (151mg), a similar reaction to that of Reference Example B1 was performed to obtain the title compound (167mg) as the colorless crystals.
mp 91-93 °C(Ethyl acetate-hexane).
$^{1}$H-NMR($CDCl_3$)δ: 1.5-1.8 (4H, m), 2.46 (3H, s), 2.59 (2H, t, J = 7.0Hz)/ 2.79 (2H, t, J = 5.6Hz), 3.82 (2H, t, J = 7.0Hz), 4.03 (2H, t, J = 5.6Hz), 5.02 (2H, s), 6.8-6.9 (3H, m), 6.92 (2H, d, J = 8.6Hz), 7.07 (2H, d, J = 8.6Hz), 7.2-7.3 (3H, m) , 7.55-7.65 (1H, m), 7.66 (1H, s), 7.79 9 (1H, d, J = 16.2Hz).
IR (KBr) : 1508, 1464, 1231, 1061, 1009, 862, 752cm$^{-1}$.

| Elemental analysis: for $C_{28}H_{31}N_3O_3$ 0.2$H_2O$ | | | |
|---|---|---|---|
| Cal'd(%) | C,72.92; | H, 6.86; | N, 9.11. |
| Found(%) | C,72.98; | H, 6.70; | N, 9.23. |

Reference Example B13

2-[1-[4-[4-[[2-[(E)-2-(4-ethylphenyl)ethenyl]-1,3-oxazol-4-yl]methoxy]phenyl]butyl]-1H-imidazol-2-yl]-1-ethanol

60% oily sodium hydride (44mg) was added to a solution of 4-[4-[2-(2-hydroxyethyl)-1H-imidazol-1-yl]butyl] phenol (260mg) in DMF (4mL) under ice-cooling. After stirred at room temperature for 30 minutes, (E)-4-chloromethyl-2-[2-(4-ethylphenyl)ethenyl]oxazole (272mg) was added under ice-cooling. After stirred at room temperature overnight, water was added under ice-cooling. The precipitates were filtered, and washed with water, dissolved in ethyl acetate, dried (magnesium sulfate) and concentrated under reduced pressure. The residue was recrystallized from ethyl acetate-hexane to obtain the title compound (297mg) as colorless crystals.
mp 94-95 °C.
$^{1}$H-NMR($CDCl_3$)δ: 1.25 (3H, t, J = 7.4Hz), 1.5-1.85 (4H, m), 2.59 (2H, t, J = 7.0Hz), 2.67 (2H, q, J = 7.4Hz), 2.79 (2H, t, J = 5.4Hz), 3.82 (2H, t, J = 7.0Hz), 4.04 (2H, t, J = 5.4), 5.01 (2H, s), 6.8-7.0 (3H, m), 6.92 (2H, d, J = 8.4Hz), 7.07 (2H, d, J = 8.4Hz), 7.2-7.3 (2H, m), 7.4-7.5 (2H, m), 7.53 (1H, d, J = 17.2Hz), 7.65 (1H, s).
IR (KBr) : 1508, 1462, 1231, 1181, 1061, 1007, 864, 833cm$^{-1}$.

| Elemental analysis: for $C_{29}H_{33}N_3O_3$ | | | |
|---|---|---|---|
| Cal'd(%) | C,73.86; | H,7.05; | N,8.91. |
| Found(%) | 0,73.73; | H,6.79; | N,8.76. |

Reference Example B14

2-(1-{4-[4-({2-[(E)-2-(4-fluorophenyl)ethenyl]-1,3-oxazol-4-yl}methoxy)phenyl]butyl}-1H-imidazol-2-yl)-1-ethanol

Using 4-[4-[2-(2-hydroxyethyl)-1H-imidazol-1-yl]butyl]phenol (391mg), 65% oily sodium hydride (60mg) and 4-(chloromethyl)-2-(E)-2-(4-fluorophenyl)ethenyl]-1,3-oxazole (375mg), a similar reaction to that of Reference Example B2 was performed to obtain the title compound (583mg).
mp 130-132 °C.
$^{1}$H-NMR($CDCl_3$)δ:1.56-1.84(4H,m), 2.10-2.90(1H,br), 2.58(2H,t,J=7.1Hz), 2.78(2H,t,J=5.5Hz), 3.82(2H, t, J=7.1Hz), 4.03(2H,t,J=5.5Hz), 5.01(2H,s), 6.80-6.94(5H,m), 7.04-7.13(4H,m), 7.46-7.55(3H,m), 7.65(1H,s).
IR (KBr):3150, 3113, 3048, 2936, 2861, 1642, 1599, 1582, 1532, 1512, 1464, 1422, 1399, 1375, 1337, 1302, 1277, 1246, 1229, 1209, 1177, 1159, 1148, 1105, 1051, 1001 cm$^{-1}$.

| Elemental analysis: for $C_{27}H_{28}N_3O_3F$ | | | |
|---|---|---|---|
| Cal'd(%) | )C,70.26; | H,6.11; | N,9.10. |
| Found(%) | C,70.15; | H,6.06; | N,9.35 |

Reference Example B15

2-[1-[4-[4-[[2-[(E)-2-(4-chlorophenyl)ethenyl]-1,3-oxazol-4-yl]methoxy]phenyl]butyl]-1H-imidazol-2-yl]-1-ethanol

60% oily sodium hydride (22mg) was added to a solution of 4-[4-[2-(2-hydroxyethyl)-1H-imidazol-1-yl]butyl] phenol (130mg) in DMF (4mL) under ice-cooling. After stir at room temperature for 30 minutes, (E)-4-chloromethyl-2-[2-(4-chlorophenyl)ethenyl]oxazole (140mg) was added under ice-cooling. After stir at 0°C for 1 hour and at room temperature overnight, water was added under ice-cooling. The precipitates were filtered, washed with water, and dissolved in a mixed solution of THF and ethyl acetate. This solution was dried with magnesium sulfate, and concentrated under reduced pressure. The residue was recrystallized from methanol-ethyl acetate-diethyl ether to obtain the title compound (168mg) as colorless crystals.
mp 127-128 °C.
$^1$H-NMR($CDCl_3$)δ: 1.5-1.8 (4H, m), 2.58 (2H, t, J = 7.0Hz), 2.78 (2H, t, J = 5.4Hz), 3.82 (2H, t, J = 7.0Hz), 4.03 (2H, t, J = 5.4Hz), 5.01 (2H, s), 6.8-7.0 (5H, m), , 7.07 (2H, d, J = 8.8Hz), 7.35 (2H, d, J = 8.4Hz), 7.46 (2H, d, J = = 8.4Hz), 7.4-7.55 (1H, m), 7.66 (1H, s).
IR (KBr) : 1514, 1474, 1341, 1264, 1246, 1076, 966, 814cm$^{-1}$.

| Elemental analysis: for $C_{27}H_{28}ClN_30_3$ | | | |
|---|---|---|---|
| Cal'd(%) | C,67.85; | H,5.90; | N,8.79. |
| Found(%) | C,67.85; | H,5.72; | N,9.09. |

Reference Example B16

2-[1-[4-[4-[[2 [(E)-2-(4-bromophenyl)ethenyl]-1,3-oxazol-4-yl]methoxy]phenyl]butyl]-1H-imidazol-2-yl]-1-ethanol

60% oily sodium hydride (176mg) was added to a solution of 2-(1H-imidazol-2-yl)-ethanol (449mg) in DMF (10mL under ice-cooling. After stir at room temperature for 30 minutes, 4-[[4-(4-iodobutyl)phenoxy]methyl-2-[(E)-2-(4-bromophenyl)ethenyl]-1,3-oxazole (2.15g) was added under ice-cooling. After stir at room temperature overnight, water was added under ice-cooling, extracted with a mixed solution of ethyl acetate-THF, dried with magnesium sulfate, and concentrated under reduced pressure. The residue was recrystallized from ethyl acetate-hexane to obtain the title compound (2,09g) as pale yellow crystals.
mp 149-150 °C.
$^1$H-NMR($CDCl_3$)δ: 1.55-1.8 (4H, m), 2.58 (2H, t, J = 7.0Hz), 2.78 (2H, t, J = 5.6Hz), 3.82 (2H, t, J = 7.0Hz), 4.03 (2H, t, J = 5.6Hz), 5.01 (2H, s), 6.91 (2H, d, J = 8.8Hz), 6.92 (1H/ d, J = 16.3Hz)/ 6.8-7.0 (2H, m), 7.07 (2H, d, J = 8.8Hz), 7.38 (2H, d, J = 8.6Hz), 7.47 (1H, d, J = 16.3Hz), 7.52 (2H, d, J = 8.6Hz), 7.66 (1H, s).
IR (KBr) : 1514, 1487, 1254, 1055, 972, 826, 814cm$^{-1}$.

| Elemental analysis: for $C_{27}H_{28}BrN_3O_3$ | | | |
|---|---|---|---|
| Cal'd(%) | C,62.07; | H,5.40; | N,8.04. |
| Found(%) | C,61.82; | H,5.26; | N,7.90. |

Reference Example B17

2-[1-[4-[4-[2-[(E)-2-(4-trifluoromethylphenyl)ethenyl]oxazol-4-yl]methoxyphenyl]butyl-1H-imidzol-2-yl)-1-ethanol

DMF (4mL) was added to 65% sodium hydride (40.6mg) and 4-[4-[2-(2-hydroxyethyl)-1H-imidazol-1-yl]butyl] phenol (260mg) at 0°C under the argon atmosphere. After stir at room temperature for 30 minutes, [2-(E)-2-(4-trifluoromethylphenyl)ethenyl]oxazol-4-yl]methyl chloride (316mg) was added at 0°C, and the mixture was stirred at room temperature for 15 hours. Water was added to the reaction solution, the precipitated crystals were filtered, washed with water and isopropyl ether, and recrystallized from acetone-hexane to obtain the title compound (393mg) as pale yellow needle-like crystals.
$^1$H-NHR ($CDCl_3$)δ: 1.56-1.74 (4H, m), 2.59 (2H, t, J = 6.6 Hz), 2.78 (2H, t, J = 5.4 Hz), 3.82 (2H, t, J = 6.8 Hz), 4.03 (2H, t, J = 5.4 Hz), 5.02 (2H, d, J = 1.2 Hz), 6.81 (1H, d, J = 1.6 Hz), 6.90-6.95 (4H, m), 7.02 (2H, d, J = 16.2 Hz), 7.52-7.69 (6H, m).
IR (KBr): 1512, 1323, 1244, 1175, 1132, 1113, 1067, 1055 cm$^{-1}$.

Reference Example B18

2-[1-[3-[4-[2-[(E)-2-(4-trifluoromethylphenyl)ethenyl]oxazol-4-yl]methoxyphenyl]propyl]-1H-imidazol-2-yl]-1-ethanol

Using 65% sodium hydride (40.6mg), 4-[3-[2-(hydroxyethyl)-1H-imidazol-1-yl]propyl]phenol (246mg) and [2-[(E)-2-(4-trifluoromethylphenyl)ethenyl] oxazol-4-yl]methyl chloride (316mg), a similar reaction to that of Reference Example B17 was performed to obtain the title compound (330mg) as colorless needle-like crystals.
$^1$H-NMR ($CDCl_3$) δ: 2.01-2.08 (2H, m), 2.60 (2H, t, J = 7.8 Hz), 2.74 (2H, t, J = 5.8 Hz), 3.83 (2H, t, J = 7.4 Hz), 4.03 (2H, t, J = 5.8 Hz), 5.03 (2H, s), 6.84 (1H, d, J = 1.2 Hz), 6.96-7.12 (6H, m), 7.52-7.70 (6H, m).
IR (KBr): 1512, 1327, 1246, 1173, 1125, 1069, 1017, 826 $cm^{-1}$.

Reference Example B19

2-[1-[4-[4-[[2-[(E)-2-(2,4-difluorophenyl)ethenyl]-1,3-ethanol

60% oily sodium hydride (44mg) was added to a solution of 4-[4-[2-(2-hydroxyethyl)-1H-imidazol-1-yl]butyl] phenol (260mg) in DMF (4mL) under ice-cooling. After stir at room temperature for 30 minutes, (E)-4-chloromethyl-2-[2-(2,4-difluorophenyl)ethenyl]oxazole (281mg) was added under ice-cooling. After stir at room temperature for 3 days, water was added under ice-cooling. The precipitates were filtered, and washed with water, dissolved in a mixed solution of ethyl acetate-THF, dried with magnesium sulfate, and concentrated under reduced pressure. The residue was recrystallized from ethyl acetate-hexane to obtain the title compound (275mg) as pale yellow crystals.
mp 93-95 °C.
$^1$H-NMR($CDCl_3$)δ : 1.55-1.85 (4H, m), 2.58 (2H, t, J = = 7.0Hz), 2.78 (2H, t, J = 5.4Hz), 3.82 (2H, t, J = 7.0Hz), 4.03 (2H, t, J = 5.4Hz), 5.01 (2H, s), 6.8-7.0 (6H, m), 6.98 (1H, d, J = 16.3Hz), 7.07 (2H, d, J = 8.8Hz), 7.5-7.6 (1H, m), 7.59 (1H, d, J = 16.3Hz), 7.67 (1H, s).
IR (KBr) : 1611, 1508, 1277, 1231, 1140, 1103, 1063, 970, 860$cm^{-}$ 1

| Elemental analysis: for $C_{27}H_{27}F_2N_3O_3$ 0.1$H_2O$ | | | |
|---|---|---|---|
| Cal'd(%) | C,67.38; | H,5.70; | N,8.73. |
| Found(%) | C,67.24; | H,5.74; | N,8.55. |

Reference Example B20

2-[1-[3-[4-[[2-[(E)-2-(2,4-difluorophenyl)ethenyl]-1,3-oxazol-4-yl]methoxy]phenyl]propyl]-1H-imidazol-2-yl]-1-ethanol

60% oily sodium hydride (44mg) was added to a solution of 4-[4-[2-(2-hydroxyethyl)-1H-imidazol-1-yl]propyl] phenol (246mg) in DMF (4mL) under ice-cooling. After stir at room temperature for 30 minutes, (E)-4-chloromethyl-2-[2-(2,4-difluorophenyl)ethenyl]oxazole (281mg) was added under ice-cooling. After stir at room temperature overnight, water was added under ice-cooling. The precipitates were filtered, washed with water, dissolved in ethyl acetate, dried with magnesium sulfate, and concentrated under reduced pressure. The residue was recrystallized from ethyl acetate-diethyl ether-hexane to obtain the title compound (272mg) as colorless crystals.
mp 94-96 °C.
$^1$H-NMR($CDCl_3$)δ: 1.95-2.15 (2H, m), 2.5-2.65 (2H, m), 2.65-2.8 (2H, m), 3.75-3.9 (2H,m), 3.95-4.1 (2H, m), 5.02 (2H, s), 6.8-7.15 (9H, m), 7.45-7.7 (3H, m) .
IR(KBr) : 1609, 1512, 1277, 1231, 1140, 1061, 1020, 974, 860$cm^{-1}$.

| Elemental analysis: for $C_{26}H_{25}F_2N_3O_3$ 0.4$H_2O$ | | | |
|---|---|---|---|
| Cal'd(%) | C, 66.06; | H,5.50; | N,8.89. |
| Found(%) | C, 66.13; | H,5.38; | N,8.55. |

Reference Example B21

2-[1-[3-[4-[[2-[(E)-2-(2,6-difluorophenyl)ethenyl]-1,3-oxazol-4-yl]methoxy]phenyl]propyl]-1H-imidazol-2-yl]-1-ethanol

Using 2- (2-hydroxyethyl)-1-[4-(4-hydroxyphenyl)butyl]imidazole (260mg), 60% oily sodium hydride (41mg) and (E)-4-chloromethyl-2-[2-(2,6-difluorophenyl)ethenyl]oxazole (281mg), a similar reaction to that of Reference Example B19 was performed to obtain the title compound (359mg) as colorless crystals.
mp 106-107 °C.
$^{1}$H-NMR($CDCl_3$)δ: 1.5-1.8 (4H, m), 2.58 (2H, t, J = 7.0Hz), 2.78 (2H, t, J = 5.6Hz), 3.82 (2H, t, J = 7.0Hz), 4.03 (2H, t, J = 5.6Hz), 5.02 (2H, s), 6.8-7.0 (6H, m), 7.07 (2H, d, J = 8.4Hz), 7.2-7.35 (2H, m), 7.61 (1H, d, J = 16.8Hz), 7.68 (1H, s).
IR (KBr) : 1618, 1516, 1472, 1456, 1246, 1065, 1001, 974, 789cm$^{-1}$.

| Elemental analysis: for $C_{27}H_{27}F_2N_3O_3$ | | | |
|---|---|---|---|
| Cal'd(%) | C, 67.63; | H, 5.68; | N, 8.76. |
| Found(%) | C, 67.78; | H, 5.57; | N, 9.01. |

Reference Example B22

3-(1-{4-[4-({2-[(E)-2-(3-methylpheyl)ethenyl]-1,3-oxazol-4-yl}methoxy)phenyl]butyl}-1H-imidazol-2-yl)-1,2-propanediol

Using 3-{1-[4-(4-hydroxypheny)butyl]-1H-imidazol-2-yl}-1,2-propanediol (154mg), 65% oily sodium hydride (21mg) and 4-(chloromethyl)-2-[(E)-2-(3-methylpheyl)ethenyl]-1,3-oxazole (131mg), a similar reaction to that of Reference Example B2 was performed to obtain the title compound (156mg).
mp 102-104 °C.
$^{1}$H-NMR($CDCl_3$)δ:1.52-1.82(4H,m), 2.39(3H,s), 2.59(2H,t,J=7.0Hz), 2.77(1H,d,J=5.0Hz), 2.78(1H,d,J=6.8Hz) 3.64 (1H,dd,J=4.8Hz,11.2Hz), 3.76(1H,dd,J=4.2Hz,11.2Hz), 3.82(2H,t,J=7.0Hz), 4.12-4.24(1H,m) 5.02(2H,s), 6.80(1H,d, J=1.4Hz), 6.92(1H,d,J=1.4Hz), 6.93(1H,d,J=16.2Hz) 6.93(1H,d,J=8.8Hz) 7.08(2H,d,J=8.8Hz), 7.13-7.39(4H,m), 7.52 (1H,d,J=16.2Hz), 7.66(1H,s).
IR (KBr):3500-3200, 3112, 3029, 2934, 2865, 1645, 1609, 1584, 1510, 1491, 1462, 1379, 1350, 1242, 1177, 1150, 1123, 1100, 1026 cm$^{-1}$.

| Elemental analysis: for $C_{29}H_{33}N_3O_4$ 0.5$H_2O$ | | | |
|---|---|---|---|
| Cal'd(%) | C,70.14; | H,6.90; | N,8.46. |
| Found(%) | C,70.39; | H,6.63; | N,8.51. |

Reference Example B23

3-(1-{4-[4-({2-[(E)-2-(4-fluorophenyl)ethenyl]-1,3-oxazol-4-yl}methoxy)phenyl]butyl}-1H-imidazol-2-yl)-1,2-propanediol

Using 3-{1-[4-(4-hydroxyphenyl)butyl]-1H-imidazol-2-yl}-1,2-propanediol (291mg), 65% oily sodium hydride (39mg) and 4-(chloromethyl)-2-[(E)-2-(4-fluorophenyl)ethenyl]-1,3-oxazole (250mg), a similar reaction to that of Reference Example B2 was performed to obtain the title compound (347mg).
mp 114-116 °C.
$^{1}$H-NMR($CDCl_3$)δ.1.52-1.83(4H,m), 2.59(2H,t,J=7.2Hz), 2.76(1H,d,J=5.2Hz), 2.77(1H,d,J=7.0Hz), 3.64(1H,dd, J=4.8Hz,11.4Hz), 3.76(1H, dd, J=4.2Hz,11.4Hz), 3.82(2H,t,J=6.8Hz), 4.12-4.24(1H,m), 5.01(2H,s), 6.80(1H,d, J=1.4Hz), 6.86(1H,d,J=16.8Hz), 6.92(1H,d,J=1.4Hz), 6.93(2H, d, J=8.8Hz), 7.07(2H,d,J=8.8Hz), 7.09(2H,d,J=8.7Hz), 7.46-7.56(3H,m), 7.66(1H,s).
IR (KBr):3500-3200, 3152, 3104, 3044, 2940, 2865, 1644, 1599, 1584, 1532, 1512, 1495, 1462, 1422, 1400, 1339, 1300, 1246, 1177, 1159, 1098, 1047 cm$^{-1}$.

| Elemental analysis: for $C_{28}H_{30}N_3O_4F$ | | | |
|---|---|---|---|
| Cal'd(%) | C,68.42; | H,6.15; | N,8.55. |
| Found(%) | C,68.16; | H,5.98; | N,8.46. |

Reference Example B24

3-[1-(4-{4-[(2-{(E)-2-{4-(trifluoromethyl}phenyl}ethenyl}-1, 3-oxazol-4-yl)methoxy]phenyl}butyl]-1H-imidazol-2-yl]-1,2-propanediol

Using 3-{1-[4-(4-hydroxyphenyl)butyl]-1H-imidazol-2-yl}-1,2-propanediol (204mg), 65% oily sodium hydride (28mg) and 4-(chloromethyl)-2-{(E)-2-[4-(trifluoromethyl)phenyl]ethenyl}-1,3-oxazole (212mg), a similar reaction to that of Reference Example B2 was performed to obtain the title compound (285mg).
mp 142-143 °C.
$^1$H-NMR($CDCl_3$)δ:1.53-1.82(4H,m), 2.59(2H,t,J=7.1Hz), 2.76(1H,d,J=5.0Hz), 2.77(1H,d,J=7.0Hz), 3.64(1H,dd, J=4.8Hz,11.4Hz), 3.76(1H,dd,J=4.2Hz,11.4Hz) , 3.83(2H,t,J=6.8Hz), 4.12-4.24(1H,m), 5.02(2H,s), 6.81(1H, d, J=1.4Hz), 6.92(1H, d, J=1.4Hz), 6.93(2H,d,J=8.8Hz), 6.95(1H, d, J=16.4Hz),7.08(2H,d,J=8.8Hz), 7.56(1H,d, J=16.4Hz), 7.64(4H,s), 7.70(1H,s).
IR (KBr):3500-3200, 3148, 3071, 2936, 2867, 1642, 1615, 1582, 1510, 1491, 1466, 1416, 1397, 1323, 1246, 1173, 1138, 1117, 1067, 1046, 1017 $cm^{-1}$.

| Elemental analysis: for $C_{29}H_{30}N_3O_4F_3$ | | | |
|---|---|---|---|
| Cal'd(%) | C,64.32; | H,5.58; | N,7.76. |
| Found(%) | C,64.26; | H,5.70; | N,7.62. |

Reference Example B25

3-[1-(3-{3-[(2-{(E)-2-[4-(trifluoromethyl)phenyl]ethenyl}-1,3-oxazol-4-yl)methoxy]phenyl}propyl)-1H-imidazol-2-yl]-1,2-propanediol

Using 3-{1-[3-(3-hydroxyphenyl)propyl]-1H-imidazol-2-yl}-1,2-propanediol (194mg), 65% oily sodium hydride (28mg) and 4-(chloromethyl)-2-{(E)-2-[4-(trifluoromethyl)phenyl]ethenyl}-1,3-oxazole (212mg), a similar reaction to that of Reference Example B2 was performed to obtain the title compound (255mg).
mp 102-104 °C.
$^1$H-NMR($CDCl_3$)δ:2.08(2H, quintet, J=7.0Hz), 2.62(2H,t,J=7.4Hz), 2.72(1H, d, J=4.8Hz) 2 . 73(1H,d,J=7.6Hz), 3.63 (1H,dd,J=4.8Hz,11.4Hz), 3.74(1H,dd,J=4.2Hz,11.4Hz), 3.83(2H,t,J=7.2Hz), 4.13-4.24(1H,m), 5.03(2H,s), 6.77-6.91 (3H,m), 6.84(1H,d,J=1.4Hz), 6.94(1H,d,J=1.4Hz), 7.02 (1H,d,J=16.4Hz), 7.25(1H,t,J=7.8Hz), 7.57(1H,d,J=16.4Hz), 7.64(4H,s), 7.71(1H,s).
IR (KBr):3500-3200, 3108, 3056, 2932, 2867, 1613, 1599, 1586, 1534, 1489, 1451, 1416, 1325, 1260, 1167, 1125, 1069, 1030, 1017 $cm^{-1}$.

| Elemental analysis: for $C_{28}H_{28}N_3O_4F_3$ | | | |
|---|---|---|---|
| Cal'd(%) | C,63.75; | H,5.35; | N,7.97. |
| Found(%) | C,63.60; | H,5.32; | N,7.88. |

Reference Example B26

3-(1-{4-[4-({2-[(E)-2-(2,4-difluorophenyl)ethenyl]-1,3-oxazol-4-yl}methoxy)phenyl]butyl}-1H-imidazol-2-yl)-1,2-propanediol

Using 3-{1-[4-(4-hydroxyphenyl)butyl]-1H-imidazol-2-yl}-1,2-propanediol (204mg), 65% oily sodium hydride (28mg) and 4-(chloromethyl)-2-[(E)-2-(2,4-difluorophenyl)ethenyl]-1,3-oxazole (188mg), a similar reaction to that of Reference Example B2 was performed to obtain the title compound (223mg).
mp 126-128 °C.
$^1$H-NMR($CDCl_3$)δ:1.52-1.81(4H,m), 2.58(2H,t,J=6.9Hz), 2.77(2H, d, J=5.4Hz), 3.63(1H,dd,J=4.8Hz,11.4Hz), 3.75(1H, dd,J=4.2Hz,11.4Hz), 3.82(2H,t,J=7.0Hz), 4.10-4.24(1H,m), 5.01(2H,s), 6.76-7.02(7H,m), 7.07(2H,d,J=8.6Hz), 7.48-7.51(1H,m 7.59(1H,d,J=16.6Hz), 7.67(1H,s).
IR (KBr) :3500-3200, 3106, 3073, 3032, 2934, 2865, 1644, 1613, 1593, 1532, 1512, 1495, 1462, 1431, 1354, 1298, 1275, 1244, 1177, 1142, 1090, 1028 $cm^{-1}$.

| Elemental analysis: for $C_{28}H_{29}N_3O_4F_2$ | | | |
|---|---|---|---|
| Cal'd(%) | C,66.00; | H,5.74; | N,8.25. |
| Found(%) | C,65.89; | H,5.94; | N,8.37. |

Reference Example B27

3-(1-{3-[3-({2-[(E)-2-(2,4-difluorophenyl)ethenyl]-1,3-oxazol-4-yl}methoxy)phenyl]propyl}1H-imidazol-2-yl)-1,2-propanediol

Using 3-{1-[3-(3-hydroxyphenyl)propyl]-1H-imidazol-2-yl}-1,2-propanediol (203mg), 65% oily sodium hydride (29mg) and 4-(chloromethyl)-2-[(E)-2-(2,4-difluorophenyl)ethenyl]-1,3-oxazole (197mg), a similar reaction to that of Reference Example B2 was performed to obtain the title compound (220mg).
mp 92-94 °C.
$^1$H-NMR($CDCl_3$)δ:2.08(2H, quintet, J=7.2Hz), 2.62(2H,t,J=7.3Hz), 2.73(1H,d,J=5.0Hz), 2.74 (1H, d, J=7.0Hz), 3.63 (1H,dd,J=4.8Hz,11.2Hz), 3.74(1H,dd,J=4.2Hz,11.2Hz), 3.83(2H,t,J=7.4Hz), 4.14-4.24(1H,m), 5.02 (2H,s), 6.76-6.98 (5H,m), 6.84(1H,d,J=1.4Hz), 6.93(1H,d,J=1.4Hz), 6.98(1H, d, J=16.4Hz), 7.25(1H,t,J=7.9Hz), 7.48-7.61(1H,m), 7.60 (1H,d,J=16.4Hz), 7.69(1H,s).
IR (KBr):3500-3200, 3106, 3067, 3042, 2938, 2872, 1644, 1613, 1599, 1534, 1495, 1453, 1431, 1379, 1354, 1275, 1155, 1142, 1123, 1090, 1028 $cm^{-1}$.

| Elemental analysis: for $C_{27}H_{27}N_3O_4F_2$ | | | |
|---|---|---|---|
| Cal'd(%) | C,65.44; | H,5.49; | N,8.48. |
| Found(%) | C,65.39; | H,5.32; | N,8.62. |

Reference Example B28

3-[1-[4-[4-[[2-[(E)-2-(2,6-difluorophenyl) ethenyl] -1,3-oxazol-4-yl]methoxy]phenyl]butyl]-1H-imidazol-2-yl]-1,2-propanediol

Using 3-{1-[3-(3-hydroxyphenyl)propyl]-1H-imidazol-2-yl}-1,2-propanediol (142mg), 60% oily sodium hydride (40mg) and 4-(chlorophenyl)2-[(E)-2-(2,6-difluorophenyl)ethenyl]-1,3-oxazole (495mg), a similar reaction to that of Reference Example B2 was performed to obtain the title compound (395mg) as colorless crystals.
mp 123-125 °C.
$^1$H-NMR($CDCl_3$)δ: 1.5-1.8 (4H, m), 2.59 (2H, t, J = 7.0), 2.7-2.8 (2H, m), 3.6-3.75 (2H, m), 3.83 (2H, t, J = 7.0Hz), 4.1-4.25 (1H, m), 5.03 (2H, s), 6.8-7.0 (4H, m), 6.92 (2H, d, J = 8.6Hz), 7.07 (2H, d, J = 8.6Hz), 7.2-7.3 (1H, m) , 7.29 (1H, d, J = 16.8Hz), 7.61 (1H, d, J = 16.8Hz), 7.69 (1H, s).
IR (KBr): 1620, 1508, 1458, 1236, 1051, 1001, 789$cm^{-1}$.

| Elemental analysis: for $C_{28}H_{29}F_2N_3O_4$ | | | |
|---|---|---|---|
| Cal'd(%) | C,66.00; | H,5.74; | N,8.25. |
| Found(%) | C,65.71; | H,5.78; | N,8.09. |

Reference Example B29

(2R)-3-[[1-[4-[4-[[2-[(E)-2-(2,4-difluorophenyl)ethenyl]-1,3-oxazol-4-yl]methoxy]phenyl]butyl]-1H-imidazol-2-yl]-1,2-propanediol

60% oily sodium hydride (37mg) was added to a solution of (2R)-3-(1H-imidazol-2-yl)-1,2-propanediol (127mg) in DMF (4mL) under ice-cooling. After stir at room temperature for 30 minutes, 4-[[4-(4-iodobutyl)phenoxy] methyl]-2-[(E)-2-(2,4-difluorophenyl)ethenyl]-1,3-oxazole (485mg) was added under ice-cooling. After stir at room temperature for 3 hours, water was added under ice-cooling, extracted with a mixed solution of THF-ethyl acetate, washed with water and a brine, dried with magnesium sulfate, and concentrated under reduced pressure. The residue was purified by silica gel column chromatography (eluent; ethyl acetate : methanol=10:1), and recrystallized from ethyl acetate-hexane to obtain the title compound (262mg) as colorless crystals.

mp 104-106 °C.
$^1$H-NMR($CDCl_3$)δ: 1.5-1.8 (4H, m), 2.59 (2H, t, J = 7.0Hz), 2.7-2.8 (2H, m), 3.55-3.75 (2H, m), 3.79 (2H, t, J = 7.0Hz), 4.1-4.2 (1H, m), 5.01 (2H, s), 6.8-7.1 (5H, m), 6.92 (2H, d, J = 8.4Hz), 7.07 (2H, d, J = 8.4Hz), 7.5-7.6 (1H, m), 7.59 (1H, d, J = 16.2Hz), 7.67 (1H, s).
IR (KBr):1507, 1472, 1273, 1235, 1140, 1092, 966, 858cm$^{-1}$.

| Elemental analysis: for $C_{28}H_{29}F_2N_3O_4$ | | | |
|---|---|---|---|
| Cal'd(%) | C,66.00; | H,5.74; | N,8.25. |
| Found(%) | C,65.69; | H,5.82; | N,8.06. |

$[\alpha]^{22}_D$ = + 4.2° (c = 1.0, methanol).

Reference Example B30

(2S)-3-[[1-[4-[4-[[2-[(E)-2-(2,4-difluorophenyl)ethenyl]-1,3-oxazole-4-yl]methoxy]phenyl]butyl]-1H-imidazol-2-yl]-1,2-propanediol

Using (2S)-3-(1H-imidazol-2-yl)-1,2-propanediol, 60% oily sodium hydride (50mg) and 4-[[4-(4-iodobutyl)phenoxy]methyl]-2-[(E)-2-(2,4-difluorophenyl)ethenyl]-1,3-oxazole (415mg), a similar reaction to that of Reference Example B29 was performed to obtain the title compound (219mg) as colorless crystals.
mp 106-108 °C.
$^1$H-NMR($CDCl_3$)δ : 1.5-1.8 (4H, m), 2.58 (2H, t, J = 6.8Hz), 2.7-2.8 (2H, m), 3.6-3.75 (2H, m), 3.82 (2H, t, J = 7.0Hz), 4.1-4.2 (1H, m), 5.01 (2H, s), 6.8-7.1 (5H, m), 6.89 (2H, d, J = 8.4Hz), 7.07 (2H, d, J = 8.4Hz), 7.5-7.6 (1H, m), 7.59 (1H, d, J = 16.4Hz), 7.67 (1H, s).
IR(KBr) : 1615, 1512, 1497, 1273, 1246, 1229, 1140, 1094, 1046, 966, 847cm$^{-1}$.

| Elemental analysis: for $C_{28}H_{29}F_2N_3O_4$ | | | |
|---|---|---|---|
| Cal'd(%) | C,66.00; | H,5.74; | N,8.25. |
| Found(%) | C,65.75; | H,5.60; | N,8.12. |

$[\alpha]^{22}_D$ = - 3.5° (c = 1.0, methanol).

Reference Example C1

Inhibition of phosphorylation of tyrosine in receptor of human breast cancer cell

500μl of a suspension of human breast cancer cell MCF-7 (3000,000 cells) was seeded on a 24-well plate, cultured at 37°C in a 5% carbonic acid gas incubator. Next day, 250μm of a 4-fold stepwisely diluted test compound solution was added, after 2 hours, 250μl of a halegrin solution adjusted to a final concentration of 0.8μg/mL was added. After 5 minutes, an extracting solution was added to stop the reaction, and the protein was extracted. This protein was fractionated by electrophoresis of proteins, and the protein in an electrophoresis gel was transferred to a nylon filter. This filter and a phosphorylated tyrosine-specific antibody were reacted, and the reaction product was fluorescently labeled to react a photosensitive film. The sensitized amount of the photosensitive film was quantitated with an image analyzing apparatus. Assuming that an amount of phosphorylated HER2 tyrosine of cells in a group to which halegrin had been added was 100%, a ratio of an amount of phosphorylated HER2 tyrosine of cells was obtained in a group to which a test compound solution at each concentration was added, and the concentration of the test compound necessary to suppress the amount of phosphorylated HER2 tyrosine to 50% of that of the control ($IC_{5o}$ value) was calculated.
The results are shown in [Table 1].
From this, it was demonstrated that the test compound strongly inhibits the phosphorylation reaction of the tyrosine residue in the receptor protein, which is caused by activation of receptor tyrosinekinase accompanied with growth factor stimulation when human breast cancer cells receive stimulation from growth factor halegrin.

[Table 1]

| Reference Example No. (Compound No.) | Intracellular HER2 phosphorylation inhibition MCF-7 ($IC_{50}$: μM) |
|---|---|
| B2 | 1.9 |
| B3 | 0.18 |
| B4 | 0.10 |
| B6 | 1.2 |
| B11 | 1.1 |
| B20 | 1.5 |
| B22 | 1.9 |
| B26 | 0.92 |

Reference Example C2

In vitro inhibitory activity on growth of breast cancer cell BT-474

100μl of a suspension of human breast cancer cell BT-474 (1,000 cells) was seeded on a 96-well microplate, and cultured at 37°C in a 5% carbonic acid gas incubator. Next day, 100μl of each test compound solution which had been 2-fold stepwisely diluted with a halegrin solution with the final concentration of which was adjusted to 0.04 μg/mL was added, and cultured for 5 days. A culture solution containing a test compound was removed, cells were washed, and fixed with a 50% trichloroacetic acid solution, then a pigment SRB 0.4% (w/v) solution (dissolved in 1% acetic acid) was added to fix and stain cell proteins (Skehan et al, Journal of National Cancer Institute vol., pp. 1107-1112, 1990). The pigment solution was removed, washed with a 1% acetic acid solution, 100μl of an extracting solution (10mM Tris buffer) was added to extract the pigment, and absorbance at an absorption wavelength of 550nm was measured to determine an amount of cells as a protein mass. Assuming that a protein mass in a control group to which no test compound solution had been added was 100%, a ratio of a mass of remaining proteins in each treated group was obtained, and the concentration of the compound necessary to suppress the amount of remaining cells to 50% of that of the control ($IC_{50}$) was calculated.

The results are shown in [Table 2].

From this, it was shown that the test compound strongly inhibits growth of human breast cancer cell strain BT-474.

[Table 2]

| Reference Example No. (Compound No.) | Cell growth inhibition BT-474 ($IC_{50}$: μM) |
|---|---|
| B 2 | <0.05 |
| B 3 | <0.05 |
| B 4 | <0.05 |
| B 6 | <0.05 |
| B 1 1 | <0.05 |
| B 1 9 | 0.017 |
| B 2 0 | <0.05 |
| B 2 2 | <0.05 |
| B 2 6 | <0.05 |

Reference Example C3

In vivo inhibitory activity on breast cancer growth

Five million cells of human breast cancer cell BT-474 were suspended in a matrigel solution, and transplanted to Balb/C line male nude mouse (6 week age) transdermally at a chest (Freedman et al., Proceedings of National Academy of Science USA vol. 87, pp.6698-6702, 1990). In order to enhance a take rate of tumor at a time of transplantation and 7 days after transplantation, 50μL of estradiol dipropionate (5mg/mL) was intramuscularly administered at a rear paw. Fourteen days after transplantation, a diameter of tumor was measured, and five mice having similar tumor size were used in an experiment per group. A suspension of the present Compound (4, 6, 14, 17, 19, 20, 23, 24, 26) in 5% gum arabic (saline solution) was orally administered at the concentration of 30mg/kg twice a day for 10 days. At completion of administration, a diameter of tumor was measured, and a tumor volume was calculated by:

$$\text{Formula: Tumor volume} = \text{long diameter} \times \text{short diameter} \times \text{short diameter} \times (1/2).$$

A ratio of the value obtained by subtracting the tumor volume on the day of administration initiation from the tumor volume on the day of administration completion in the gum arabic solution-administered control group, to the value obtained by subtracting the tumor volume on the day of administration initiation from the tumor volume on the day of administration completion in the drug-administered group, was calculated as the growth rate.
The results are shown in [Table 3].
The test compound inhibited growth of human breast cancer cells transplanted to nude mouse. In addition, the weight of mouse was measured over an experimental period, and no decrease in the weight was recognized due to administration of the test compound.

[Table 3]

| Reference Example No. (Compound No.) | Growth rate (%) |
|---|---|
| B4 | 5 |
| B6 | 28 |
| B23 | 27 |
| B24 | 28 |
| B26 | 15 |

Example 1a

| | |
|---|---|
| Compound of Reference Example B4 | 0.1g |
| Polyvinylpyrrolidone | 0.8g |
| Lactose | 0.1g |
| Total | 1.0g |

The compound of Reference Example B4 (0.1g) was dissolved in methylene chloride (10mL), and polyvinylpyrrolidone (K-30, Wako Pure Chemical Industries, Ltd., 0.8g) was added thereto and dissolved. Lactose (0.1g) was added to this solution and suspended uniformly, and the organic solvent was evaporated. The residue product was dried for 14 hours under reduced pressure in a vacuum drier, and ground with a ball mill for 90 minutes to obtain a solid dispersion (0.55g) of the compound of Reference Example B4.

Example 1b

| | |
|---|---|
| Compound of Reference Example B6 | 0.1g |
| Polyvinylpyrrolidone | 0.8g |
| Lactose | 0.1g |
| Total | 1.0g |

The compound of Reference Example B6 (0.1g) is dissolved in methylene chloride (10mL) and polyvinylpyrrolidone (K-30, Wako Pure Chemical Industries, Ltd., 0.8g) is added thereto and dissolved. Lactose (0.1g) is added to this solution and suspended uniformly, and the organic solvent is evaporated. The residue product is dried for 14 hours under reduced pressure in a vacuum drier, and ground with a ball mill for 90 minutes to obtain a solid dispersion of the compound of Reference Example B6.

Example 1c

| | |
|---|---|
| Compound of Reference Example B26 | 0.1g |
| Polyvinylpyrrolidone | 0.8g |
| Lactose | 0.1g |
| Total | 1.0g |

The compound of Reference Example B26 (0.1g) is dissolved in methylene chloride (10mL) and polyvinylpyrrolidone (K-30, Wako Pure Chemical Industries, Ltd., 0.8g) is added thereto and dissolved. Lactose (0.1g) was added to this solution and suspended uniformly, and the organic solvent was evaporated. The residue product is dried for 14 hours under reduced pressure in a vacuum drier, and ground with a ball mill for 90 minutes to obtain a solid dispersion of the compound of Reference Example B26.

Example 2a

| | |
|---|---|
| Compound of Reference Example B4 | 0.1g |
| Hydroxypropylmethylcellulose phthalate | 0.8g |
| Lactose | 0.1g |
| Total | 1.0g |

The compound of Reference Example B4 (0.1g) was dissolved in a mixed solution of methylene chloride (9.4mL) and ethanol (0.6mL), and hydroxypropylmethylcellulose phthalate (HP-50, Shin-Etsu Chemical Co., Ltd., 0.8g) was added thereto and dissolved. Lactose (0.1g) was added to this solution and suspended uniformly, and the organic solvent was evaporated. The residue product was dried for 14 hours under reduced pressure in a vacuum drier, and ground with a ball mill for 90 minutes to obtain a solid dispersion (0.61g) of the compound of Reference Example B4.

Example 2b

| | |
|---|---|
| Compound of Reference Example B6 | 0.1g |
| Hydroxypropylmethylcellulose phthalate | 0.8g |
| Lactose | 0.1g |
| Total | 1.0g |

The compound of Reference Example B6 (0.1g) is dissolved in a mixed solution of methylene chloride (9.4mL)

and ethanol (0.6mL), and hydroxypropylmethylcellulose phthalate (HP-50, Shin-Etsu Chemical Co., Ltd., 0.8g) is added thereto and dissolved. Lactose (0.1g) is added to this solution and suspended uniformly, and the organic solvent is evaporated. The residue product is dried for 14 hours under reduced pressure in a vacuum drier, and ground with a ball mill for 90 minutes to obtain a solid dispersion of the compound of Reference Example B6.

Example 2c

| | |
|---|---|
| Compound of Reference Example B6 | 0.1g |
| Hydroxypropylmethylcellulose phthalate | 0.8g |
| Lactose | 0.1g |
| Total | 1.0g |

The compound of Reference Example B26 (0.1g) is dissolved in a mixed solution of methylene chloride (9.4mL) and ethanol (0.6mL), and hydroxypropylmethylcellulose phthalate (HP-50, Shin-Etsu Chemical Co., Ltd., 0.8g) is added thereto and dissolved. Lactose (0.1g) is added to this solution and suspended uniformly, and the organic solvent is evaporated. The residue product is dried for 14 hours under reduced pressure in a vacuum drier, and ground with a ball mill for 90 minutes to obtain a solid dispersion of the compound of Reference Example B26.

Example 3a

| | |
|---|---|
| Compound of Reference Example B4 | 0.4g |
| Hydroxypropylmethylcellulose phthalate | 1.4g |
| Lactose | 0.2g |
| Total | 2.0g |

The compound of Reference Example B4 (0.4g) was dissolved in a mixed solution of methylene chloride (24mL) and ethanol (16mL), and hydroxypropylmethylcellulose phthalate (HP-50, Shin-Etsu Chemical Co., Ltd., 1.4g) was added thereto and dissolved. Lactose (0.2g) was added to this solution and suspended uniformly, and the organic solvent was evaporated using a spray drier (manufactured by Yamato Scientific Co., Ltd.). The product was dried for 14 hours under reduced pressure in a vacuum drier to obtain a solid dispersion (1.2g) of the compound of Reference Example B4.

Example 3b

| | |
|---|---|
| Compound of Reference Example B6 | 0.4g |
| Hydroxypropylmethylcellulose phthalate | 1.4g |
| Lactose | 0.2g |
| Total | 2.0g |

The compound of Reference Example B6 (0.4g) is dissolved in a mixed solution of methylene chloride (24mL) and ethanol (16mL), and hydroxypropylmethylcellulose phthalate (HP-50, Shin-Etsu Chemical Co., Ltd., 1.4g) is added thereto and dissolved. Lactose (0.2g) is added to this solution and suspended uniformly, and the organic solvent is evaporated using a spray drier (manufactured by Yamato Scientific Co., Ltd.). The product is dried for 14 hours under reduced pressure in a vacuum drier to obtain a solid dispersion of the compound of Reference Example B6.

Example 3c

| | |
|---|---|
| Compound of Reference Example B26 | 0.4g |
| Hydroxypropylmethylcellulose phthalate | 1.4g |
| Lactose | 0.2g |
| Total | 2.0g |

The compound of Reference Example B26 (0.4g) is dissolved in a mixed solution of methylene chloride (24mL) and ethanol (16mL)/ and hydroxypropylmethylcellulose phthalate (HP-50, Shin-Etsu Chemical Co., Ltd., 1.4g) is added thereto and dissolved. Lactose (0.2g) is added to this solution and suspended uniformly, and the organic solvent is evaporated using a spray drier (manufactured by Yamato Scientific Co., Ltd.). The product is dried for 14 hours under reduced pressure in a vacuum drier to obtain a solid dispersion of the compound of Reference Example B26.

Example 4a

| | |
|---|---|
| Compound of Reference Example B4 | 0.4g |
| Hydroxypropylmethylcellulose phthalate | 1.4g |
| Lactose | 0.2g |
| Total | 2.0g |

The compound of Reference Example B4 (0.4g) was dissolved in acetone (20mL) and ethanol (20mL), and hydroxypropylmethylcellulose phthalate (HP-50, Shin-Etsu Chemical Co., Ltd., 1.4g) was added thereto and dissolved. Lactose (0.2g) was added to this solution and suspended uniformly, and the organic solvent was evaporated using a spray drier (manufactured by Yamato Scientific Co., Ltd.). The product was dried for 14 hours under reduced pressure in a vacuum drier to obtain a solid dispersion (1.2g) of the compound of Reference Example B4.

Example 4b

| | |
|---|---|
| Compound of Reference Example B6 | 0.4g |
| Hydroxypropylmethylcellulose phthalate | 1.4g |
| Lactose | 0.2g |
| Total | 2.0g |

The compound of Reference Example B6 (0.4g) is dissolved in acetone (20mL) and ethanol (20mL), and hydroxypropylmethylcellulose phthalate (HP-50, Shin-Etsu Chemical Co., Ltd., 1.4g) is added thereto and dissolved. Lactose (0.2g) is added to this solution and suspended uniformly, and the organic solvent was evaporated using a spray drier (manufactured by Yamato Scientific Co., Ltd.). The product is dried for 14 hours under reduced pressure in a vacuum drier to obtain a solid dispersion of the compound of Reference Example B6.

Example 4c

| | |
|---|---|
| Compound of Reference Example B26 | 0.4g |
| Hydroxypropylmethylcellulose phthalate | 1.4g |
| Lactose | 0.2g |
| Total | 2.0g |

The compound of Reference Example B26 (0.4g) is dissolved in acetone (20mL) and ethanol (20mL), and

hydroxypropylmethylcellulose phthalate (HP-50, Shin-Etsu Chemical Co., Ltd., 1.4g) is added thereto and dissolved. Lactose (0.2g) is added to this solution and suspended uniformly, and the organic solvent was evaporated using a spray drier (manufactured by Yamato Scientific Co., Ltd.). The product is dried for 14 hours under reduced pressure in a vacuum drier to obtain a solid dispersion of the compound of Reference Example B26.

Example 5a

| | |
|---|---|
| Compound of Reference Example B4 | 0.4g |
| Hydroxypropylmethylcellulose phthalate | 1.6g |
| Total | 2.0g |

The compound of Reference Example B4 (0.4g) was dissolved in acetone(20mL) and ethanol (20mL), and hydroxypropylmethylcellulose phthalate (HP-50, Shin-Etsu Chemical Co., Ltd., 1.6g) was added thereto and dissolved, and the organic solvent was evaporated using a spray drier (manufactured by Yamato Scientific Co., Ltd.). The product was dried for 14 hours under reduced pressure in a vacuum drier to obtain a solid dispersion (1.2g) of the compound of Reference Example B4.

Example 5b

| | |
|---|---|
| Compound of Reference Example B6 | 0.4g |
| Hydroxypropylmethylcellulose phthalate | 1.6g |
| Total | 2.0g |

The compound of Reference Example B6 (0.4g) is dissolved in acetone (20mL) and ethanol (20mL), and hydroxypropylmethylcellulose phthalate (HP-50, Shin-Etsu Chemical Co., Ltd., 1.6g) is added thereto and dissolved, and the organic solvent is evaporated using a spray drier (manufactured by Yamato Scientific Co., Ltd.). The product is dried for 14 hours under reduced pressure in a vacuum drier to obtain a solid dispersion of the compound of Reference Example B6.

Example 5c

| | |
|---|---|
| Compound of Reference Example B26 | 0.4g |
| Hydroxypropylmethylcellulose phthalate | 1.6g |
| Total | 2.0g |

The compound of Reference Example B26 (0.4g) is dissolved in acetone (20mL) and ethanol (20mL), and hydroxypropylmethylcellulose phthalate (HP-50, Shin-Etsu Chemical Co., Ltd., 1.6g) is added thereto and dissolved, and the organic solvent is evaporated using a spray drier (manufactured by Yamato Scientific Co., Ltd.). The product is dried for 14 hours under reduced pressure in a vacuum drier to obtain a solid dispersion of the compound of Reference Example B26.

Example 6a

| | |
|---|---|
| Compound of Reference Example B4 | 0.4g |
| Carboxymethylethylcellulose | 1.6g |
| Total | 2.0g |

The compound of Reference Example B4 (0.4g) was dissolved in acetone (20mL) and ethanol (20mL), and

carboxymethylethylcellulose (manufactured by Freund Industrial Co., Ltd., 1.6g) was added thereto and dissolved, and the organic solvent was evaporated using a spray drier (manufactured by Yamato Scientific Co., Ltd.). The product was dried for 14 hours under reduced pressure in a vacuum drier to obtain a solid dispersion(1.2g) of the compound of Reference Example B4.

Example 6b

| | |
|---|---|
| Compound of Reference Example B6 | 0.4g |
| Carboxymethylethylcellulose | 1.6g |
| Total | 2.0g |

The compound of Reference Example B6 (0.4g) is dissolved in acetone (20mL) and ethanol (20mL), and carboxymethylethylcellulose (manufactured by Freund Industrial Co., Ltd., 1.6g) is added thereto and dissolved, and the organic solvent is evaporated using a spray drier (manufactured by Yamato Scientific Co., Ltd.). The product is dried for 14 hours under reduced pressure in a vacuum drier to obtain a solid dispersion of the compound of Reference Example B6.

Example 6c

| | |
|---|---|
| Compound of Reference Example B26 | 0.4g |
| Carboxymethylethylcellulose | 1.6g |
| Total | 2.0g |

The compound of Reference Example B26 (0.4g) is dissolved in acetone (20mL) and ethanol (20mL), and carboxymethylethylcellulose (manufactured by Freund Industrial Co., Ltd., 1.6g) is added thereto and dissolved, and the organic solvent is evaporated using a spray drier (manufactured by Yamato Scientific Co., Ltd.). The product is dried for 14 hours under reduced pressure in a vacuum drier to obtain a solid dispersion of the compound of Reference Example B26.

Example 7a

| | |
|---|---|
| Compound of Reference Example B4 | 0.4g |
| Eudragit | 1.6g |
| Total | 2.0g |

The compound of Reference Example B4 (0.4g) was dissolved in acetone (20mL) and ethanol (20mL), and Eudragit (L100-55, manufactured by Rohm GmbH GmbH, 1.6g) was added thereto and dissolved, and the organic solvent was evaporated using a spray drier (manufactured by Yamato Scientific Co., Ltd.). The product was dried for 14 hours under reduced pressure in a vacuum drier to obtain a solid dispersion (1.2g) of the compound of Reference Example B4.

Example 7b

| | |
|---|---|
| Compound of Reference Example B6 | 0.4g |
| Eudragit | 1.6g |
| Total | 2.0g |

The compound of Reference Example B6 (0.4g) is dissolved in acetone (20mL) and ethanol (20mL), and

Eudragit (L100-55, manufactured by Rohm GmbH, 1.6g) is added thereto and dissolved, and the organic solvent is evaporated using a spray drier (manufactured by Yamato Scientific Co., Ltd.). The product is dried for 14 hours under reduced pressure in a vacuum drier to obtain a solid dispersion of the compound of Reference Example B6.

Example 7c

| | |
|---|---|
| Compound of Reference Example B26 | 0.4g |
| Eudragit | 1.6g |
| Total | 2.0g |

The compound of Reference Example B26 (0.4g) is dissolved in acetone (20mL) and ethanol (20mL), and Eudragit (L100-55, manufactured by Rohm GmbH, 1.6g) is added thereto and dissolved, and the organic solvent is evaporated using a spray drier (manufactured by Yamato Scientific Co., Ltd.). The product is dried for 14 hours sunder reduced pressure in a vacuum drier to obtain a solid dispersion of the compound of Reference Example B26.

Example 8a

| | |
|---|---|
| Compound of Reference Example B4 | 1.5g |
| Hydroxypropylmethylcellulose phthalate | 3.5g |
| Total | 5.0g |

The compound of Reference Example B4 (1.5g) was dissolved in acetone (75mL) and ethanol (25mL), and hydroxypropylmethylcellulose phthalate (HP-55, manufactured by Shin-Etsu Chemical Co., Ltd., 3.5g) was added thereto and dissolved, and the organic solvent was evaporated using a spray drier (manufactured by Yamato Scientific Co., Ltd.). The product was dried for 14 hours under reduced pressure in a vacuum drier to obtain a solid dispersion (3.5g) of the compound of Reference Example.B4.

Example 8b

| | |
|---|---|
| Compound of Reference Example B6 | 1.5g |
| Hydroxypropylmethylcellulose phthalate | 3.5g |
| Total | 5.0g |

The compound of Reference Example B6 (1.5g) is dissolved in acetone (75mL) and ethanol (25mL), and hydroxypropylmethylcellulose phthalate (HP-55, manufactured by Shin-Etsu Chemical Co., Ltd., 3.5g) is added thereto and dissolved, and the organic solvent is evaporated using a spray drier (manufactured by Yamato Scientific Co., Ltd.). The product is dried for 14 hours under reduced pressure in a vacuum drier to obtain a solid dispersion of the compound of Reference Example B6.

Example 8c

| | |
|---|---|
| Compound of Reference Example B26 | 1.5g |
| Hydroxypropylmethylcellulose phthalate | 3.5g |
| Total | 5.0g |

The compound of Reference Example B26 (1.5g) is dissolved in acetone (75mL) and ethanol (25mL), and hydroxypropylmethylcellulose phthalate (HP-55, manufactured by Shin-Etsu Chemical Co., Ltd., 3.5g) is added thereto and dissolved, and the organic solvent is evaporated using a spray drier (manufactured by Yamato Scientific Co., Ltd.).

The product is dried for 14 hours under reduced pressure in a vacuum drier to obtain a solid dispersion of the compound of Reference Example B26.

Example 9a

| | |
|---|---|
| Compound of Reference Example B4 | 1.5g |
| Carboxymethylethylcellulose | 3.5g |
| Total | 5.0g |

The compound of Reference Example B4 (1.5g) was dissolved in acetone (75mL) and ethanol (25mL), and carboxymethylethylcellulose (manufactured by Freund Industrial Co., Ltd., 3.5g) was added thereto and dissolved, and the organic solvent was evaporated using a spray drier (manufactured by Yamato Scientific Co., Ltd.). The product was dried for 14 hours under reduced pressure in a vacuum drier to obtain a solid dispersion (3.5g) of the compound of Reference Example B4.

Example 9b

| | |
|---|---|
| Compound of Reference Example B6 | 1.5g |
| Carboxymethylethylcellulose | 3.5g |
| Total | 5.0g |

The compound of Reference Example B6 (1.5g) is dissolved in acetone (75mL) and ethanol (25mL), and carboxymethylethylcellulose (manufactured by Freund Industrial Co., Ltd., 3.5g) is added thereto and dissolved, and the organic solvent is evaporated using a spray drier (manufactured by Yamato Scientific Co., Ltd.). The product is dried for 14 hours under reduced pressure in a vacuum drier to obtain a solid dispersion of the compound of Reference Example B6.

Example 9c

| | |
|---|---|
| Compound of Reference Example B26 | 1.5g |
| Carboxymethylethylcellulose | 3.5g |
| Total | 5.0g |

The compound of Reference Example B26 (1.5g) is dissolved in acetone (75mL) and ethanol (25mL), and carboxymethylethylcellulose (manufactured by Freund Industrial Co., Ltd., 3.5g) is added thereto and dissolved, and the organic solvent is evaporated using a spray drier (manufactured by Yamato Scientific Co., Ltd.). The product is dried for 14 hours under reduced pressure in a vacuum drier to obtain a solid dispersion of the compound of Reference Example B26.

Example 10a

| | |
|---|---|
| Compound of Reference Example B4 | 1.5g |
| Eudragit | 3.5g |
| Total | 5.0g |

The compound of Reference Example B4 (1.5g) was dissolved in acetone (75mL) and ethanol (25mL), and Eudragit (L100-55, manufactured by Rohm GmbH, 3.5g) was added thereto and dissolved, and the organic solvent was evaporated using a spray drier (manufactured by Yamato Scientific Co., Ltd.). The product was dried for 14 hours

under reduced pressure in a vacuum drier to obtain a solid dispersion (3.5g) of the compound of Reference Example B4.

Example 10b

| | |
|---|---|
| Compound of Reference Example B6 | 1.5g |
| Eudragit | 3.5g |
| Total | 5.0g |

The compound of Reference Example B6 (1.5g) is dissolved in acetone (75mL) and ethanol (25mL), and Eudragit (L100-55, manufactured by Rohm GmbH, 3.5g) is added thereto and dissolved, and the organic solvent was evaporated using a spray drier (manufactured by Yamato Scientific Co., Ltd.). The product is dried for 14 hours under reduced pressure in a vacuum drier to obtain a solid dispersion of the compound of Reference Example B6.

Example 10c

| | |
|---|---|
| Compound of Reference Example B26 | 1.5g |
| Eudragit | 3.5g |
| Total | 5.0g |

The compound of Reference Example B26 (1.5g) is dissolved in acetone (75mL) and ethanol (25mL), and Eudragit (L100-55, manufactured by Rohm GmbH, 3.5g) is added thereto and dissolved, and the organic solvent is evaporated using a spray drier (manufactured by Yamato Scientific Co., Ltd.). The product is dried for 14 hours under reduced pressure in a vacuum drier to obtain a solid dispersion of the compound of Reference Example B26.

Example 11a

| | |
|---|---|
| Compound of Reference Example B4 | 1.65g |
| Hydroxypropylmethylcellulose phthalate | 3.35g |
| Total | 5.0g |

The compound of Reference Example B4 (1.65g) was dissolved in acetone (75mL) and ethanol (25mL), and hydroxypropylmethylcellulose phthalate (HP-55, manufactured by Shin-Etsu Chemical Co., Ltd., 3.35g) was added thereto and dissolved, and the organic solvent was evaporated using a spray drier (manufactured by Yamato Scientific Co., Ltd.). The product was dried for 14 hours under reduced pressure in a vacuum drier to obtain a solid dispersion (3.5g) of the compound of Reference Example B4.

Example 11b

| | |
|---|---|
| Compound of Reference Example B6 | 1.65g |
| Hydroxypropylmethylcellulose phthalate | 3.35g |
| Total | 5.0g |

The compound of Reference Example B6 (1.65g) is dissolved in acetone (75mL) and ethanol (25mL), and hydroxypropylmethylcellulose phthalate (HP-55, manufactured by Shin-Etsu Chemical Co., Ltd., 3.35g) was added thereto and dissolved, and the organic solvent was evaporated using a spray drier (manufactured by Yamato Scientific Co., Ltd.). The product was dried for 14 hours under reduced pressure in a vacuum drier to obtain a solid dispersion of the compound of Reference Example B6.

Example 11c

| | |
|---|---|
| Compound of Reference Example B26 | 1.65g |
| Hydroxypropylmethylcellulose phthalate | 3.35g |
| Total | 5.0g |

The compound of Reference Example B26 (1.65g) is dissolved in acetone (75mL) and ethanol (25mL), and hydroxypropylmethylcellulose phthalate (HP-55, manufactured by Shin-Etsu Chemical Co., Ltd., 3.35g) is added thereto and dissolved, and the organic solvent is evaporated using a spray drier (manufactured by Yamato Scientific Co., Ltd.). The product is dried for 14 hours under reduced pressure in a vacuum drier to obtain a solid dispersion of the compound of Reference Example B26.

Example 12a

| | |
|---|---|
| Compound of Reference Example B4 | 1.65g |
| Eudragit | 3.35g |
| Total | 5.0g |

The compound of Reference Example B4 (1.65g) was dissolved in acetone (75mL) and ethanol (25mL), and Eudragit (L100-55, manufactured by Rohm GmbH, 3.35g) was added thereto and dissolved, and the organic solvent was evaporated using a spray drier (manufactured by Yamato Scientific Co., Ltd.). The product was dried for 14 hours under reduced pressure in a vacuum drier to obtain a solid dispersion (3.5g) of the compound of Reference Example B4.

Example 12b

| | |
|---|---|
| Compound of Reference Example B6 | 1.65g |
| Eudragit | 3.35g |
| Total | 5.0g |

The compound of Reference Example B6 (1.65g) is dissolved in acetone (75mL) and ethanol (25mL), and Eudragit (L100-55, manufactured by Rohm GmbH, 3.35g) is added thereto and dissolved, and the organic solvent is evaporated using a spray drier (manufactured by Yamato Scientific Co., Ltd.). The product is dried for 14 hours under reduced pressure in a vacuum drier to obtain a solid dispersion of the compound of Reference Example B6.

Example 12c

| | |
|---|---|
| Compound of Reference Example B26 | 1.65g |
| Eudragit | 3.35g |
| Total | 5.0g |

The compound of Reference Example B26 (1.65g) is dissolved in acetone (75mL) and ethanol (25mL), and Eudragit (L100-55, manufactured by Rohm GmbH, 3.35g) is added thereto and dissolved, and the organic solvent is evaporated using a spray drier (manufactured by Yamato Scientific Co., Ltd.). The product is dried for 14 hours under reduced pressure in a vacuum drier to obtain a solid dispersion of the compound of Reference Example B26.

Example 13a

| | |
|---|---|
| Compound of Reference Example B4 | 12.5kg |
| Hydroxypropylmethylcellulose phthalate | 50.0kg |
| Total | 62.5kg |

The compound of Reference Example B4 (12.5kg) was dissolved in acetone (625L) and ethanol (625L), hydroxypropylmethylcellulose phthalate (HP-55, manufactured by Shin-Etsu Chemical Co., Ltd., 50.0kg) was added thereto and dissolved, and the organic solvent was evaporated using a spray drier (manufactured by Nippon Sharyo). The product was dried for 20 hours under reduced pressure in a vacuum drier to obtain a solid dispersion (47.2kg) of the compound of Reference Example B4.

Example 13b

| | |
|---|---|
| Compound of Reference Example B6 | 12.5kg |
| Hydroxypropylmethylcellulose phthalate | 50.0kg |
| Total | 62.5kg |

The compound of Reference Example B6 (12.5kg) is dissolved in acetone (625L) and ethanol (625L), hydroxypropylmethylcellulose phthalate (HP-55, manufactured by Shin-Etsu Chemical Co., Ltd., 50.0kg) is added thereto and dissolved, and the organic solvent is evaporated using a spray drier (manufactured by Nippon Sharyo). The product is dried for 20 hours under reduced pressure in a vacuum drier to obtain a solid dispersion of the compound of Reference Example B6.

Example 13c

| | |
|---|---|
| Compound of Reference Example B26 | 12.5kg |
| Hydroxypropylmethylcellulose phthalate | 50.0kg |
| Total | 62.5kg |

The compound of Reference Example B26 (12.5kg) is dissolved in acetone (625L) and ethanol (625L), hydroxypropylmethylcellulose phthalate (HP-55, manufactured by Shin-Etsu Chemical Co., Ltd., 50.0kg) is added thereto and dissolved, and the organic solvent is evaporated using a spray drier (manufactured by Nippon Sharyo). The product is dried for 20 hours under reduced pressure in a vacuum drier to obtain a solid dispersion of the compound of Reference Example B26.

Example 14a

| | |
|---|---|
| Compound of Reference Example B4 | 0.6kg |
| Hydroxypropylmethylcellulose phthalate | 1.4kg |
| Total | 2.0kg |

The compound of Reference Example B4 (0.6kg) was dissolved in acetone (25L) and ethanol (25L), hydroxypropylmethylcellulose phthalate (HP-55, manufactured by Shin-Etsu Chemical Co., Ltd., 1.4kg) was added thereto and dissolved, and the organic solvent was evaporated using a spray drier (manufactured by Nippon Sharyo). The product was dried for 20 hours under reduced pressure in a vacuum drier to obtain a solid dispersion (1.4kg) of the compound of Reference Example B4.

Example 14b

| | |
|---|---|
| Compound of Reference Example B6 | 0.6kg |
| Hydroxypropylmethylcellulose phthalate | 1.4kg |
| Total | 2.0kg |

The compound of Reference Example B6 (0.6kg) is dissolved in acetone (25L) and ethanol (25L), hydroxypropylmethylcellulose phthalate (HP-55, manufactured by Shin-Etsu Chemical Co., Ltd., 1.4kg) is added thereto and dissolved, and the organic solvent is evaporated using a spray drier (manufactured by Nippon Sharyo). The product is dried for 20 hours under reduced pressure in a vacuum drier to obtain a solid dispersion of the compound of Reference Example B6.

Example 14c

| | |
|---|---|
| Compound of Reference Example B26 | 0.6kg |
| Hydroxypropylmethylcellulose phthalate | 1.4kg |
| Total | 2.0kg |

The compound of Reference Example B26 (0.6kg) is dissolved in acetone (25L) and ethanol (25L), hydroxypropylmethylcellulose phthalate (HP-55, manufactured by Shin-Etsu Chemical Co., Ltd., 1.4kg) is added thereto and dissolved, and the organic solvent is evaporated using a spray drier (manufactured by Nippon Sharyo). The product is dried for 20 hours under reduced pressure in a vacuum drier to obtain a solid dispersion of the compound of Reference Example B26.

Example 15a

| | |
|---|---|
| Compound of Reference Example B4 | 0.67kg |
| Eudragit | 1.33kg |
| Total | 2.0kg |

The compound of Reference Example B4 (0.67kg) was dissolved in acetone (27.8L) and ethanol (27.8L), Eudragit (L100-55, manufactured by Rohm GmbH, 1.33kg) was added thereto and dissolved, and the organic solvent was evaporated using a spray drier (manufactured by Nippon Sharyo). The product was dried for 20 hours under reduced pressure in a vacuum drier to obtain a solid dispersion (1.4kg) of the compound of Reference Example B4.

Example 15b

| | |
|---|---|
| Compound of Reference Example B6 | 0.67kg |
| Eudragit | 1.33kg |
| Total | 2.0kg |

The compound of Reference Example B6 (0.67kg) is dissolved in acetone (27.8L) and ethanol (27.8L), Eudragit (L100-55, manufactured by Rohm GmbH, 1.33kg) is added thereto and dissolved, and the organic solvent is evaporated using a spray drier (manufactured by Nippon Sharyo). The product is dried for 20 hours under reduced pressure in a vacuum drier to obtain a solid dispersion of the compound of Reference Example B6.

Example 15c

| | |
|---|---|
| Compound of Reference Example B26 | 0.67kg |
| Eudragit | 1.33kg |
| Total | 2.0kg |

The compound of Reference Example B26 (0.67kg) is dissolved in acetone (27.8L) and ethanol (27.8L), Eudragit (L100-55, manufactured by Rohm GmbH, 1.33kg) is added thereto and dissolved, and the organic solvent is evaporated using a spray drier (manufactured by Nippon Sharyo). The product is dried for 20 hours under reduced pressure in a vacuum drier to obtain a solid dispersion of the compound of Reference Example B26.

Example 16a

The solid dispersion (2044g) obtained in Example 13a, lactose (6928g), crystalline cellulose (13880g), light silicic acid anhydride (144g), calcium carmelose (720g), and magnesium stearate (288g) were placed in a tumbler mixing machine, and mixed for 30 minutes, and compressed with a tabletting machine to obtain tablets having a long diameter 14mm x a short diameter 8mm. The tablets were ground with a power mill, mixed again with a tumbler mixing machine, and compressed with a tabletting machine to obtain 30000 tablets having a long diameter 14mm x a shorter diameter 8mm.

Example 16b

The solid dispersion (2044g) obtained in Example 13b, lactose (6928g), crystalline cellulose (13880g), light silicic acid anhydride (144g), calcium carmelose (720g), and magnesium stearate (288g) are placed in a tumbler mixing machine, mixed for 30 minutes, and compressed with a tabletting machine to obtain tablets having a long diameter 14mm x a short diameter 8mm. The tablets are ground with a power mill, mixed again with a tumbler mixing machine, and compressed with a tabletting machine to obtain tablets having a long diameter 14mm x a shorter diameter 8mm.

Example 16c

The solid dispersion (2044g) obtained in Example 13c, lactose (6928g), crystalline cellulose (13880g), light silicic acid anhydride (144g), calcium carmelose (720g), and magnesium stearate (288g) are placed in a tumbler mixing machine, mixed for 30 minutes, and compressed with a tabletting machine to obtain tablets having a long diameter 14mm x a short diameter 8mm. The tablets are ground with a power mill, mixed again with a tumbler mixing machine, and compressed with a tabletting machine to obtain tablets having a long diameter 14mm x a shorter diameter 8mm.

Example 17a

The solid dispersion (10220g) obtained in Example 13a, lactose (7856g), crystalline cellulose (27620g), light silicic acid anhydride (288g), calcium carmelose (1440g), and magnesium stearate (576g) were placed in a tumbler mixing machine, mixed for 30 minutes, and compressed with a tabletting machine to obtain tablets having a long diameter 14mm x a short diameter 8mm. The tablets were ground with a power mill, mixed again with a tumbler mixing machine, and compressed with a tabletting machine to obtain 65000 tablets having a long diameter 14mm x a shorter diameter 8mm.

Example 17b

The solid dispersion (10220g) obtained in Example 13b, lactose (7856g), crystalline cellulose (27620g), light silicic acid anhydride (288g), calcium carmelose (1440g), and magnesium stearate (576g) are placed in a tumbler mixing machine, and mixed for 30 minutes, and compressed with a tabletting machine to obtain tablets having a long diameter 14mm x a short diameter 8mm. The tablets are ground with a power mill, mixed again with a tumbler mixing machine, and compressed with a tabletting machine to obtain tablets having a long diameter 14mm x a shorter diameter 8mm.

Example 17c

The solid dispersion (10220g) obtained in Example 13c, lactose (7856g), crystalline cellulose (27620g), light silicic acid anhydride (288g), calcium carmelose (1440g), and magnesium stearate (576g) are placed in a tumbler mixing machine, mixed for 30 minutes, and compressed with a tabletting machine to obtain tablets having a long diameter 14mm x a short diameter 8mm. The tablets are ground with a power mill, mixed again with a tumbler mixing machine, and compressed with a tabletting machine to obtain tablets having a long diameter 14mm x a shorter diameter 8mm.

Example 18a

The solid dispersion (16870g) obtained in Example 13a, lactose (1914g), crystalline cellulose (28340g), light silicic acid anhydride (297g), calcium carmelose (1485g), and magnesium stearate (594g) were placed in a tumbler mixing machine, and mixed for 30 minutes, and compressed with a tabletting machine to obtain tablets having a long diameter 14mm x a short diameter 8mm. The tablets were ground with a power mill, and the ground material obtained in two cycles was mixed again with a tumbler mixing machine, and then compressed with a tabletting machine to obtain 140000 tablets having a long diameter 14mm x a shorter diameter 8mm.

Example 18b

The solid dispersion (16870g) obtained in Example 13b, lactose (1914g), crystalline cellulose (28340g), light silicic acid anhydride (297g), calcium carmelose (1485g), and magnesium stearate (594g) are placed in a tumbler mixing machine, and mixed for 30 minutes, and compressed with a tabletting machine to obtain tablets having a long diameter 14mm x a short diameter 8mm. The tablets are ground with a power mill, and the ground material obtained in two cycles is mixed again with a tumbler mixing machine, and then compressed with a tabletting machine to obtain tablets having a long diameter 14mm x a shorter diameter 8mm.

Example 18c

The solid dispersion (16870g) obtained in Example 13c, lactose (1914g), crystalline cellulose (28340g), light silicic acid anhydride (297g), calcium carmelose (1485g), and magnesium stearate (594g) are placed in a tumbler mixing machine, and mixed for 30 minutes, and compressed with a tabletting machine to obtain tablets having a long diameter 14mm x a short diameter 8mm. The tablets are ground with a power mill, and the ground material obtained in two cycles is mixed again with a tumbler mixing machine, and compressed with a tabletting machine to obtain tablets having a long diameter 14mm x a shorter diameter 8mm.

Example 19a

| | |
|---|---|
| (1) Solid dispersion of Example 1a | 50mg |
| (2) Lactose | 34mg |
| (3) Corn starch | 10.6mg |
| (4) Corn starch (geletinized) | 5mg |
| (5) Magnesium stearate | 0.4mg |
| (6) Potassium carboxymethylcellulose | 20mg |
| | Total 120mg |

According to the conventional method, the above (1) to (6) are mixed, and compressed with a tabletting machine to obtain tablets.

By using each of solid dispersions of Examples 1b to 10c instead of the solid dispersion of Example 1a, a tablet can be obtained.

Test Example 1: Experiment for measuring solubility

(1) Sample

(A) Solid dispersion prepared in Example 4a
(B) Crystal of Reference Example B4 itself as a control

(2) Method

According to the conventional method, the solubility of the compound of Reference Example B4 in the 2nd fluid (pH6.8) of 13th revision Japanese Pharmacopoeia was measured. That is, 10mL of the 2nd fluid (pH6.8) of 13th revision Japanese Pharmacopoeia was placed into a centrifuge tube, the above-mentioned experimental sample (containing 20mg of the compound of Reference Example B4) was added thereto, and shaken at room temperature. After continuous shaking for 3 hours, the material was filtered with a membrane filter having a pore size of 0.45μm. An amount of HER2 inhibitory substance of Reference Example B4 dissolved in the filtrate was measured by HPLC.

(3) Results

[Table 4]

| Experimental sample | Solubility (μg/mL) |
|---|---|
| A | 2172 |
| B | 0.0006 |

Test Example 2: Dissolution test

(1) Sample

(A) Crystal of the compound of Reference Example B4 itself as a control
(B) Solid dispersion of Example 1a
(C) Solid dispersion of Example 2a
(D) Solid dispersion of Example 4a
(E) Solid dispersion of Example 5a
(F) Solid dispersion of Example 6a
(G) Solid dispersion of Example 7a
(H) Solid dispersion of Example 8a
(I) Solid dispersion of Example 9a
(J) Solid dispersion of Example 10a

(2) Method

According to method 2 (paddle method) of the dissolution test of 13th revision Japanese Pharmacopoeia and using 500mL of the 2nd fluid of 13th revision Japanese Pharmacopoeia as a test fluid, tests were performed on 100mg of the compound of Reference Example B4, and a dissolved amount was measured at 5, 10, 30 and 120 min after starting the dissolution test under at 100rpm.

(3) Results

% dissolved of the compound of Reference Example B4 from the solid dispersion are shown in the following Table 5.

[Table 5]

| Experimental sample | % Dissolved | | | |
|---|---|---|---|---|
| | 5min | 10min | 30min | 120min |
| A | 0 | 0 | 0 | 0 |
| B | 71.7 | 78.5 | 81.4 | 78.6 |
| C | 90.2 | 93.6 | 94.8 | 97.4 |
| D | 98.6 | 100.6 | 98.6 | 98.5 |
| E | 93.7 | 94.6 | 95.2 | 95.3 |
| F | 77.8 | 86.8 | 90.5 | 97.1 |
| G | 53.6 | 77.4 | 102.1 | 102.5 |
| H | 94.6 | 94.2 | 96.2 | 97.2 |
| I | 78.8 | 91.2 | 94.4 | 95.5 |
| J | 94.7 | 103.7 | 99.7 | 100.3 |

Test Example 3: Bioavailability test

(1) Sample

(A) Crystal of the compound of Reference Example B4 itself as a control
(B) Solid dispersion of Example 1a
(C) Solid dispersion of Example 4a
(D) Solid dispersion of Example 5a
(E) Solid dispersion of Example 8a

(2) Method

The above sample (3mg/kg) containing the solid dispersion of the compound Reference Example B4 was orally administered to 9-week IGS/SD male rats under fed conditions. The concentration of the compound of Reference Example B4 in plasma was measured by HPLC at 1, 2, 4, 8 and 24 hours after administration.

(3) Results

The concentrations of the solid dispersion and the crystal of the compound of Reference Example 4 in plasma are shown in the following Table 6.

[Table 6]

| Experimental sample | Concentration in Plasma (µg/mL) | | | | |
|---|---|---|---|---|---|
| | 1 hour | 2 hours | 4 hours | 8 hours | 24 hours |
| A | 0.000 | 0.004 | 0.029 | 0.043 | 0.021 |
| B | 0.018 | 0.060 | 0.152 | 0.262 | 0.161 |
| C | 0.044 | 0.122 | 0.206 | 0.254 | 0.164 |
| D | 0.015 | 0.059 | 0.141 | 0.193 | 0.141 |
| E | 0.079 | 0.166 | 0.162 | 0.214 | 0.122 |

Industrial applicability

The solid dispersion of the present invention remarkably improves the solubility, the oral absorption and/or bioavailability of a water-poorly soluble or insoluble HER2 inhibitory substance.

## Claims

1. A pharmaceutical composition comprising a water-poorly soluble or insoluble HER2 inhibitory substance, wherein the solubility in water of the HER2 inhibitory substance is improved.

2. The composition according to claim 1, which is a solid dispersion.

3. The composition according to claim 1 or 2, which comprises a water-poorly soluble or insoluble HER2 inhibitory substance and a hydrophilic polymer.

4. The composition according to claim 1 or 2, wherein the HER2 inhibitory substance is amorphous.

5. The composition according to claim 3, wherein the hydrophilic polymer is cellulose derivative, polyalkenylpyrrolidone, polyalkylene glycol or methacrylic acid copolymer.

6. The composition according to claim 3, wherein the hydrophilic polymer is an enteric polymer.

7. The composition according to claim 3, wherein the hydrophilic polymer is hydroxypropylmethylcellulose phthalate.

8. The composition according to any one of claims 1 to 7, which further contains lactose.

9. The composition according to any one of claims 1 to 8, wherein the water-poorly soluble or insoluble HER2 inhibitory substance has a solubility in water of lower than 10mg/m at 25°C.

10. The composition according to any one of claims 1 to 8, wherein the water-poorly soluble or insoluble HER2 inhibitory substance is a compound represented by the formula:

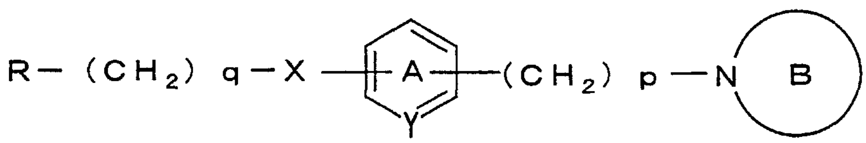

wherein R denotes an optionally substituted aromatic heterocyclic group, X denotes an oxygen atom, an optionally oxidized sulfur atom, -C(=O)- or -CH(OH)-, Y denotes CH or N, p denotes an integer of 0 to 10, q denotes an integer of 1 to 5, a group represented by the formula:

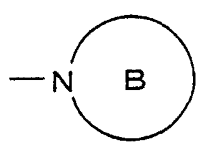

denotes an optionally substituted aromatic azole group, and ring A may be further substituted, or a salt thereof or a prodrug thereof.

11. The composition according to any one of claims 1 to 8, wherein the water-poorly soluble or insoluble HER2 inhibitory substance is a compound represented by the formula:

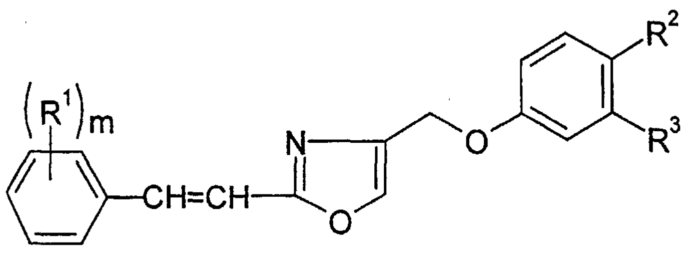

wherein m denotes 1 or 2, $R^1$ denotes halogen or optionally halogenated $C_{1-2}$alkyl, one of $R^2$ and $R^3$ denotes hydrogen atom, and the other denotes a group represented by the formula :

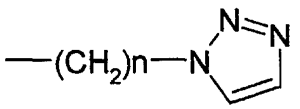

or

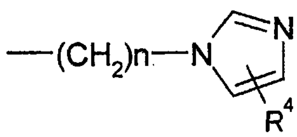

wherein n denotes 3 or 4, and $R^4$ denotes a $C_{1-4}$alkyl group substituted with 1 or 2 hydroxy group(s), or a salt thereof or a prodrug thereof.

**12.** The composition according to any one of claims 1 to 8, wherein the water-poorly soluble or insoluble HER2 inhibitory substance is (i) 1-(4-{4-[(2-{(E)-2-[4-(trifluoromethyl)phenyl]ethenyl}-1,3-oxazole-4-yl)methoxy]phenyl}butyl)-1H-1,2,3-triazole, (ii) 1-(3-{3-[(2-{(E)-2-[4-(trifluoromethyl)phenyl]ethenyl}-1,3-oxazole-4-yl)methoxy]phenyl}propyl)-1H-1,2,3-triazole, or (iii) 3-(1-{4-[4-({2-[(E)-2-(2,4-difluorophenyl)ethenyl]-1,3-oxazol-4-yl}methoxy)phenyl]butyl}-1H-imidazole-2-yl)-1,2-propanediol, or a salt thereof or a prodrug thereof.

**13.** The composition according to claim 3, wherein a weight ratio of the water-poorly soluble or insoluble HER2 inhibitory substance to the hydrophilic polymer is 1:1 to 1:20;

**14.** The composition according to claim 3, wherein a weight ratio of the water-poorly soluble or insoluble HER2 inhibitory substance to the hydrophilic polymer is 1:1 to 1:5.

**15.** The composition according to claim 3, wherein a weight ratio of the water-poorly soluble or insoluble HER2 inhibitory substance to the hydrophilic polymer is 1:2 to 1:4.

**16.** The composition according to claim 3, wherein a weight ratio of the water-poorly soluble or insoluble HER2 inhibitory substance to the hydrophilic polymer is 1:3 to 1:5.

**17.** The composition according to claim 1, which is an anti-cancer agent.

**18.** The composition accvording to claim 1, which is an agent for preventing or treating breast cancer or prostate cancer.

**19.** A method for preparing a solid dispersion comprising a water-poorly soluble or insoluble HER2 inhibitory substance and a hydrophilic polymer, which comprises removing an organic solvent from a suspension or a solution of a water-poorly soluble or insoluble HER2 inhibitory substance and a hydrophilic polymer in an organic solvent.

**20.** A pharmaceutical composition containing the solid dispersion according to claim 2.

## INTERNATIONAL SEARCH REPORT

International application No.
PCT/JP01/10768

A. CLASSIFICATION OF SUBJECT MATTER
Int.Cl[7] C07D413/12, A61K31/422, 9/10, 9/14, 47/20, 47/26, 47/28, 47/32, 47/38, 47/40, A61P35/00

According to International Patent Classification (IPC) or to both national classification and IPC

B. FIELDS SEARCHED

Minimum documentation searched (classification system followed by classification symbols)
Int.Cl[7] C07D413/12, A61K31/422, 9/10, 9/14, 47/20, 47/26, 47/28, 47/32, 47/38, 47/40, A61P35/00

Documentation searched other than minimum documentation to the extent that such documents are included in the fields searched

Electronic data base consulted during the international search (name of data base and, where practicable, search terms used)
CAPLUS, REGISTRY (STN)

C. DOCUMENTS CONSIDERED TO BE RELEVANT

| Category* | Citation of document, with indication, where appropriate, of the relevant passages | Relevant to claim No. |
|---|---|---|
| X | WO 98/3505 A2 (Takeda Chemical Industries, Ltd.), 29 January, 1998 (29.01.1998), & AU 9734616 A & EP 912562 A1 & CN 1223653 A & ZA 9706378 A & JP 11-60571 A & US 6211215 A | 1-20 |
| Y | WO 96/19239 A1 (Yamanouchi Pharm. Co., Ltd.), 27 June, 1996 (27.06.1996), & AU 9643141 A | 1-20 |
| Y | WO 98/51281 A1 (Senju Pharm. Co., Ltd.), 19 November, 1998 (19.11.1998), & JP 11-29463 A & EP 995435 A1 & US 6274634 A & KR 2001012188 A | 1-20 |

☒ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 05 February, 2002 (05.02.02) | 19 February, 2002 (19.02.02) |

| Name and mailing address of the ISA/ | Authorized officer |
|---|---|
| Japanese Patent Office | |
| Facsimile No. | Telephone No. |

Form PCT/ISA/210 (second sheet) (July 1992)

**INTERNATIONAL SEARCH REPORT**

International application No.
PCT/JP01/10768

C (Continuation). DOCUMENTS CONSIDERED TO BE RELEVANT

| Category* | Citation of document, with indication, where appropriate, of the relevant passages | Relevant to claim No. |
|---|---|---|
| Y | WO 86/6626 A1 (Eurand Ital S.p.A.),<br>20 November, 1986 (20.11.1986),<br>& PT 82541 A & ZA 8603399 A<br>& NO 8605162 A & AU 8659031 A<br>& EP 222856 A & FI 8605336 A<br>& ES 8707103 A & JP 62-502859 A<br>& DK 8700074 A & CA 1274144 A<br>& US 5008117 A | 1-20 |
| Y | JP 53-52617 A (Shinetsu Chem. Ind. Co., Ltd.),<br>13 May, 1978 (13.05.1978),<br>(Family: none) | 7 |

Form PCT/ISA/210 (continuation of second sheet) (July 1992)

**INTERNATIONAL SEARCH REPORT**

International application No.
PCT/JP01/10768

The invention as set forth in claim 1 pertains to an HER2 inhibitor. Although its chemical structure is not specified, the specific substance as set forth in claim 11 is exclusively disclosed in the detailed description of the invention. Namely, no other chemical is substantially cited therein. Therefore, this international search has been practiced mainly on the compound as set forth in claim 11.

Form PCT/ISA/210 (extra sheet) (July 1992)